# EUROPEAN PATENT APPLICATION

(11) **EP 4 360 629 A1**
(43) Date of publication of application: **01.05.2024**
(21) Application number: 22828825.4
(22) Date of filing: 24.06.2022
(51) Int. Cl.: A61K 31/17, A61K 31/402, A61K 31/5375, A61K 31/4245, A61P 31/14

(54) **PHARMACEUTICAL COMPOSITION COMPRISING LIPASE INHIBITOR FOR TREATMENT OR TREATMENT OF RNA VIRAL INFECTIONS**

(30) Priority: 25.06.2021 KR 20210083385; 23.06.2022 KR 20220077063
(71) Applicant: Industry Foundation of Chonnam National University, Gwangju 61186 (KR); Korea Research Institute of Bioscience and Biotechnology, Daejeon 34141 (KR)
(72) Inventor: CHO, Kyoung Oh, Gwangju 61185 (KR); JEON, Tae Il, Gwangju 61708 (KR); LEE, Sun Woo, Gwangju 61026 (KR); BAEK, Yeong Bin, Gwangju 61217 (KR); KWON, Hyung Jun, Sejong 30147 (KR); CHOI, Hueng Sik, Gwangju 61176 (KR); PARK, Sang Ik, Gwangju 61903 (KR); SHARIF, Muhammad, Gwangju 61184 (KR); LIM, Jeong Ah, Boseong-gun, Jeollanam-do 59447 (KR)
(74) Representative: Plougmann Vingtoft a/s
(86) International application number: PCT/KR2022/009041
(87) International publication number: WO 2022/270978

(57) **Abstract**

The present invention relates to a therapeutic agent of a lipase inhibitor for a wide spectrum of RNA viral infections in humans and animals. More specifically, the lipase inhibitor can be used as a therapeutic agent for influenza A virus infection, bovine coronavirus infection, porcine epidemic diarrhea coronavirus infection, bovine rotavirus infection, porcine reproductive and respiratory syndrome virus infection, and sapoviral infection and furthermore, can be utilized as a therapeutic agent for various RNA viral infections in humans and animals, such as infections with SARS CoV-1, MERS-CoV, Zika virus, dengue fever virus, and hepatitis A and C viruses.

## Description

### Technical Field

The present disclosure has been made with the support of the Ministry of Science and ICT under Project ID. No. 1711128903, and sub-project No. 2020R1A2B5B03002517, which was conducted by the Chonnam National University in the research program named "Elucidation of the Correlation between Host Nuclear Receptors and Metabolism Induced by Livestock RNA Virus Infection" as a branch of the research project titled "Individual Basic Research (Ministry of Science and ICT) (R&D)" under the research management of the National Research Foundation of Korea, from March 01, 2021 to February 28, 2021.

Also, the present disclosure has been made with the support of the Ministry of Science and ICT under Project ID. No. 1711170603, and sub-project No. KGM5242221, which was conducted by the Korea Research Institute of Bioscience and Biotechnology in the research program named "Development and Commercialization of High-Value Functional Bio-Materials" as a branch of the research project titled "Support for Research Operation Expenses of the Korea Research Institute of Bioscience and Biotechnology (Major Project Expenses)", under the research management of the Korea Research Institute of Bioscience and Biotechnology, from January 01, 2022 to December 31, 2022.

This application claims priority to and the benefit of Korean Application Numbers 10-2021-0083385 and 10-2022-0077063, filed in the Korean Intellectual Property Office on June 25, 2021 and June 23, 2022, respectively, the entire contents of which are incorporated herein by reference.

The present disclosure relates to a pharmaceutical composition including a lipase inhibitor for the prevention or treatment of RNA viral infections and, more specifically, to a pharmaceutical composition including an adipose triglyceride lipase (ATGL) inhibitor derivative for the prevention or treatment of RNA viral infections.

### Background Art

RNA viruses cause various diseases in humans and animals. Notably, the recent emergence of severe acute respiratory syndrome coronavirus types 1 and 2 (SARS-CoV-1, SARS-CoV-2), Middle East respiratory syndrome coronavirus (MERS-CoV), influenza virus, Zika virus, and dengue fever virus in humans are all RNA viruses.

RNA polymerases, which amplify RNA virus genes, do not have a proofreading function. This increases the mutation rate of RNA virus genes, making them more prone to mutations than DNA viruses. Consequently, viral proteins undergo mutations that can lead to the emergence of viruses resistant to therapeutic agents targeting them.

A classic example is the H3N2 influenza virus, of which 93% show resistance to adamantane derivatives, which are inhibitors of the matrix-2 (M2) protein, an influenza virus membrane ion channel protein. Also, resistance is increasing against oseltamivir, an inhibitor of the viral neuraminidase.

Globally, zoonotic viral infections such as influenza, SARS, MERS, Ebola, and Zika are seriously threatening human health and causing enormous socioeconomic damage. The global antiviral market size, which was 36.1 billion dollars in 2018, is projected to grow at an average annual rate of 3.2%, reaching approximately 44.2 billion dollars by 2026.

For influenza, the global market size is also projected to grow at an annual average of 4.5%, from $860 million in 2018 to approximately $1.12 billion in 2026. The global death toll from COVID-19 (caused by SARS-CoV-2) stands at 6.23 million as of April 30, 2022. The economic damage amounts to a monthly loss of KRW 440 trillion (as of August 1, 2020), and if it persists for 2 years, the total damage is estimated to exceed KRW 1,400 quadrillion. Additionally, viral zoonotic diseases inflict significant economic damage on livestock worldwide. For instance, a highly pathogenic avian influenza outbreak in South Korea in 2017 led to the culling of over 30 million birds and economic losses exceeding KRW 1 trillion.

Most of these causative agents are RNA viruses, which mutate easily, leading to various genotypes or phenotypes. For example, since the initial outbreak of COVID-19, variants such as the Alpha variant (from the UK), the Beta variant (from South Africa), the Delta variant (from India), and the Omicron variant (from South Africa) have emerged and are currently re-spreading in the world.

Recent research trends are focusing on developing treatments that target cellular factors necessary for viral replication.

### Detailed Description of the Invention

### Technical Problem

In the present disclosure, it was observed that when cells were infected with viruses exhibiting representative RNA virus characteristics, lipid droplets increased from the early to mid-stages of infection, and then decreased in the later stages. In addition, 3-(4'-(dimethylamino)-(1,1'-biphenyl)-3-yl)-1,1-dimethylurea (Atglistatin [DABPU-DM]), which is an inhibitor against the lipase ATGL, demonstrated superb broad-spectrum antiviral efficacy in vitro and in vivo, compared to CAY10499 (Cayman Chemicals; CAS Number: 359714-55-9). Furthermore, the derivative 3-(4'-dimethylamino)-(1,1'-biphenyl)-3-yl)-1,1-diethylurea[DABPU-DE] exhibited even more remarkable broad-spectrum antiviral activity.

Accordingly, an aspect of the present disclosure is to provide a pharmaceutical composition including a lipase inhibitor for the prevention or treatment of RNA viral infections.

Another aspect of the present disclosure is to provide an antiviral composition including a lipase inhibitor.

A further aspect of the present disclosure pertains to the use of a lipase inhibitor for preventing or treating RNA viral infections and for antiviral purposes.

### Technical Solution

The present disclosure concerns a pharmaceutical composition including a lipase inhibitor for preventing or treating RNA viral infections. The composition according to the present disclosure exhibits a prophylactic or therapeutic effect on RNA viral infections.

In the present disclosure, it was found that when cells were infected with viruses exhibiting representative RNA virus characteristics, 3-(4'-(dimethylamino)-(1,1'-biphenyl)-3-yl)-1,1-dimethylurea (Atglistatin [DABPU-DM]), which is an inhibitor against the lipase ATGL, exhibited superb broad-spectrum antiviral efficacy in vitro and in vivo, compared to CAY10499 (Cayman Chemicals; CAS Number: 359714-55-9) that inhibits adipose triglyceride lipase(adipose triglyceride lipase (ATGL), hormone-sensitive lipase (HSL), and monoacylglycerol lipase (MGL) in a non-selective manner. Furthermore, its derivative 3-(4'-dimethylamino)-(1,1'-biphenyl)-3-yl)-1,1-diethylurea [DABPU-DE] demonstrated even more remarkable broad-spectrum antiviral activity.

As used herein, the term "broad-spectrum antiviral efficacy" refers to the ability to inhibit the proliferation of RNA viruses regardless of whether the RNA virus has an outer membrane, whether its genetic material is positive-sense, negative-sense, or double-sense, and whether it is single-stranded linear or segmented.

Below, a detailed description will be given of the present disclosure.

An aspect of the present disclosure pertains to a pharmaceutical composition including a lipase inhibitor for preventing or treating RNA virus infections.

In the present disclosure, the lipase inhibitor may be at least one selected from the group consisting of an adipose triglyceride lipase (ATGL) inhibitor, a hormone-sensitive lipase (HSL) inhibitor, and a monoacylglycerol lipase (MGL) inhibitor.

As used herein, the term "adipose triglyceride lipase" refers to an intracellular lipase that hydrolyzes triglyceride into free fatty acids and diglyceride.

As used herein, the term "hormone-sensitive lipase" refers to an intracellular triglyceride lipase that hydrolyzes diglyceride into free fatty acids and monoglyceride.

As used herein, the term "monoacylglycerol lipase" (MGL)" refers to an intracellular triglyceride lipase that hydrolyzes monoglyceride into free fatty acids and glycerol.

The ATGL inhibitor may be 3-(4'-(dimethylamino)-(1,1'-biphenyl)-3-yl)-1,1-dimethylurea (Atglistatin [DABPU-DM]) or a derivative thereof.

As used herein, the term "3-(4'-(dimethylamino)-(1,1'-biphenyl)-3-yl)-1,1-dimethylurea" (Atglistatin [DABPU-DM]) means the compound that has 1-(4'-dimethylamino)-[1,1'-biphenyl]-3-yl-urea as a framework with a dimethylamine radical bonded to one amine radical of the urea moiety.

The derivative of 3-(4'-(dimethylamino)-(1,1'-biphenyl)-3-yl)-1,1-dimethylurea (Atglistatin [DABPU-DM]) may be at least one selected from the group consisting of 3-(4'-dimethylamino)-(1,1'-biphenyl)-3-yl)-1,1-diethylurea [DABPU-DE], 3-(4'-(dimethylamino)-(1,1'-biphenyl)-3-yl)-1-ethyl-1-methylurea [DABPU-EM], 3-(4'-dimethylamino)-(1,1'-biphenyl)-3-yl)-1,1-diphenylurea [DABPU-DPh], N-(4'-dimethylamino)-(1,1'-biphenyl)-3-yl)pyrolidine-1-carboxamide [DABPU-Pyrrol] and N-(4'-(dimethylamino)-(1,1`-biphenyl)-3-yl)morpholine-4-carboxamide [DABPU-Morph], with preference for 3-(4'-dimethylamino)-(1,1'-biphenyl)-3-yl)-1,1-diethylurea [DABPU-DE] or 3-(4'-(dimethylamino)-(1,1'-biphenyl)-3-yl)-1-ethyl-1-methylurea [DABPU-EM], for example, 3-(4'-dimethylamino)-(1,1'-biphenyl)-3-yl)-1,1-diethylurea [DABPU-DE], but with no limitations thereto.

As used herein, the term "3-(4'-dimethylamino)-(1,1'-biphenyl)-3-yl)-1,1-diethylurea" [DABPU-DE] means the compound having 1-(4'-dimethylamino)-[1,1'-biphenyl]-3-yl-urea as a framework in which one amine radical of the urea moiety in atglistatin is bonded with diethylamine, instead of dimethylamine.

As used herein, the term "3-(4'-(dimethylamino)-(1,1'-biphenyl)-3-yl)-1-ethyl-1-methylurea" [DABPU-EM] refers to the compound having 1-(4'-dimethylamino)-[1,1'-biphenyl]-3-yl-urea as a framework in which one amine radical of the urea moiety in atglistatin is bonded with ethylmethylamine, instead of dimethylamine.

As used herein, the term "3-(4'-dimethylamino)-(1,1'-biphenyl)-3-yl)-1,1-diphenylurea" [DABPU-DPh] refers to the compound having 1-(4'-dimethylamino)-[1,1'-biphenyl]-3-yl-urea as a framework in which one amine radical of the urea moiety in atglistatin is bonded with diphenylamine, instead of dimethylamine.

As used herein, the term "N-(4'-dimethylamino)-(1,1'-biphenyl)-3-yl)pyrolidine-1-carboxamide" [DABPU-Pyrrol] refers to the compound having 1-(4'-dimethylamino)-[1,1'-biphenyl]-3-yl-urea as a framework in which one amine radical of the urea moiety in atglistatin is bonded with pyrrole, instead of dimethylamine.

As used herein, the term "N-(4'-(dimethylamino)-(1,1'-biphenyl)-3-yl)morpholine-4-carboxamide" [DABPU-Morph] refers to the compound having 1-(4'-dimethylamino)-[1,1'-biphenyl]-3-yl-urea as a framework in which one amine radical of the urea moiety in atglistatin is bonded with morpholine, instead of dimethylamine.

The pharmaceutical composition may further include at least one antiviral agent selected from the group consisting of oseltamivir and remdesivir, but with no limitations thereto.

In an embodiment of the present disclosure, the pharmaceutical composition may include the lipase inhibitor and oseltamivir at a volume ratio of 2:2 to 8:2 and preferably at a volume ratio of 3:2 to 7:2, for example, 4:2 to 6:2, but with no limitations thereto.

In an embodiment of the present disclosure, the pharmaceutical composition may include the lipase inhibitor and remdesivir at a volume ratio of 8:1 to 20:1 and preferably at a volume ratio of 10:1 to 18:1, for example, 15:1 to 16:1, but with no limitations thereto.

In the present disclosure, the RNA virus may be at least one selected from the group consisting of SARS coronavirus types 1 and 2 (SARS-CoV-1, SARS-CoV-2), influenza A virus (IAV), bovine coronavirus (BCoV), porcine epidemic diarrhea coronavirus (PEDV), bovine species A rotavirus (RVA), porcine reproductive and respiratory syndrome virus (PRRSV), porcine sapovirus (PSaV), norovirus, feline infectious peritonitis virus (FIPV), MERS coronavirus, Zika virus, dengue fever virus, and hepatitis viruses A and B.

The term "coronavirus", as used herein, refers to viruses within the subfamily Orthocoronavirinae of the family Coronaviridae. These viruses are classified into four genera: alpha-, beta-, gamma-, delta-coronavirus. Within the alpha-coronavirus genus, there are viruses such as the porcine epidemic diarrhea coronavirus and the feline infectious peritonitis virus. Bovine coronavirus, SARS coronavirus types 1 and 2 (SARS-CoV-1, SARS-CoV-2), and the MERS coronavirus (MERS-CoV) fall within the beta-coronavirus genus. Representative of the gamma-coronavirus genus is the chicken infectious bronchitis virus, and examples of delta-coronavirus include human and pig delta coronaviruses. Coronaviruses have a size range of 80-120 nm and possess spikes on their envelope. Bovine coronaviruses, which belong to the gamma-coronavirus genus, additionally feature an external hemagglutinin/esterase protein spike. Inside the envelope is a positive-sense, single-stranded RNA genome.

As used herein, the term "influenza A virus" refers to a virus of the Alphainfluenzavirus genus within the Orthomyxoviridae family. This virus is 80-120 nm in size. The surface of the virus's envelope has spikes of hemagglutinin and neuraminidase oriented outwardly. Inside the envelope, there are eight segments of a negative-sense genome. The influenza A virus infects various mammals, including humans, pigs, horses, dogs, and a range of birds, notably chickens.

The term "bovine coronavirus" (BCoV), as used herein, refers to a virus that causes severe diarrhea in calves and mature cows domestically and worldwide.

The term "porcine epidemic diarrhea coronavirus" or "porcine epidemic diarrhea virus" (PEDV), as used herein, denotes a virus that causes diarrhea across all age groups both domestically and globally, and leads to a mortality rate of over 60% in suckling pigs.

The term "rotavirus", as used herein, refers typically to species A rotavirus (RVA). Within the Rotavirus genus in the Reoviridae family, there are nine species: A, B, C, D, F, G, H, I, and J. Among them, humans are primarily infected by species A rotavirus, while species A through I rotaviruses cause diseases in animals other than humans. The H type infects pigs, the D, F, and G types infect birds, the I type infects cats, and the J type has been reported in bats. Rotavirus possesses 11 segments of a double-stranded genome inside its 80-nm size three capsid layers. Rotavirus is a major causative agent of diarrhea in infants under 5 years of age and young livestock.

As used herein, the term "porcine reproductive and respiratory syndrome virus" (PRRSV) belongs to the Arterivirus genus of the Arteriviridae family. It is categorized into Type 1 (European type) and Type 2 (North American type). PRRSV has an envelope of 50-60 nm size with envelope protein and glycoprotein 4 spikes. Inside the envelope, there is a single-stranded, positive-sense RNA genome approximately 15 kb in length. PRRSV induces reproductive disorders like abortion and stillbirth in pregnant sows and interstitial pneumonia in piglets.

As used herein, the term "sapovirus" refers to a virus belonging to the Sapovirus genus within the Caliciviridae family. It is a causative agent for diarrhea in humans, pigs, and minks.

The term "feline infectious peritonitis virus" (FIPV), as used herein, denotes a pathogenic virus in cats that causes vasculitis, leading to severe pleural and abdominal effusions or chronic granulomatous nodules in various organs such as the kidneys.

The term "caliciviruses", as used herein, refers to viruses within the Caliciviridae family, which possess a linear, positive-sense single-stranded RNA genome within a capsid layer 27-40 nm in size. Within the Caliciviridae family, there are currently 11 recognized genera: Norovirus, Sapovirus, Vesivirus, Lagovirus, Nebovirus, Recovirus, Valovirus, Babovirus, Nacovirus, Salovirus, and Minovirus.

Another aspect of the present disclosure pertains to an antiviral composition including a lipase inhibitor.

In the present disclosure, the lipase inhibitor may be at least one selected from the group consisting of an ATGL inhibitor, an HSL inhibitor, and an MGL inhibitor.

The ATGL inhibitor may be 3-(4'-(dimethylamino)-(1,1'-biphenyl)-3-yl)-1,1-dimethylurea (Atglistatin [DABPU-DM]) and a derivative thereof.

The derivative of 3-(4'-(dimethylamino)-(1,1'-biphenyl)-3-yl)-1,1-dimethylurea (Atglistatin [DABPU-DM]) may be at least one selected from the group consisting of 3-(4'-dimethylamino)-(1,1'-biphenyl)-3-yl)-1,1-diethylurea [DABPU-DE], 3-(4'-(dimethylamino)-(1,1'-biphenyl)-3-yl)-1-ethyl-1-methylurea [DABPU-EM], 3-(4'-dimethylamino)-(1,1'-biphenyl)-3-yl)-1,1-diphenylurea [DABPU-DPh], N-(4'-dimethylamino)-(1,1'-biphenyl)-3-yl)pyrolidine-1-carboxamide [DABPU-Pyrrol] and N-(4'-(dimethylamino)-(1,1`-biphenyl)-3-yl)morpholine-4-carboxamide [DABPU-Morph and preferably from 3-(4'-dimethylamino)-(1,1'-biphenyl)-3-yl)-1,1-diethylurea [DABPU-DE] or 3-(4'-(dimethylamino)-(1,1'-biphenyl)-3-yl)-1-ethyl-1-methylurea [DABPU-EM], for example, 3-(4'-dimethylamino)-(1,1'-biphenyl)-3-yl)-1,1-diethylurea [DABPU-DE], but with no limitations thereto.

The pharmaceutical composition may further include at least one antiviral agent selected from the group consisting of oseltamivir and remdesivir, but with no limitations thereto.

In an embodiment of the present disclosure, the pharmaceutical composition may include a lipase inhibitor and oseltamivir at a volume ratio of 2:2 to 8:2 and preferably at a volume ratio of 3:2 to 7:2, for example, at a volume ratio of 4:2 to 6:2, but with no limitations thereto.

In an embodiment of the present disclosure, the pharmaceutical composition may include a lipase inhibitor and remdesivir at a volume ratio of 8:1 to 20:1 and preferably at a volume ratio of 10:1 to 18:1, for example, 15:1 to 16:1, but with no limitations thereto.

In the present disclosure, the RNA virus may be at least one selected from the group consisting of SARS-CoV-1, SARS-CoV-2, IAV, BCoV, PEDV, RVA, PRRSV, PSaV, norovirus, FIPV, MERS Corona Virus, Zika virus, dengue fever virus, and hepatitis viruses A and C.

### Advantageous Effects

The present disclosure concerns a lipase inhibitor-based therapeutic agent for a broad range of RNA viral infections in humans and animals. More specifically, the lipase inhibitor can be used as a therapeutic agent for influenza A virus infections and SARS-CoV-2 in humans and animals, and bovine coronavirus infections, porcine epidemic diarrhea coronavirus infections, bovine species A rotavirus infections, porcine reproductive and respiratory syndrome virus infections, and sapovirus infections in animals. Additionally, the inhibitor can be utilized as a therapeutic agent for various RNA viral infections in humans and animals, including SARS-CoV-1, MERS Coronavirus, Zika virus, dengue fever virus, and hepatitis viruses A and C.

### Brief Description of the Drawings

FIG. 1A shows photographic images and a plot illustrating the numbers of cytoplasmic lipid droplets in cultured cells infected with SARS-CoV-2 in accordance with an embodiment of the present disclosure.
FIG. 1B shows photographic images and a plot illustrating the numbers of cytoplasmic lipid droplets in cultured cells infected with influenza A virus (IAV) in accordance with an embodiment of the present disclosure.
FIG. 1C is a graph showing levels of cytoplasmic triglyceride (TG) and cholesterol in cultured cells infected with SARS-CoV-2 in accordance with an embodiment of the present disclosure.
FIG. 1D is a graph showing levels of cytoplasmic triglyceride (TG) and cholesterol in cultured cells infected with IAV in accordance with an embodiment of the present disclosure.
FIG. 2A shows photographic images and a plot illustrating the numbers of cytoplasmic lipid droplets in cultured cells infected with bovine coronavirus (BCoV) in accordance with an embodiment of the present disclosure.
FIG. 2B shows photographic images and a plot illustrating the numbers of cytoplasmic lipid droplets in cultured cells infected with porcine epidemic diarrhea coronavirus (PEDV) in accordance with an embodiment of the present disclosure.
FIG. 2C shows photographic images and a plot illustrating the numbers of cytoplasmic lipid droplets in cultured cells infected with bovine species A rotavirus (RVA) in accordance with an embodiment of the present disclosure.
FIG. 2D shows photographic images and a plot illustrating the numbers of cytoplasmic lipid droplets in cultured cells infected with porcine reproductive and respiratory syndrome virus (PRRSV) in accordance with an embodiment of the present disclosure.
FIG. 2E shows photographic images and a plot illustrating the numbers of cytoplasmic lipid droplets in cultured cells infected with porcine sapovirus (PSaV) in accordance with an embodiment of the present disclosure.
FIG. 3A shows photographic images illustrating changes in cytoplasmic lipid droplet levels in alveolar epithelial cells from SARS-CoV-2-infected hamsters in accordance with an embodiment of the present disclosure.
FIG. 3B shows photographic images illustrating changes in cytoplasmic lipid droplet levels in bronchiolar epithelial cells from IVA-infected mice in accordance with an embodiment of the present disclosure.
FIG. 3C is a graph showing quantities of cytoplasmic triglyceride and cholesterol in alveolar epithelial cells from SARS-CoV-2-infected hamsters in accordance with an embodiment of the present disclosure.
FIG. 3D is a graph showing quantities of cytoplasmic triglyceride and cholesterol in alveolar epithelial cells from IVA-infected mice in accordance with an embodiment of the present disclosure.
FIG. 4A shows photographic images and a graph illustrating changes in phosphorylated hormone-sensitive lipase (HSL) in the cytoplasm of cultured cells infected with SARS-CoV-2 in accordance with an embodiment of the present disclosure.
FIG. 4B shows photographic images and a graph illustrating changes in phosphorylated hormone-sensitive lipase in the cytoplasm of cultured cells infected with IAV in accordance with an embodiment of the present disclosure.
FIG. 4C is a graph showing quantities of cytoplasmic cytoplasmic free fatty acid (FFA) and cholesterol in cultured cells infected with SARS-CoV-2 in accordance with an embodiment of the present disclosure.
FIG. 4D is a graph showing quantities of cytoplasmic free fatty acid and cholesterol in cultured cells infected with IVA in accordance with an embodiment of the present disclosure.
FIG. 5A shows photographic images and a graph illustrating changes in cytoplasmic phosphorylated hormone-sensitive lipase in cultured cells infected with BCoV in accordance with an embodiment of the present disclosure.
FIG. 5B shows photographic images and a graph illustrating changes in cytoplasmic phosphorylated hormone-sensitive lipase in cultured cells infected with PEDV in accordance with an embodiment of the present disclosure.
FIG. 5C shows photographic images and a graph illustrating changes in cytoplasmic phosphorylated hormone-sensitive lipase in cultured cells infected with RVA in accordance with an embodiment of the present disclosure.
FIG. 5D shows photographic images and a graph illustrating changes in cytoplasmic phosphorylated hormone-sensitive lipase in cultured cells infected with PRRSV in accordance with an embodiment of the present disclosure.
FIG. 5E shows photographic images and a graph illustrating changes in cytoplasmic phosphorylated hormone-sensitive lipase in cultured cells infected with PSaV in accordance with an embodiment of the present disclosure.
FIG. 6A shows photographic images illustrating changes in cytoplasmic phosphorylated hormone-sensitive lipase in alveolar epithelial cells from SARS-CoV-2-infected hamsters in accordance with an embodiment of the present disclosure.
FIG. 6B shows photographic images illustrating changes in cytoplasmic phosphorylated hormone-sensitive lipase in bronchiolar epithelial cells from IVA-infected mice in accordance with an embodiment of the present disclosure.
FIG. 6C shows photographic images and a plot illustrating changes in phosphorylated hormone-sensitive lipase in lung tissues from SARS-CoV-2-infected hamsters in accordance with an embodiment of the present disclosure.
FIG. 6D shows photographic images and a plot illustrating changes in phosphorylated hormone-sensitive lipase in lung tissues from IVA-infected hamsters in accordance with an embodiment of the present disclosure.
FIG. 6E is a graph showing changes in free fatty acid and glycerol levels in lung tissues from SARS-CoV-2-infected hamsters in accordance with an embodiment of the present disclosure.
FIG. 6F is a graph showing changes in free fatty acid and glycerol levels in lung tissues from IVA-infected mice in accordance with an embodiment of the present disclosure.
FIG. 7A is a graph showing changes in cytoplasmic free fatty acid and glycerol levels in Vero E6 cells infected with SARS-CoV-2 in accordance with an embodiment of the present disclosure.
FIG. 7B is a graph showing changes in cytoplasmic free fatty acid and glycerol levels in A549 cells infected with IAV in accordance with an embodiment of the present disclosure.
FIG. 7C is a graph showing changes in cytoplasmic free fatty acid and glycerol levels in HRT-18G cells infected with BCoV in accordance with an embodiment of the present disclosure.
FIG. 7D is a graph showing changes in cytoplasmic free fatty acid and glycerol levels in Vero E6 cells infected with PEDV in accordance with an embodiment of the present disclosure.
FIG. 7E is a graph showing changes in cytoplasmic free fatty acid and glycerol levels in MA104 cells infected with RVA in accordance with an embodiment of the present disclosure.
FIG. 7F is a graph showing changes in cytoplasmic free fatty acid and glycerol levels in MARC-145 cells infected with PRRSV in accordance with an embodiment of the present disclosure.
FIG. 7G is a graph showing changes in cytoplasmic free fatty acid and glycerol levels in LLC-PK cells infected with PSaV in accordance with an embodiment of the present disclosure.
FIG. 8A is a plot of viral genome copies in SARS-CoV-2-infected cells treated with atglistatin (MedChemExpress; CAS Number: 1469924-27-3) and CAY10499 (Cayman Chemicals; CAS Number: 359714-55-9) in accordance with an embodiment of the present disclosure.
FIG. 8B is a plot of progeny virus numbers in SARS-CoV-2-infected cells treated with atglistatin and CAY10499 in accordance with an embodiment of the present disclosure.
FIG. 8C is a graph of cytoplasmic free fatty acid and glycerol levels in SARS-CoV-2-infected cells treated with atglistatin and CAY10499 in accordance with an embodiment of the present disclosure.
FIG. 8D shows photographic images illustrating synthesized levels of virus spike protein (S) in SARS-CoV-2-infected cells treated with atglistatin and CAY10499 in accordance with an embodiment of the present disclosure.
FIG. 8E is a plot of viral genome copies in IAV-infected cells treated with atglistatin and CAY10499 in accordance with an embodiment of the present disclosure.
FIG. 8F is a plot of progeny virus numbers in IAV-infected cells treated with atglistatin and CAY10499 in accordance with an embodiment of the present disclosure.
FIG. 8G is a graph of cytoplasmic free fatty acid and glycerol levels in IAV-infected cells treated with atglistatin and CAY10499 in accordance with an embodiment of the present disclosure.
FIG. 8H shows photographic images illustrating synthesized levels of virus spike protein (S) in IAV-infected cells treated with atglistatin and CAY10499 in accordance with an embodiment of the present disclosure.
FIG. 8I shows photographic images and a graph illustrating the palmitoylation of spike proteins in SARS-CoV-2-infected cells treated with atglistatin and CAY10499 in accordance with an embodiment of the present disclosure.
FIG. 8J shows photographic images illustrating synthesized levels of hemagglutinin (H) and M2 protein in IAV-infected cells treated with atglistatin and CAY10499 in accordance with an embodiment of the present disclosure.
FIG. 9A is a plot showing the inhibitory effects of atglistatin and CAY10499 on SARS-CoV-2 in accordance with an embodiment of the present disclosure.
FIG. 9B is a plot showing the inhibitory effects of atglistatin and CAY10499 on IAV in accordance with an embodiment of the present disclosure.
FIG. 9C is a plot showing the inhibitory effects of atglistatin and CAY10499 on BCoV in accordance with an embodiment of the present disclosure.
FIG. 9D is a plot showing the inhibitory effects of atglistatin and CAY10499 on PEDV in accordance with an embodiment of the present disclosure.
FIG. 9E is a plot showing the inhibitory effects of atglistatin and CAY10499 on RVA in accordance with an embodiment of the present disclosure.
FIG. 9F is a plot showing the inhibitory effects of atglistatin and CAY10499 on PRRSV in accordance with an embodiment of the present disclosure.
FIG. 9G is a plot showing the inhibitory effects of atglistatin and CAY10499 on PSaV in accordance with an embodiment of the present disclosure.
FIG. 10A is a plot of viral genome copies of SARS-CoV-2, IAV, BCoV, PEDV, RVA, PRRSV, and PSaV versus treatment with atglistatin in accordance with an embodiment of the present disclosure.
FIG. 10B is a plot of viral genome copies of SARS-CoV-2, IAV, BCoV, PEDV, RVA, PRRSV, and PSaV versus treatment with CAY10499 in accordance with an embodiment of the present disclosure.
FIG. 10C is a plot of progeny virus numbers of SARS-CoV-2, IAV, BCoV, PEDV, RVA, PRRSV, and PSaV versus treatment with atglistatin in accordance with an embodiment of the present disclosure.
FIG. 10D is a plot of progeny virus numbers of SARS-CoV-2, IAV, BCoV, PEDV, RVA, PRRSV, and PSaV versus treatment with CAY10499 in accordance with an embodiment of the present disclosure.
FIG. 11A is a schematic diagram illustrating the process of constructing IVA-infected mice and administering atglistatin and CAY10499 thereto in accordance with an embodiment.
FIG. 11B is a plot of survival rates of IAV-infected mice injected with atglistatin in accordance with an embodiment of the present disclosure.
FIG. 11C is a plot of body weights in IAV-infected mice injected with atglistatin in accordance with an embodiment of the present disclosure.
FIG. 11D is a plot of clinical symptoms in IVA-infected mice injected with atglistatin in accordance with an embodiment of the present disclosure.
FIG. 11E is a plot of survival rates of IAV-infected mice injected with CAY10499 in accordance with an embodiment of the present disclosure.
FIG. 11F a plot of body weights in IAV-infected mice injected with CAY10499 in accordance with an embodiment of the present disclosure.
FIG. 11G is a plot of clinical symptoms in IVA-infected mice injected with CAY10499 in accordance with an embodiment of the present disclosure.
FIG. 11H is a graph of free fatty acid and glycerol levels in lung tissues from IAV-infected mice injected with atglistatin and CAY10499 in accordance with an embodiment of the present disclosure.
FIG. 11I is a plot of viral genome copies in lung tissues from IAV-infected mice injected with atglistatin and CAY10499 in accordance with an embodiment of the present disclosure.
FIG. 11J is a graph of progeny virus numbers in lung tissues from IAV-infected mice injected with atglistatin and CAY10499 in accordance with an embodiment of the present disclosure.
FIG. 11K shows photographic images of bronchiolar epithelial cells from IAV-infected mice injected with atglistatin and CAY10499 in accordance with an embodiment of the present disclosure.
FIG. 11L shows photographic images illustrating lung lesions and virus-positive cell numbers in IAV-infected mice injected with atglistatin and CAY10499 in accordance with an embodiment of the present disclosure.
FIG. 12A is a schematic diagram illustrating the process of constructing SARS-CoV-2-infected hamsters and administering atglistatin and CAY10499 thereto in accordance with an embodiment.
FIG. 12B is a graph showing pneumonia severity in SARS-CoV-2-infected hamsters injected with atglistatin in accordance with an embodiment of the present disclosure.
FIG. 12C is a graph showing free fatty acid and glycerol production in SARS-CoV-2-infected hamsters injected with atglistatin in accordance with an embodiment of the present disclosure.
FIG. 12D is a plot of viral genome copies in lung tissues from SARS-CoV-2-infected hamsters injected with atglistatin in accordance with an embodiment of the present disclosure.
FIG. 12E is a plot of progeny virus numbers in lung tissues from SARS-CoV-2-infected hamsters injected with atglistatin in accordance with an embodiment of the present disclosure.
FIG. 12F is a plot of body weight changes in SARS-CoV-2-infected hamsters injected with atglistatin in accordance with an embodiment of the present disclosure.
FIG. 12G shows photographic images of alveolar epithelial cells from SARS-CoV-2-infected hamsters injected with atglistatin in accordance with an embodiment of the present disclosure.
FIG. 12H shows photographic images illustrating lung lesions and virus-positive cell numbers in SARS-CoV-2-infected hamsters injected with atglistatin in accordance with an embodiment of the present disclosure.
FIG. 13A is a schematic diagram illustrating the process of constructing IAV-infected mice and administering atglistatin and oseltamivir individually or in combination thereto in accordance with an embodiment of the present disclosure.
FIG. 13B is a plot of survival rates of IAV-infected mice injected with atglistatin and oseltamivir individually or in combination in accordance with an embodiment of the present disclosure.
FIG. 13C is a plot of body weight changes in IAV-infected mice injected with atglistatin and oseltamivir individually or in combination in accordance with an embodiment of the present disclosure.
FIG. 13D is a plot of clinical symptoms in IAV-infected mice injected with atglistatin and oseltamivir individually or in combination in accordance with an embodiment of the present disclosure.
FIG. 13E is a schematic diagram illustrating the process of constructing SARS-CoV-2-infected hamsters and administering atglistatin and remdesivir individually or in combination thereto in accordance with an embodiment of the present disclosure.
FIG. 13F is a plot of lung lesions in SARS-CoV-2-infected hamsters injected with atglistatin and remdesivir individually or in combination in accordance with an embodiment of the present disclosure.
FIG. 13G is a plot of body weight changes in SARS-CoV-2 A lineage-infected hamsters injected with atglistatin and remdesivir individually or in combination in accordance with an embodiment of the present disclosure.
FIG. 13H is a plot of clinical symptoms in SARS-CoV-2 Alpha lineage-infected hamsters injected with atglistatin and remdesivir individually or in combination in accordance with an embodiment of the present disclosure.
FIG. 13I is a plot of body weight changes in SARS-CoV-2 Beta lineage-infected hamsters injected with atglistatin and remdesivir individually or in combination in accordance with an embodiment of the present disclosure.
FIG. 14A shows the chemical formula of atglistatin.
FIG. 14B shows the chemical formula of the atglistatin derivative 3-(4'-(dimethylamino)-(1,1'-biphenyl)-3-yl)-1-ethyl-1-methylurea [DABPU-EM].
FIG. 14C shows the chemical formula of the atglistatin derivative 3-(4'-dimethylamino)-(1,1'-biphenyl)-3-yl)-1,1-diethylurea [DABPU-DE].
FIG. 14D shows the chemical formula of the atglistatin derivative 3-(4'-dimethylamino)-(1,1'-biphenyl)-3-yl)-1,1-diphenylurea [DABPU-DPh].
FIG. 14E shows the chemical formula of the atglistatin derivative N-(4'-dimethylamino)-(1,1'-biphenyl)-3-yl)pyrolidine-1-carboxamide [DABPU-Pyrrol].
FIG. 14F shows the chemical formula of the atglistatin derivative N-(4'-(dimethylamino)-(1,1'-biphenyl)-3-yl)morpholine-4-carboxamide [DABPU-Morph].
FIG. 15A shows photographic images illustrating expression levels of virus antigens in SARS-CoV-2-infected cells treated with DABPU-EM and DABPU-DE in accordance with an embodiment of the present disclosure.
FIG. 15B shows photographic images illustrating expression levels of virus antigens in IVA-infected cells treated with DABPU-EM and DABPU-DE in accordance with an embodiment of the present disclosure.
FIG. 15C shows photographic images illustrating expression levels of virus antigens in BCoV-infected cells treated with DABPU-EM and DABPU-DE in accordance with an embodiment of the present disclosure.
FIG. 15D shows photographic images illustrating expression levels of virus antigens in PEDV-infected cells treated with DABPU-EM and DABPU-DE in accordance with an embodiment of the present disclosure.
FIG. 15E shows photographic images illustrating expression levels of virus antigens in RVA-infected cells treated with DABPU-EM and DABPU-DE in accordance with an embodiment of the present disclosure.
FIG. 15F shows photographic images illustrating expression levels of virus antigens in PRRSV-infected cells treated with DABPU-EM and DABPU-DE in accordance with an embodiment of the present disclosure.
FIG. 15G shows photographic images illustrating expression levels of virus antigens in PSaV-infected cells treated with DABPU-EM and DABPU-DE in accordance with an embodiment of the present disclosure.
FIG. 16A shows plots illustrating antiviral effects of DABPU-EM and DABPU-DE in SARS-CoV-2-infected cells in accordance with an embodiment of the present disclosure.
FIG. 16B shows plots illustrating antiviral effects of DABPU-EM and DABPU-DE in IAV-infected cells in accordance with an embodiment of the present disclosure.
FIG. 16C shows plots illustrating antiviral effects of DABPU-EM and DABPU-DE in BCoV-infected cells in accordance with an embodiment of the present disclosure.
FIG. 16D shows plots illustrating antiviral effects of DABPU-EM and DABPU-DE in PEDV-infected cells in accordance with an embodiment of the present disclosure.
FIG. 16E shows plots illustrating antiviral effects of DABPU-EM and DABPU-DE in MA104-infected cells in accordance with an embodiment of the present disclosure.
FIG. 16F shows plots illustrating antiviral effects of DABPU-EM and DABPU-DE in PRRSV-infected cells in accordance with an embodiment of the present disclosure.
FIG. 16G shows plots illustrating antiviral effects of DABPU-EM and DABPU-DE in PSaV-infected cells in accordance with an embodiment of the present disclosure.
FIG. 17A is a plot of viral genome copies in cells infected with SARS-CoV-2, IAV, BCoV, PEDV, RVA, PRRSV, and PSaV and treated with DABPU-EM in accordance with an embodiment of the present disclosure.
FIG. 17B is a plot of viral genome copies in cells infected with SARS-CoV-2, IAV, BCoV, PEDV, RVA, PRRSV, and PSaV and treated with DABPU-DE in accordance with an embodiment of the present disclosure.
FIG. 17C is a plot of progeny virus numbers in cells infected with SARS-CoV-2, IAV, BCoV, PEDV, RVA, PRRSV, and PSaV and treated with DABPU-EM in accordance with an embodiment of the present disclosure.
FIG. 17D is a plot of progeny virus numbers in cells infected with SARS-CoV-2, IAV, BCoV, PEDV, RVA, PRRSV, and PSaV and treated with DABPU-DE in accordance with an embodiment of the present disclosure.
FIG. 18A is a schematic diagram illustrating the process of constructing IAV-infected mice and administering DABPU-EM and DABPU-DE individually or in combination thereto in accordance with an embodiment of the present disclosure.
FIG. 18B is a plot of survival rates of IAV-infected mice injected with DABPU-EM in accordance with an embodiment of the present disclosure.
FIG. 18C is a plot of body weight changes in IAV-infected mice injected with DABPU-EM in accordance with an embodiment of the present disclosure.
FIG. 18D is a plot of clinical symptoms in IAV-infected mice injected with DABPU-EM in accordance with an embodiment of the present disclosure.
FIG. 18E is a plot of survival rates of IAV-infected mice injected with DABPU-DE in accordance with an embodiment of the present disclosure.
FIG. 18F is a plot of body weight changes in IAV-infected mice injected with DABPU-DE in accordance with an embodiment of the present disclosure.
FIG. 18G is a plot of clinical symptoms in IAV-infected mice injected with DABPU-DE in accordance with an embodiment of the present disclosure.
FIG. 18H is a graph showing free fatty acid and glycerol levels in lung tissues from IVA-infected mice injected with DABPU-EM and DABPU-DE in accordance with an embodiment of the present disclosure.
FIG. 18I is a plot of viral genome copies in lung tissues from IAV-infected mice injected with DABPU-EM and DABPU-DE in accordance with an embodiment of the present disclosure.
FIG. 18J is a graph of progeny virus numbers in lung tissues from IAV-infected mice injected with DABPU-EM and DABPU-DE in accordance with an embodiment of the present disclosure.
FIG. 18K shows photographic images of bronchiolar epithelial cells from IAV-infected mice injected with DABPU-EM and DABPU-DE in accordance with an embodiment of the present disclosure.
FIG. 18L shows photographic images illustrating lung lesions and virus-positive cell numbers in IAV-infected mice injected with DABPU-EM and DABPU-DE in accordance with an embodiment of the present disclosure.
FIG. 19A is a schematic diagram illustrating the process of constructing SARS-CoV-2-infected hamsters and administering DABPU-EM and DABPU-DE individually or in combination thereto in accordance with an embodiment of the present disclosure.
FIG. 19B is a graph showing pneumonia severity in SARS-CoV-2-infected hamsters injected with DABPU-DM and DABPU-D in accordance with an embodiment of the present disclosure.
FIG. 19C is a graph illustrating the production of free fatty acid and glycerol in SARS-CoV-2-infected hamsters injected with DABPU-DM and DABPU-DE in accordance with an embodiment of the present disclosure.
FIG. 19D is a plot of viral genome copies in SARS-CoV-2-infected hamsters injected with DABPU-DM and DABPU-DE in accordance with an embodiment of the present disclosure.
FIG. 19E is a plot of progeny virus numbers in SARS-CoV-2-infected hamsters injected with DABPU-DM and DABPU-DE in accordance with an embodiment of the present disclosure.
FIG. 19F is a plot of body weight changes in SARS-CoV-2-infected hamsters injected with DABPU-DM and DABPU-DE in accordance with an embodiment of the present disclosure.
FIG. 20A is a schematic diagram illustrating the process of constructing IAV-infected mice and administering DABPU-DE and oseltamivir individually or in combination thereto in accordance with an embodiment of the present disclosure.
FIG. 20B is a plot of survival rates of IAV-infected mice injected with DABPU-DE and oseltamivir individually or in combination in accordance with an embodiment of the present disclosure.
FIG. 20C is a plot of body weight changes in IAV-infected mice injected with DABPU-DE and oseltamivir individually or in combination in accordance with an embodiment of the present disclosure.
FIG. 20D is a plot of clinical symptoms in IAV-infected mice injected with DABPU-DE and oseltamivir individually or in combination in accordance with an embodiment of the present disclosure.
FIG. 20E is a schematic diagram illustrating the process of constructing hamsters infected with SARS-CoV-2 A lineage, Alpha lineage, or Beta lineage and administering DABPU-DE and remdesivir individually or in combination thereto in accordance with an embodiment of the present disclosure.
FIG. 20F is a plot illustrating pneumonia severity in hamsters infected with SARS-CoV-2 A lineage, Alpha lineage, or Beta lineage and administering DABPU-DE and remdesivir individually or in combination thereto in accordance with an embodiment of the present disclosure.
FIG. 20G is a plot of body weight changes in SARS-CoV-2 A lineage-infected hamsters injected with DABPU-DE, and remdesivir individually or in combination in accordance with an embodiment of the present disclosure.
FIG. 20H is a plot of body weight changes in SARS-CoV-2 Alpha lineage-infected hamsters injected with DABPU-DE, and remdesivir individually or in combination in accordance with an embodiment of the present disclosure.
FIG. 20I is a plot of body weight changes in SARS-CoV-2 Beta lineage-infected hamsters injected with DABPU-DE, and remdesivir individually or in combination in accordance with an embodiment of the present disclosure.
FIG. 21A is a plot of viral genome copies in SARS-CoV-2-infected Vero E6 cells treated with DABPU-DM, DABPU-EM, and DABPU-DE in accordance with an embodiment of the present disclosure.
FIG. 21B is a plot of viral genome copies in SARS-CoV-2-infected Caco-2 cells treated with DABPU-DM, DABPU-EM, and DABPU-DE in accordance with an embodiment of the present disclosure.
FIG. 21C is a plot of viral genome copies in SARS-CoV-2-infected Calu-3 cells treated with DABPU-DM, DABPU-EM, and DABPU-DE in accordance with an embodiment of the present disclosure.
FIG. 21D is a plot of progeny virus number in SARS-CoV-2-infected Vero E6 cells treated with DABPU-DM, DABPU-EM, and DABPU-DE in accordance with an embodiment of the present disclosure.
FIG. 21E is a plot of progeny virus number in SARS-CoV-2-infected Caco-2 cells treated with DABPU-DM, DABPU-EM, and DABPU-DE in accordance with an embodiment of the present disclosure.
FIG. 21F is a plot of progeny virus number in SARS-CoV-2-infected Calu-3 cells treated with DABPU-DM, DABPU-EM, and DABPU-DE in accordance with an embodiment of the present disclosure.
FIG. 21G is a plot of viral genome copies in IVA-infected MRC-5 cells treated with DABPU-DM, DABPU-EM, and DABPU-DE in accordance with an embodiment of the present disclosure.
FIG. 21H is a plot of viral genome copies in IVA-infected NCI-H293 cells treated with DABPU-DM, DABPU-EM, and DABPU-DE in accordance with an embodiment of the present disclosure.
FIG. 21I is a plot of viral genome copies in IVA-infected WI-38 cells treated with DABPU-DM, DABPU-EM and DABPU-DE in accordance with an embodiment of the present disclosure.
FIG. 21J is a plot of viral genome copies in IVA-infected Calu-3 cells treated with DABPU-DM, DABPU-EM, and DABPU-DE in accordance with an embodiment of the present disclosure.
FIG. 21K is a plot of progeny virus number in IAV-CoV-2-infected MRC-5 cells treated with DABPU-DM, DABPU-EM, and DABPU-DE in accordance with an embodiment of the present disclosure.
FIG. 21L is a plot of progeny virus number in IAV-CoV-2-infected NCI-H293 cells treated with DABPU-DM, DABPU-EM, and DABPU-DE in accordance with an embodiment of the present disclosure.
FIG. 21M is a plot of progeny virus number in IAV-CoV-2-infected WI-38 cells treated with DABPU-DM, DABPU-EM, and DABPU-DE in accordance with an embodiment of the present disclosure.
FIG. 21N is a plot of progeny virus number in IAV-CoV-2-infected Calu-3 cells treated with DABPU-DM, DABPU-EM, and DABPU-DE in accordance with an embodiment of the present disclosure.

### Best Mode for Carrying Out the Invention

The present disclosure concerns a pharmaceutical composition containing a lipase inhibitor for the prevention or treatment of RNA virus infections.

### Mode for Carrying Out the Invention

A better understanding of the present disclosure may be obtained through the following examples, which are set forth to illustrate, but are not to be construed to limit, the present disclosure.

Unless otherwise stated, "%" used to indicate the concentration of a specific substance is (weight/weight) % for solid/solid, (weight/volume) % for solid/liquid, and (volume/volume) % for liquid/liquid throughout the specification.

To increase the reliability of the results under identical conditions, three independent experiments were conducted. The values for each group were converted to mean ± standard error. The statistical significance of the above results was analyzed using the One-Way ANOVA test (GraphPad Prism software version 8.4.2), and a p-value of 0.05 or higher was considered to be statistically significant.

### PREPARATION EXAMPLE 1: Virus Information

For research using a wide spectrum of RNA viruses, representative viruses that can be cell cultured were used, as shown in Table 1 below.

**TABLE 1**

| Information about Viruses Used. | | |
|---|---|---|
| Virus | Strain (genotypes) | Source |
| Severe acute respiratory syndrome coronavirus 2 (SARS-CoV-2) | KCDC03 (Lineage A) | A kind gift from the Korea Disease Control and Prevention Agency |
| Severe acute respiratory | KDCA51463 (Alpha | A kind gift from the Korea Disease Control and |
| syndrome coronavirus 2 (SARS-CoV-2) | lineage) | Prevention Agency |
| Severe acute respiratory syndrome coronavirus 2 (SARS-CoV-2) | KDCA55905 (Beta lineage) | A kind gift from the Korea Disease Control and Prevention Agency |
| Influenza A virus | A/Puerto Rico/8/1934 (H1N1) | US American Type Culture Collection |
| Bovine coronavirus | KWD20 | Isolated from fecal samples and propagated in HRT-18G and MDBK cells |
| Porcine epidemic diarrhea coronavirus | QIAP1401 (G2b) | A kind gift from the Animal and Plant Quarantine Agency, Korea |
| Bovine species A rotavirus | NCDV (G6P6[1]) | American Type Culture Collection |
| Porcine reproductive and respiratory syndrome virus | LMY (North American type) | A kind gift from the Animal and Plant Quarantine Agency, Korea |
| Porcine sapovirus | Cowden (GIII.1) | A kind gift from Dr. K.O. Chang, Kansas State University A |

### PREPARATION EXAMPLE 2: Cell Information

For use in culturing SARS-CoV-2 KCDC03 strain (lineage A), SARS-CoV-2 KDCA51463 strain (Alpha lineage), and SARS-CoV-2 KDCA55905 (Beta lineage), African green monkey kidney epithelial Vero E6 cells, human colorectal adenocarcinoma epithelial Caco-2 cells, and human lung adenocarcinoma epithelial Calu-3 cells were prepared.

For use in culturing influenza A virus PR8 strain, the human lung adenocarcinoma epithelium A549, the human lung fibroblast MRC-5, the human lung mucoepidermoid carcinoma epithelium NCI-H292, and the Human lung fibroblasts WI-38 and Calu-3 were prepared.

For use in culturing bovine coronavirus KWD20 strain, the human colorectal adenocarcinoma epithelium HRT-18G was prepared.

For use in culturing porcine epidemic diarrhea coronavirus QIAP1401 strain, Vero E6 was prepared.

For use in culturing bovine species rotavirus NCDV strain, the Rhesus monkey kidney epithelium MA104 was prepared.

For use in culturing porcine reproductive and respiratory syndrome virus LMY strain, the African green monkey kidney epithelium MARC-145 was prepared.

For use in culturing porcine sapovirus Cowden strain, the porcine kidney epithelium LLC-PK was prepared.

(FBS: fetal bovine serum, DMEM: Dulbecco's modified Eagle's medium, EMEM: Eagle's Minimum Essential Medium, α-MEM: Alpha Minimal Essential Medium, 1% P/S: 100 U/mL penicillin, 100 ug/mL streptomycin)

**TABLE 2**

| Information about Cells Used. | | | | |
|---|---|---|---|---|
| Cell line | Origin | Source | Medium | Supplementat ion* |
| Vero E6 | African green monkey kidney epithelium | ATCC | EMEM | 10% FBS, 1% P/S |
| Caco-2 | Human colorectal cancer epithelial cells | ATCC | DMEM | 10% FBS, 1% P/S |
| Calu-3 | Human lung adenocarcinoma epithelial cells | ATCC | EMEM | 10% FBS, 1% P/S |
| A549 | Human lung cancer epithelial cells | ATCC | DMEM | 10% FBS, 1% P/S |
| MRC-5 | Human lung fibroblasts | ATCC | EMEM | 10% FBS, 1% P/S |
| NCI-H292 | human mucoepidermoid pulmonary carcinoma cells | ATCC | RPMI | 10% FBS, 1% P/S |
| WI-38 | Human lung fibroblasts | ATCC | EMEM | 10% FBS, 1% P/S |
| HRT-18G | Human colorectal cancer epithelial cells | ATCC | DMEM | 10% FBS, 1% P/S |
| MA104 | Rhesus monkey kidney epithelium | ATCC | α-MEM | 10% FBS, 1% P/S |
| MARC-145 | African green monkey kidney epithelium | ATCC | DMEM | 10% FBS, 1% P/S |
| LLC-PK | Porcine kidney epithelial cells | ATCC | EMEM | 10% FBS, 1% P/S |

Various experimental methods were performed to identify the inhibitory activity and proliferation regulatory mechanism of the aforementioned lipase inhibitor against a broad spectrum of viruses.

### PREPARATION EXAMPLE 3: Information about Antibodies

**TABLE 3**

| Antibodies* | Company | Country | Solvent* |
|---|---|---|---|
| Primary antibodies | | | |
| HSL(hormone-sensitive lipase) | Cell Signaling | USA | 5% BSA in 1xTBST (Tris-Buffered Saline containing 0.1% Tween^{®}20 Detergent) |
| pHSL(phosphor ylated HSL) (S563) | Cell Signaling | USA | 5% BSA in 1xTBST |
| pHSL(S660) | Cell Signaling | USA | 5% BSA in 1xTBST |
| PLIN1(perilip in 1) | Santa Cruz | USA | 5% BSA in 1xTBST |
| PLIN3(perilip in 3) | Abcam | UK | 5% BSA in 1xTBST |
| SARS-CoV-2 S(spike protein) | Prosci | USA | 5% BSA in 1xTBST |
| SARS-CoV-2 N(nucleoprote in) | Prosci | USA | 5% BSA in 1xTBST |
| IAV M2(matrix-2 protein) | Abcam | UK | 5% BSA in 1xTBST |
| IAV HA(hemaggluti nin) | Santa Cruz | USA | 5% BSA in 1xTBST |
| IAV virion | Virostat | USA | 5% BSA in 1xTBST |
| β-actin | Thermo Scientific | USA | 5% BSA in 1xTBST |
| GAPDH(glycera | Santa Cruz | USA | 5% BSA in |
| ldehyde 3-phosphate dehydrogenase ) | | | 1xTBST |
| BCoV S | Native Antigen Company | UK | 5% BSA in 1xTBST |
| PEDV N | Medgene Labs | USA | 5% BSA in 1xTBST |
| RVA VP6(viral structural protein 6) | Median Diagnostic | South Korea | 5% BSA in 1xTBST |
| RVA NSP5^{a}(nonstru ctural protein 5) | Kindly gifted^{a} | | 5% BSA in 1xTBST |
| PRRSV M(matrix protein) | Bioss Antibodies | USA | 5% BSA in 1xTBST |
| PSaV VPg^{b}(viral genome-linked protein) | Manufactured in the lab^{b} | | 5% BSA in 1xTBST |

| Secondary antibodies | | | |
|---|---|---|---|
| Rabbit IgG(H+L) | Cell Signaling | USA | 1xTPBT |
| Goat IgG(H+L) | Cell Signaling | USA | 1xTPBT |
| Mouse IgGκ | Santa Cruz | USA | 1xTPBT |
| AF647 rabbit IgG | Thermo Scientific | USA | 1xPBS |
| AF594 rabbit IgG | Thermo Scientific | USA | 1xPBS |
| AF488 rabbit IgG | Thermo Scientific | USA | 1xPBS |
| AF647 mouse IgG | Thermo Scientific | USA | 1xPBS |
| AF594 mouse IgG | Thermo Scientific | USA | 1xPBS |
| AF488 mouse IgG | Thermo Scientific | USA | 1xPBS |

### PREPARATION EXAMPLE 4: Information about Primers

**TABLE 4**

| SEQ ID NO: | Name | Sequence (5'-3') |
|---|---|---|
| 1 | SARS-CoV-2 N F | TAATCAGACAAGGAACTGATTA |
| 2 | SARS-CoV-2 N R | CGAAGGTGTGACTTCCATG |
| 3 | IAV PB1 F | CTGCCAGAAGACAATGAACC |
| 4 | IAV PB1 R | GGCCATTGCTTCCAATACAC |
| 5 | RVA VP6 F | TAGACCAAATAACGTTGAAGTTGA |
| 6 | RVA VP6 R | GATTCACAAACTGCAGATTCAA |
| 7 | PEDV F | GCTATGCTCAGATCGCCAGT |
| 8 | PEDV R | TCTCGTAAGAGTCCGCTAGCTC |
| 9 | PRRSV M F | CACCTCCAGATGCCGTTTG |
| 10 | PRRSV M R | ATGCGTGGTTATCATTTGCC |
| 11 | PSaV VPg F | CGAAAGGGAAAAACAAACGC |
| 12 | PSaV VPg R | TCACTCACTGTCATAGGTGTCACC |
| 13 | BCoV N F | TGGATCAAGATTAGAGTTGGC |
| 14 | BCoV N R | CCTTGTCCATTCTTCTGACC |
| 15 | L32 F | TCTGGTGAAGCCCAAGATCG |
| 16 | L32 R | CTCTGGGTTTCCGCCAGT |
| 17 | Human GAPDH F | ATTCCACCCATGGCAAATTC |
| 18 | Human GAPDH R | CGCTCCTGGAAGATGGTGAT |
| 19 | Mouse GAPDH F | AAGGTCATCCCAGAGCTGAA |
| 20 | Mouse GAPDH R | CTGCTTCACCACCTTCTTGA |
| 21 | Porcine GAPDH F | ACCTCCACTACATGGTCTACA |
| 22 | Porcine GAPDH R | ATGACAAGCTTCCCGTTCTC |

### EXPERIMENTAL EXAMPLE 1: Change in Cytoplasmic Lipid Droplet in Virus-Infected Cells

Examination was made of changes in cytoplasmic lipid droplet (LD) in cells infected with nine types of viruses.

**TABLE 5**

| Information about cells for culturing SARS-CoV-2, influenza A virus, bovine coronavirus, porcine epidemic diarrhea coronavirus, bovine species A rotavirus, porcine reproductive and respiratory syndrome virus, porcine sapovirus | | | | |
|---|---|---|---|---|
| Viruses | Cell lines | Activation of viruses | Medium | Supplementat ion* |
| SARS-CoV-2 | Vero E6 | N/A | EMEM | 1 µg/mL TPCK-treated trypsin, 1% P/S |
| | Caco-2 | N/A | DMEM | 1 µg/mL TPCK-treated trypsin, 1% P/S |
| | Calu-3 | N/A | EMEM | 1 µg/mL TPCK-treated trypsin, 1% P/S |
| Influenza A virus | A549 | N/A | DMEM | 1 µg/mL TPCK-treated trypsin, 1% P/S |
| | MRC-5 | N/A | EMEM | 1 µg/mL TPCK-treated trypsin, 1% P/S |
| | NCI-H292 | N/A | RPMI | 1 µg/mL TPCK-treated trypsin, 1% P/S |
| | WI-38 | N/A | EMEM | 1 µg/mL TPCK-treated trypsin, 1% P/S |
| | Calu-3 | N/A | EMEM | 1 µg/mL TPCK-treated trypsin, 1% P/S |
| Bovine coronavirus | HRT-18G | N/A | DMEM | 5 µg/mL pancreatin, 1% P/S |
| Porcine epidemic diarrhea coronavirus | Vero E6 | N/A | EMEM | 3 µg/mL porcine pancreatic trypsin, 1% P/S |
| Bovine species A rotavirus | MA104 | Preactivation with 10 µg/mL crystalized trypsin | α-MEM | 1 µg/mL crystalized trypsin, 1% P/S |
| Porcine reproductiv e and respiratory syndrome virus | MARC-145 | N/A | DMEM | 1% P/S |
| Porcine sapovirus | LLC-PK | N/A | EMEM | 2.5% FBS, 200 µM GCDCA, 1% P/S |

### 1-1. Change of cytoplasmic lipid droplet in cultured cells due to severe acute respiratory syndrome coronavirus 2 (SARS-CoV-2) and influenza A virus (IAV) infection

For use in investigating changes of cytoplasmic lipid droplets in cultured cells due to SARS-CoV-2 and influenza A virus infection, Vero E6 cells, which are African green monkey kidney epithelial cells, were cultured in monolayers in EMEM medium supplemented with 10% fetal bovine serum, 100 u/ml penicillin, and 100 ug/ml streptomycin. Human lung cancer cells, A549, were also cultured in monolayers in EMEM medium supplemented with 10% fetal bovine serum, 100 u/ml penicillin, and 100 ug/ml streptomycin (TPCK-treated trypsin: N-tosyl-L-phenylalanine chloromethyl ketone (TPCK)-treated trypsin, Sigma Aldrich, GCDCA: glycochenodeoxycholic acid).

To measure the titers of SARS-CoV-2 KCDC03 strain (Lineage A), SARS-CoV-2 KDCA51463 strain (Alpha lineage), and SARS-CoV-2 KDCA55905 strain (Beta lineage), the monolayer cultured Vero E6 cells were inoculated with SARS-CoV-2. Afterward, EMEM supplemented with 1 ug/ml N-tosyl-L-phenylalanine chloromethyl ketone (TPCK)-treated trypsin, 100 u/ml penicillin, and 100 ug/ml streptomycin was added. After 24 hours of incubation at 37°C in a CO2 incubator, a cell culture immunofluorescence assay (CCIF assay) was performed using an antibody against SARS-CoV-2 nucleocapsid (N) protein. The titers for the SARS-CoV-2 KCDC03 strain, SARS-CoV-2 KDCA51463 strain, and SARS-CoV-2 KDCA55905 strain were expressed in ffu/mL (fluorescence focus units per milliliter).

To measure the titer of influenza A virus PR8 strain, the monolayer cultured A549 cells were inoculated with the virus. Subsequently, DMEM supplemented with 1 ug/ml TPCK-treated trypsin, 100 u/ml penicillin, and 100 ug/ml streptomycin was added. After 24 hours of incubation at 37°C in a CO2 incubator, a cell culture immunofluorescence assay was conducted using an antibody against influenza A virus nucleoprotein. The titer of the influenza A virus PR8 strain was also expressed in ffu/mL.

In 8-well chambers, the monolayer-cultured Vero E6 cells were inoculated with or without the SARS-CoV-2 KCDC03 strain at a multiplicity of infection (MOI) of 0.1 ffu/mL. They were then cultured for 4, 8, 12, 18, 24, 36, and 48 hours at 37°C in a CO2 incubator after the addition of DMEM supplemented with 1 ug/ml TPCK-treated trypsin, 100 u/ml penicillin, and 100 ug/ml streptomycin.

Also, the monolayer-cultured A549 cells were inoculated with or without the influenza A virus PR8 strain at an MOI of 1 ffu/mL in the 8-well chambers. They were then cultured for 1, 4, 8, 12, 18, 24, 36, and 48 hours at 37°C in a CO2 incubator after the addition of DMEM supplemented with 1 ug/ml TPCK-treated trypsin, 100 u/ml penicillin, and 100 ug/ml streptomycin.

For the experiments, cells were fixed with 4% paraformaldehyde and subjected to immunofluorescence staining for SARS-CoV-2 nucleocapsid protein and or BODIPY staining (BODIPY 493/503; Sigma Aldrich) for lipid droplets or immunofluorescence staining for influenza A virus M2 protein and or BODIPY staining (BODIPY 493/503; Sigma Aldrich) for lipid droplets. The fixed cells were washed with phosphate-buffered saline (PBS, pH 7.4), blocked with 5% BSA at 20°C for 1 hour, and then incubated overnight at 4°C with primary antibodies specific for the respective viruses. Next day, the cells were incubated with the Alexa Fluorescence (AF) 647-conjugated secondary antibody at 37°C for 1 hour, and mounted with Slow-Fade Gold antifade reagent with 4', 6-diamidino-2-phenylindole (DAPI) (Molecular Probes, Eugene, OR, USA) for nucleus staining.

Immunofluorescence images of the stained samples were randomly captured in ten different areas at 400x magnification using the LSM 400 confocal microscope (Carl Zeiss; Jena, Germany) and LSM software. The changes of lipid droplets in A549 cells due to influenza A virus infection were processed using Adobe Photoshop CS6 as follows:
1) BODIPY-stained lipids were counted using the NIH ImageJ software ImageJ particle analysis method, and the results were presented as mean ± standard deviation (S.D.).
2) The positive signals of each virus protein and BODIPY were quantified into pixel units and presented as mean ± S.D.
3) The statistical significance of the results was analyzed using the One-Way ANOVA test, and a p-value greater than 0.05 was considered statistically significant.

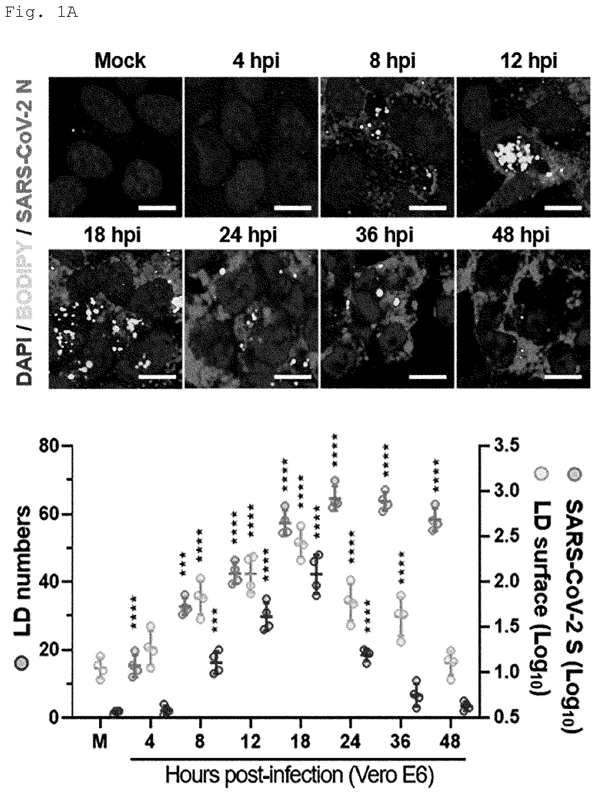

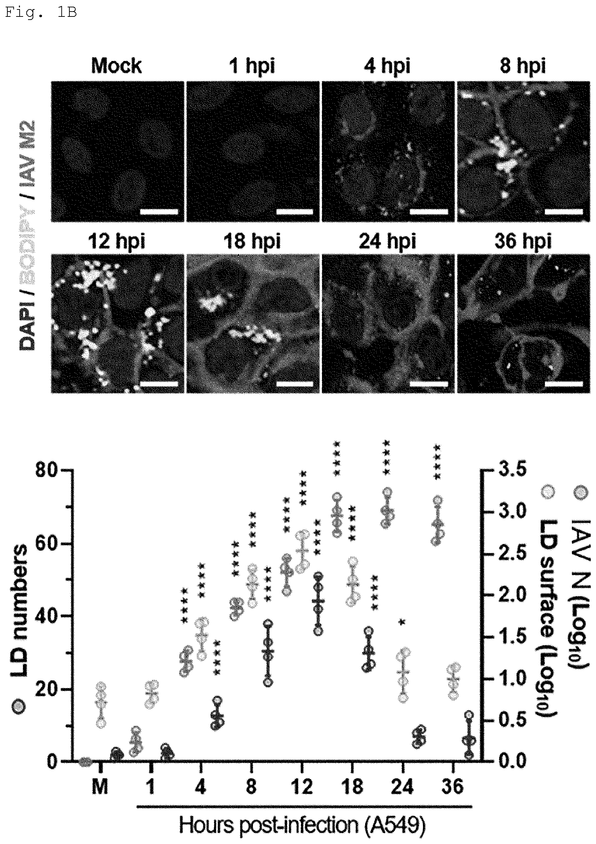

As can be seen in FIGS. 1a and 1b, while the viral replication in Vero E6 or A549 cells infected with either SARS-CoV-2 or influenza A virus increased in a time-dependent manner, there was no colocalization between the lipid droplets and viral proteins.

As shown in FIG. 1A, the number of lipid droplets in cells infected with SARS-CoV-2 increased from the start of infection until 18 hours, and then decreased after 24 hours, compared to the uninfected control group.

As shown in FIG. 1B, the number of lipids in cells infected with the influenza A virus increased from the start of infection until 12 hours and then decreased after 18 hours, compared to the uninfected control group.

To observe the changes in intracellular triglycerides and total cholesterol due to the infection of SARS-coronavirus type 2 KCDC03 strain or influenza A virus PR8 strain, cells were monolayer cultured in 100π cell culture dishes. The virus was then infected under the same conditions as previously described for the immunofluorescence antibody assay. After infection, cells were harvested at the same time point using trypsin, and countered using a hemocytometer. Aliquots of 5 × 106 cells were used for each analysis, centrifuged, washed with phosphate-buffered saline. The washed cell pellets were stored at -20°C.

Cells harvested at various time points of culturing SARS-coronavirus type 2 KCDC03 strain or influenza A virus PR8 strain were subjected to experiments using the Colorimetric Triglyceride Assay purchased from Cayman Chemicals and the Colorimetric Cholesterol Assay purchased from Abcam according to the manufacturers' instructions as follows:
For triglycerides (TG), the cell pellet was mixed with the Standard Diluent provided in the kit, sonicated, and centrifuged. The supernatant thus obtained was collected and then reacted with the enzyme mixture for 15 minutes at room temperature. The absorbance was read at 570 nm on an ELISA reader, and quantitated using the standard sample concurrently reacted.

For total cholesterol, the cell pellet was mixed with the cholesterol assay buffer provided in the kit and vortexed every minute for 20 minutes on ice. After centrifugation, the supernatant was collected and reacted with the cholesterol reaction mix containing cholesterol esterase at 37°C for 60 minutes. The values measured at 570 nm on the ELISA reader were used to calculate the total cholesterol content based on the equation derived from the standard samples.

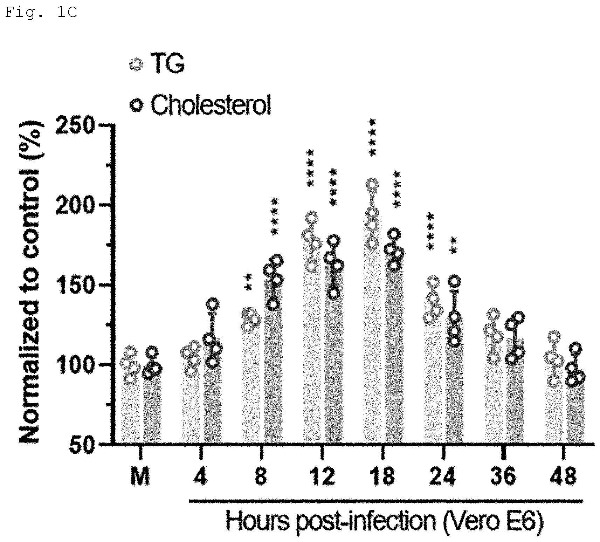

As can be seen in FIG. 1C, the SARS-CoV-2-infected Vero E6 cells showed an increase in cytoplasmic lipid droplets, triglycerides, and cholesterol, until 18 hours post infection, followed by a decrease.

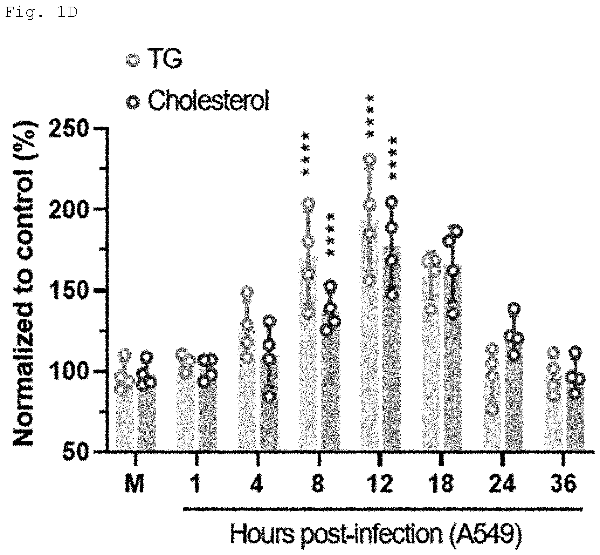

Also, as can be seen in FIG. 1D, the influenza A virus-infected A549 cells showed an increase in cytoplasmic lipid droplets and cholesterol until 18 hours post infection, followed by a decrease.

In conclusion, both SARS-CoV-2 and influenza A virus, when cultured in infected cells, lead to an increase in the quantity of lipid droplets and its components such as triglycerides and cholesterol in the initial and mid-phase of infection and then break them down in the later stage.

### 1-2. Change of cytoplasmic lipid droplet in cultured cells due to infection with bovine coronavirus (BCoV), porcine epidemic diarrhea coronavirus (PEDV), bovine species A rotavirus (RVA), porcine reproductive and respiratory syndrome virus (PRRSV), and porcine sapovirus (PSaV)

To achieve the goal, cells were infected with representative RNA viruses capable of being cultured in cells, such as bovine coronavirus KWD20 strain, porcine epidemic diarrhea coronavirus QIAP1401 strain, bovine species A rotavirus NCDV strain, porcine reproductive and respiratory syndrome virus LMY strain, and porcine sapovirus Cowden strain, and evaluated for lipid droplets.

For use in culturing bovine coronavirus, the human colorectal adenocarcinoma cell line HRT-18G, purchased from the ATCC, was monolayer cultured in DMEM supplemented with 10% fetal bovine serum 100 u/ml penicillin, and 100 ug/ml streptomycin.

To measure the titer of bovine coronavirus strain KWD20, HRT-18G cells were monolayer cultured in 96-well plates (SPL Life Science, Pocheon, South Korea). In a 96-well plate separately prepared, DMEM was used as a diluent to generate ten-fold serial dilutions of the virus and the final well contained only DMEM medium without any virus. The medium was removed from the monolayer-cultured HRT-18G cells which were then washed with phosphate-buffered saline before inoculation with each of the serial virus dilutions or DMEM without virus. The virus was then allowed to adsorb to the cells for 1 hour in a 37°C CO2 incubator.

Afterwards, the cells were washed with phosphate-buffered saline and cultured in DMEM supplemented with 5 µg/mL porcine pancreatin (Gibco, Fort Worth, TX, USA), 100 u/ml penicillin, and 100 ug/ml streptomycin in a 37°C incubator. After 24 hours, the medium was removed from the 96-well plates where the virus had been cultured. Washing with PBS was followed by fixation with 4% paraformaldehyde at 20°C for 10 minutes. An immunofluorescence assay was conducted using an antibody against the bovine coronavirus spike protein (Native Antigen Company, Killington, UK). The titer of the bovine coronavirus strain KWD20 used in this disclosure was expressed in ffu/mL (focus-forming units per milliliter).

To this end, HRT-18G cells were monolayer-cultured in 8-well chambers (SPL) which were then washed with phosphate-buffered saline. The bovine coronavirus strain KWD20 with a multiplicity of infection (MOI) of 0.1 ffu/cell, as determined by immunofluorescence assay, was inoculated. The virus was adsorbed onto the cells at 37°C for 1 hour. The cells were then washed with phosphate-buffered saline and cultured in DMEM supplemented with 5 µg/mL porcine pancreatin and the aforementioned antibiotics. The cells were incubated in a 37°C CO2 incubator for 18, 36, and 54 hours. The cells in each chamber were then washed with phosphate-buffered saline, fixed with 4% paraformaldehyde, and permeabilized with 0.2% Triton-X at 20°C for 10 minutes.

To observe changes in viral protein and lipid droplets due to the infection of bovine coronavirus KWD20, primary antibodies against the spike protein of the bovine coronavirus and an AF647-conjugated secondary antibody were reacted according to the aforementioned method. Subsequently, lipid droplets were stained with BODIPY. Thereafter, nuclei were stained with DAPI, followed by mounting.

The immunofluorescence assay results for lipid droplets and hormone-sensitive lipase after each viral infection were analyzed using Adobe Photoshop CS6, in the same manner as described in Experimental Example 1-1.

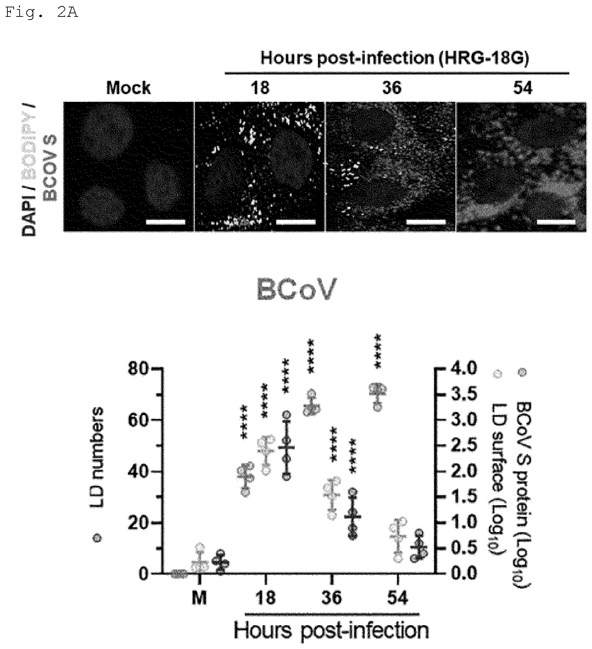

As can be seen from FIG. 2A, when the monolayer-cultured HRT-18G cell line was inoculated with or without bovine coronavirus KWD20 strain, the viral antigen continuously increased until the end of the experiment at 54 hours post-infection. However, the number of lipid droplets increased initially but decreased after 36 hours of infection.

For use in culturing porcine epidemic diarrhea coronavirus, the African green monkey kidney epithelial cell line Vero E6, purchased from the ATCC, was monolayer-cultured in EMEM supplemented with 10% fetal bovine serum, 100 u/ml penicillin, and 100 ug/ml streptomycin.

To measure the titer of the porcine epidemic diarrhea coronavirus strain Q1401, Vero E6 cells were monolayer-cultured in 96-well plates. After removal of the medium from the Vero E6 cell culture, the cells were washed with phosphate-buffered saline and inoculated with each of 10-fold serial virus dilutions or virus-free EMEM, followed by incubation in a 37°C CO2 incubator for 1 hour to allow the virus to adsorb to the cells.

Subsequently, washing with phosphate-buffered saline was conducted before culturing the viruses in EMEM supplemented with 3 ug/mL porcine pancreatic trypsin (Sigma, St. Louis, MO, USA), 100 u/ml penicillin, and 100 ug/ml streptomycin in a 37°C incubator. After 24 hours, the medium was removed from the 96-well plates where the viruses had been cultured. Washing with PBS was followed by fixation with 4% paraformaldehyde at 20°C for 10 minutes. An immunofluorescence assay was conducted using an antibody against the nucleocapsid (N) protein of the porcine epidemic diarrhea coronavirus (Medgene Labs, Brookings, SD, USA). The titer of the porcine epidemic diarrhea coronavirus strain Q1401 used in this disclosure was expressed in ffu/mL.

To this end, LLC-PK cells were monolayer-cultured in 8-well chambers (SPL) which were then washed with phosphate-buffered saline. The porcine epidemic diarrhea coronavirus strain Q1401 with a multiplicity of infection (MOI) of 0.1 ffu/cell, as determined by immunofluorescence assay, was inoculated. The virus was adsorbed onto the cells at 37°C for 1 hour. The cells were then washed with phosphate-buffered saline and cultured in DMEM supplemented with 3 µg/mL porcine pancreatin and the aforementioned antibiotics. The cells were incubated in a 37°C CO2 incubator for 18, 36, and 54 hours. The cells in each chamber were then washed with phosphate-buffered saline, fixed with 4% paraformaldehyde, and permeabilized with 0.2% Triton-X at 20°C for 10 minutes.

To observe changes in viral protein and lipid droplets due to the infection of porcine epidemic diarrhea coronavirus Q1401 strain, primary antibodies against the nucleocapsid protein of the porcine epidemic diarrhea coronavirus and an AF647-conjugated secondary antibody were reacted according to the aforementioned method. Subsequently, lipid droplets were stained with BODIPY. Thereafter, nuclei were stained with DAPI, followed by mounting.

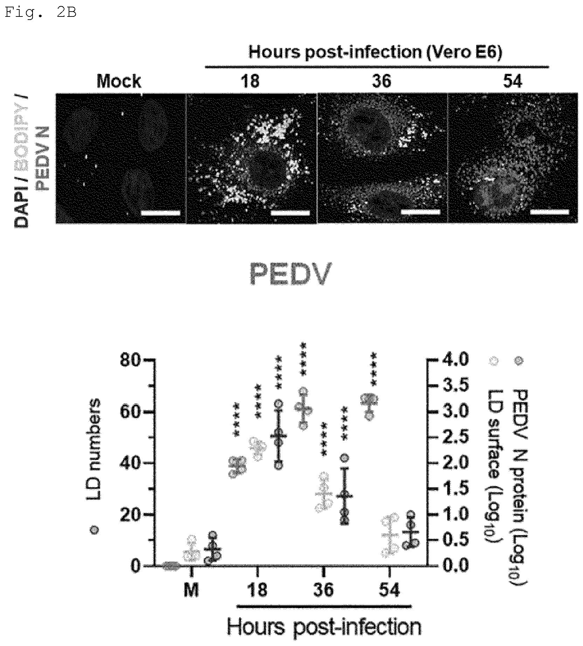

As can be seen from FIG. 2B, when the monolayer-cultured Vero E6 cell line was inoculated with or without porcine epidemic diarrhea coronavirus QIAP1401 strain, the viral antigen continuously increased until the end of the experiment at 54 hours post infection. However, the number of lipid droplets increased initially but decreased after 36 hours of infection.

Bovine species A rotavirus NCDV (G6P6[1]) strain was purchased from the ATCC, and for use in culturing the virus, Rhesus monkey kidney epithelial MA104 strain from the ATCC was monolayer cultured in α-MEM supplemented with 10% fetal bovine serum, 100 u/ml penicillin, and 100 ug/ml streptomycin.

To measure the titer of bovine species A rotavirus NCDV strain, MA104 cells were monolayer cultured in 96-well plates. In a 96-well plate separately prepared, α-MEM was used as a diluent to generate ten-fold serial dilutions of the virus and the final well contained only α-MEM medium without any virus. The medium was removed from the monolayer-cultured MA104 cells which were then washed with phosphate-buffered saline before inoculation with each of the serial virus dilutions or α-MEM without virus. The virus was then allowed to adsorb to the cells for 1 hour in a 37°C CO2 incubator.

Subsequently, washing with phosphate-buffered saline was conducted before culturing the viruses in α-MEM supplemented with 10 µg/mL crystalized trypsin (Gibco), 100 u/ml penicillin, and 100 ug/ml streptomycin in a 37°C incubator. After 24 hours, the medium was removed from the 96-well plates where the viruses had been cultured. Washing with PBS was followed by fixation with 4% paraformaldehyde at 20°C for 10 minutes. An immunofluorescence assay was conducted using an antibody against the bovine species A rotavirus VP6 protein (Median Diagnostic, Chuncheon, South Korea). The titer of bovine species A rotavirus NCDV strain used in this disclosure was expressed in ffu/mL.

To this end, MA104 cells were monolayer-cultured in 8-well chambers (SPL) which were then washed with phosphate-buffered saline. The bovine species A rotavirus NCDV strain with a multiplicity of infection (MOI) of 0.1 ffu/cell, as determined by immunofluorescence assay, was inoculated. The virus was adsorbed onto the cells at 37°C for 1 hour. The cells were then washed with phosphate-buffered saline and cultured in α-MEM supplemented with 5 µg/mL pancreatin and the aforementioned antibiotics. The cells were incubated in a 37°C CO2 incubator for 12, 24, and 36 hours. The cells in each chamber were then washed with phosphate-buffered saline, fixed with 4% paraformaldehyde, and permeabilized with 0.2% Triton-X at 20°C for 10 minutes.

To observe changes in viral protein and lipid droplets due to the infection of bovine species A rotavirus NCDV strain, primary antibodies against the VP6 protein of bovine species A rotavirus and an AF647-conjugated secondary antibody were reacted according to the aforementioned method. Subsequently, lipid droplets were stained with BODIPY. Thereafter, nuclei were stained with DAPI, followed by mounting.

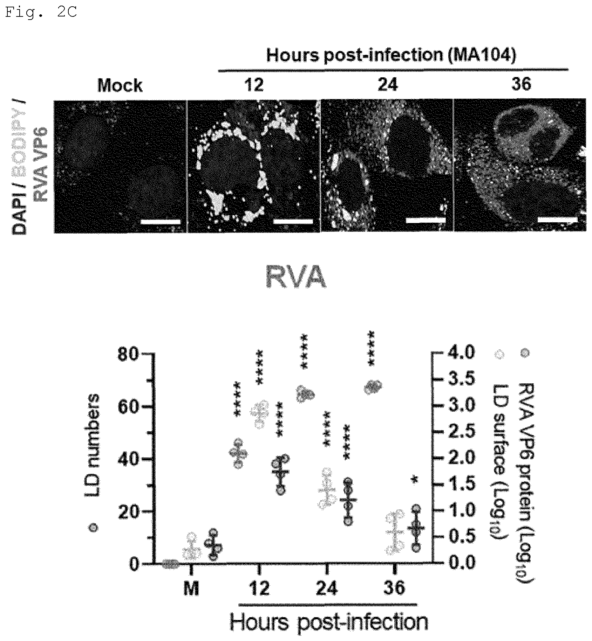

As can be seen from FIG. 2C, when the monolayer-cultured MA104 cell line was inoculated with or without bovine species A rotavirus NCDV strain, the viral antigen continuously increased until the end of the experiment at 36 hours post infection. However, the number of lipid droplets increased initially but decreased after 24 hours of infection.

For use in culturing porcine reproductive and respiratory syndrome virus, the African green monkey MARC-145 cells, purchased from the ATCC, were monolayer cultured in DMEM supplemented with 10% fetal bovine serum, 100 u/ml penicillin, and 100 ug/ml streptomycin.

To measure the titer of porcine reproductive and respiratory syndrome virus LMY strain, MARC-145 cells were monolayer cultured in 96-well plates (SPL Life Science, Pocheon, South Korea). In a 96-well plate separately prepared, DMEM was used as a diluent to generate ten-fold serial dilutions of the virus and the final well contained only DMEM medium without any virus. The medium was removed from the monolayer-cultured MARC-145 cells which were then washed with phosphate-buffered saline before inoculation with each of the serial virus dilutions or DMEM without virus. The virus was then allowed to adsorb to the cells for 1 hour in a 37°C CO2 incubator.

Subsequently, washing with phosphate-buffered saline was conducted before culturing the viruses in DMEM supplemented with 100 u/ml penicillin and 100 ug/ml streptomycin in a 37°C incubator. After 24 hours, the medium was removed from the 96-well plates where the viruses had been cultured. Washing with PBS was followed by fixation with 4% paraformaldehyde at 20°C for 10 minutes. An immunofluorescence assay was conducted using an antibody against porcine reproductive and respiratory syndrome virus matrix (Bioss Antibodies, Woburn, MA, USA) . The titer of porcine reproductive and respiratory syndrome virus LMY strain used in this disclosure was expressed in ffu/mL.

To this end, MARC-145 cells were monolayer-cultured in 8-well chambers (SPL) which were then washed with phosphate-buffered saline. The porcine reproductive and respiratory syndrome virus LMY strain with a multiplicity of infection (MOI) of 0.1 ffu/cell, as determined by immunofluorescence assay, was inoculated. The virus was adsorbed onto the cells at 37°C for 1 hour. The cells were then washed with phosphate-buffered saline and cultured in DMEM supplemented with the aforementioned antibiotics. The cells were incubated in a 37°C CO2 incubator for 24, 48, and 72 hours. The cells in each chamber were then washed with phosphate-buffered saline, fixed with 4% paraformaldehyde, and permeabilized with 0.2% Triton-X at 20°C for 10 minutes.

To observe changes in viral protein and lipid droplets due to the infection of porcine reproductive and respiratory syndrome virus LMY strain, primary antibodies against the matrix protein of porcine reproductive and respiratory syndrome virus and an AF647-conjugated secondary antibody were reacted according to the aforementioned method. Subsequently, lipid droplets were stained with BODIPY. Thereafter, nuclei were stained with DAPI, followed by mounting.

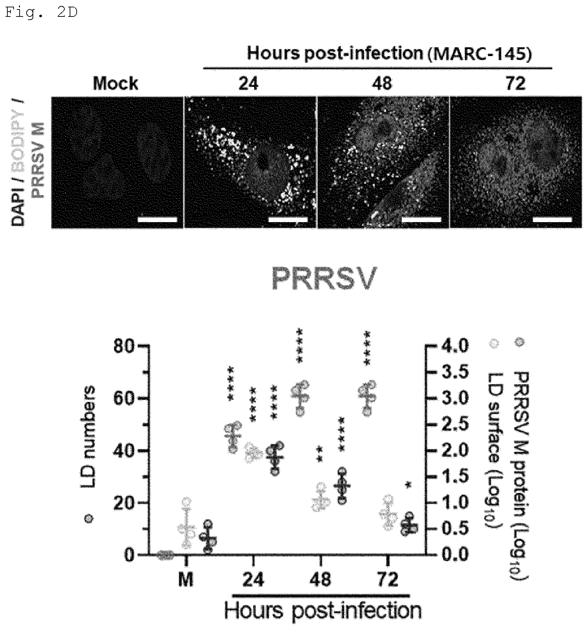

As can be seen from FIG. 2D, when the monolayer-cultured MARC-145 cell line was inoculated with or without porcine reproductive and respiratory syndrome virus LMY strain, the viral antigen continuously increased until the end of the experiment at 72 hours post infection. However, the number of lipid droplets increased initially but decreased after 48 hours of infection.

For use in culturing porcine sapovirus, the porcine kidney epithelium LLC-PK cells, purchased from the ATCC, were monolayer cultured in DMEM supplemented with 10% fetal bovine serum and 100 ug/ml streptomycin.

To measure the titer of porcine sapovirus Cowden strain, LLC-PK cells were monolayer cultured in 96-well plates. In a 96-well plate separately prepared, DMEM was used as a diluent to generate ten-fold serial dilutions of the virus and the final well contained only DMEM medium without any virus. The medium was removed from the monolayer-cultured LLC-PK cells which were then washed with phosphate-buffered saline before inoculation with each of the serial virus dilutions or DMEM without virus. The virus was then allowed to adsorb to the cells for 1 hour in a 37°C CO2 incubator.

Subsequently, washing with phosphate-buffered saline was conducted before culturing the viruses in DMEM supplemented with 200 µM GCDCA (Sigma Aldrich), 100 u/ml penicillin, and 100 ug/ml streptomycin in a 37°C incubator. After 24 hours, the medium was removed from the 96-well plates where the viruses had been cultured. Washing with PBS was followed by fixation with 4% paraformaldehyde at 20°C for 10 minutes. An immunofluorescence assay was conducted using an antibody (in-house prepared) against porcine sapovirus VPg protein. The titer of porcine sapovirus Cowden strain used in this disclosure was expressed in ffu/mL.

To this end, LLC-PK cells were monolayer-cultured in 8-well chambers (SPL) which were then washed with phosphate-buffered saline. The porcine sapovirus Cowden with a multiplicity of infection (MOI) of 0.1 ffu/cell, as determined by immunofluorescence assay, was inoculated. The virus was adsorbed onto the cells at 37°C for 1 hour. The cells were then washed with phosphate-buffered saline and cultured in EMEM supplemented with 3 ug/mL porcine pancreatic trypsin and the aforementioned antibiotics. The cells were incubated in a 37°C CO2 incubator for 18, 36, and 54 hours. The cells in each chamber were then washed with phosphate-buffered saline, fixed with 4% paraformaldehyde, and permeabilized with 0.2% Triton-X at 20°C for 10 minutes.

To observe changes in viral protein and lipid droplets due to the infection of porcine sapovirus Cowden strain, primary antibodies against the VPg protein of porcine sapovirus and an AF647-conjugated secondary antibody were reacted according to the aforementioned method. Subsequently, lipid droplets were stained with BODIPY. Thereafter, nuclei were stained with DAPI, followed by mounting.

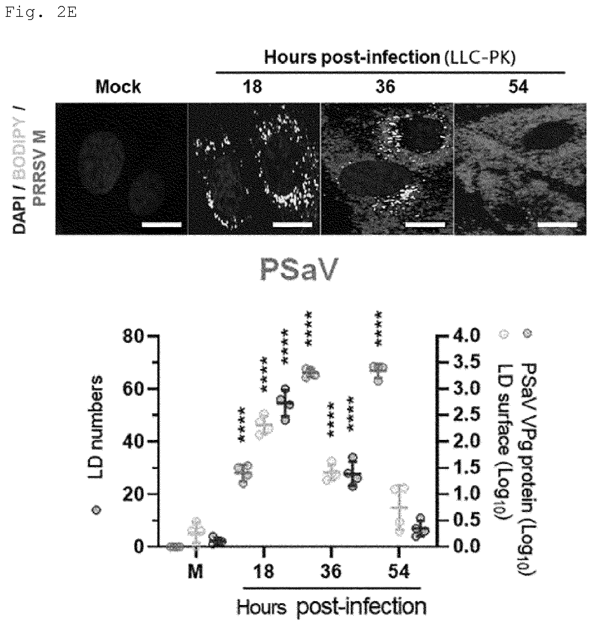

As can be seen from FIG. 2E, when the monolayer-cultured LLC-PK cell line was inoculated with or without porcine sapovirus Cowden strain, the viral antigen continuously increased until the end of the experiment at 54 hours post infection. However, the number of lipid droplets increased initially but decreased after 36 hours of infection.

### 1-3. Change of cytoplasmic lipid droplet in alveolar epithelial cells from SARS-CoV-2-infected hamster and bronchiolar epithelial cells from influenza A virus (IVA)-infected mice

It was examined whether the changes of lipid droplets observed in the virus-infected cultured cells also manifest in animals infected by the virus. In this regard, hamsters were challenged with the SARS-CoV-2 KCDC03 strain, or mice were challenged with mouse-adapted influenza A virus PR8 strain. Lung tissues were collected at different time points and the following experiments were conducted:

All procedures and experiments were conducted in a manner that minimized the number of animals used, and the suffering caused. In the animal experiments, all procedures were performed in accordance with the institutional animal care and use committees' requirements at Chonnam National University (CNU IACUC-YB-2018-41) and the Korea Research Institute of Bioscience & Biotechnology (approval no.: KRIBB-AEC-21203, KRIBB-IBC-20210206).

As test animals, female golden Syrian hamsters 11 weeks old were purchased from Janvier Labs (Saint-Berthevin, France) before seven days before the start of the experiment. The hamsters were kept in standard cages and exposed to a 12:12 hour light/dark cycle at 22-24°C and 40-55% humidity with solid foods and sterilized water ad libitum in a facility. Blood was collected from the orbital venous plexus of representative hamsters and tested for antibodies to SARS-CoV-2 using ELISA. As a result, no antibodies to SARS-CoV-2 were detected in the blood.

To determine the titer of SARS-CoV-2 KCDC03 strain, a Median Tissue Culture Infectious Dose assay (TCID50) was performed. Initially, a 96-well plate was prepared, and the virus was serially diluted by tenfold in DMEM, leaving only virus-free DMEM in the last well. The medium was removed from monolayer cultured Vero E6 cells in the 96-well plate, and after washing with phosphate-buffered saline (PBS), each serially diluted virus or virus-free DMEM was inoculated onto the Vero E6 cells and incubated for 1 hour at 37°C in a CO2 incubator to allow viral adsorption. Following another wash with PBS, EMEM supplemented with 1 ug/ml TPCK-treated trypsin, 100 u/ml penicillin, and 100 ug/ml streptomycin was added, and the cells were incubated at 37°C in a CO2 incubator for 96 hours before recording the cytopathic effect. The titer of SARS-CoV-2 KCDC03 strain was then calculated as TCID50/ml using the Reed and Muench method.

To observe lung lesions in hamsters caused by SARS-CoV-2, 12-week-old female Golden Syrian hamsters were anesthetized with a combination of xylene and Zoletil, and then inoculated intratracheally with either 100 µL of PBS or 100 µL of PBS containing 105 TCID50 of the virus.

After infection, three hamsters were euthanized daily on days 0 (negative control group), 2, 4, 6, 8, and 10, followed by a necropsy. The left lobe of the extracted lungs was fixed at room temperature for a week in 4% paraformaldehyde for histopathological analysis and for preparing frozen sections. The right cranial, middle, caudal, and accessory lobes were all combined together to form a homogenate used for measuring intracellular free fatty acids, triglycerides, and glycerol, and for evaluating the presence of SARS-CoV-2 Spike protein and hormone-sensitive lipase.

For each experimental animal, wild-type C57BL/6J mice were procured from Samtako (Osan, South Korea) seven days before the experiment commenced. The mice were kept in standard cages and exposed to a 12:12 hour light/dark cycle at 25°C with solid food and sterilized water ad libitum in a specific-pathogen-free facility. Blood was collected from the orbital venous plexus of representative mice and tested for antibodies to influenza A virus using ELISA. As a result, no antibodies to influenza A viruswere detected in the blood.

To determine the 50% lethal dose (LD50) of the influenza A virus PR8 strain, 8-week-old female wild-type C57BL/6J mice were used. The experimental groups were divided into five categories: a non-inoculated control group, and groups inoculated with 10¹ PFU, 10² PFU, 10³ PFU, and 10⁴ PFU of the virus, respectively. Mice in each experimental group were anesthetized with a mixture of xylene and zoletil, then administered 50 µL of phosphate-buffered saline (PBS) or the same volume of PBS containing the mentioned virus doses via intranasal administration. Over the following 15 days, survival rate, body weight, and clinical symptoms were recorded, and the number of deceased mice was noted. The LD50 value was calculated using the Reed and Muench method.

To investigate lung lesions induced in mice by the influenza A virus, a dose 20 times the LD50 (10,000 PFU) was administered to 8-week-old female wild-type C57BL/6J mice. After infection, mice were euthanized and necropsied daily on day 0 (negative control group), 1, 2, 3, 4, 5, 6, and 7, with 4 mice each day. The left lung lobe was fixed in 4% paraformaldehyde at room temperature for a week for histopathological analysis and cryosectioning. The right anterior, middle, posterior, and accessory lobes were combined to create a homogenate, which was then used for the measurement of free fatty acids, triglycerides, glycerol in the cytoplasm, and assessment of influenza A virus PB1 protein and hormone-sensitive lipase.

To evaluate lipid droplets and SARS-CoV-2 or influenza A virus antigens in the lung tissues, 3 µm cryosections were prepared from the fixed tissues. After blocking with 5% BSA, the sections were incubated with primary antibodies against the nucleocapsid (N) of SARS-CoV-2 or the M2 (membrane ion channel) antigen of influenza A virus at 4°C for 16 hours, followed by incubation with AF594-conjugated mouse IgG (H+L) secondary antibodies at 20°C for one hour. The tissue sections were then washed with phosphate-buffered saline, stained with 10 µM BODIPY for 15 minutes at 20°C for lipid droplet staining. Following another wash, the sections were mounted with Slow-Fade Gold antifade reagent for nuclear staining.

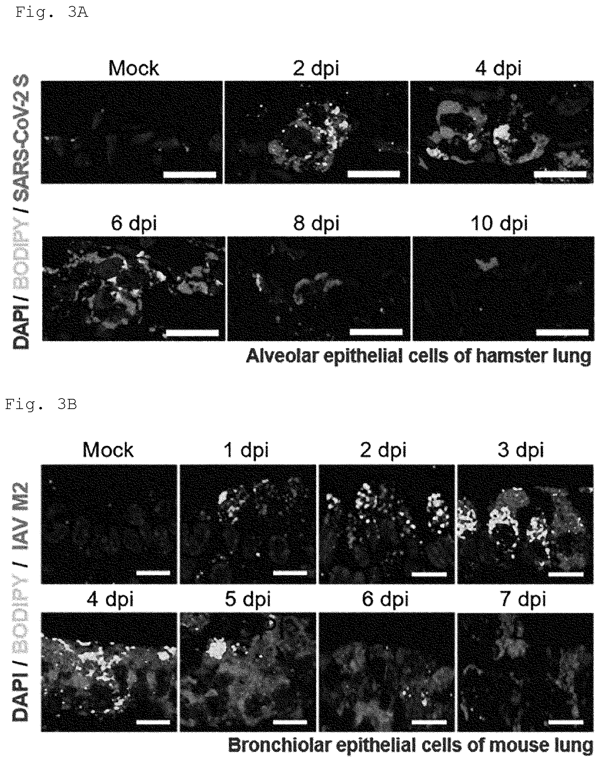

As evident in FIGS. 3a and 3b, lipid droplets in the alveolar epithelial cells of hamsters infected with SARS-CoV-2 increased from the onset of infection until day 4, then decreased from day 6 onwards. Similarly, lipid droplets in bronchiolar epithelial cells of mice infected with influenza A virus increased from the onset of infection until day 4, then decreased from day 5 onwards.

To perform triglyceride assays in lung tissues collected from the hamsters and mice, 100 mg of lung tissue was combined with 500 µL of NP40 Substitute Assay Reagent and homogenized using a sonicator on ice with an amplitude setting of 40%, in a cycle of 2 seconds on/10 seconds off, repeated 20 times. After centrifugation, the supernatant was collected. This supernatant was diluted five-fold with NP40 Substitute Assay Reagent, and 10 µL of the diluted sample was transferred to a 96-well plate. Then, 150 µL of triglyceride enzyme mixture was added to each well and incubated at 20°C for 15 minutes. The absorbance was read at 570 nm on an ELISA reader.

Similarly, to perform cholesterol assays in lung tissues collected from these hamsters and mice, 100 mg of lung tissue was processed using the same method as mentioned for intracellular cholesterol assay, and the absorbance was read at 570 nm using an ELISA reader.

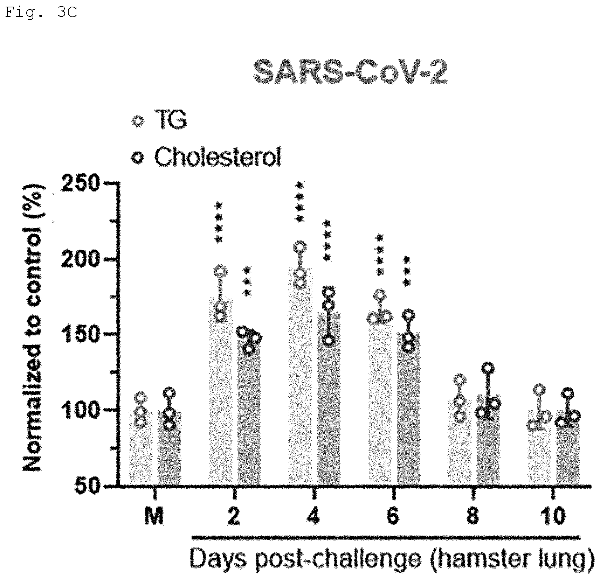

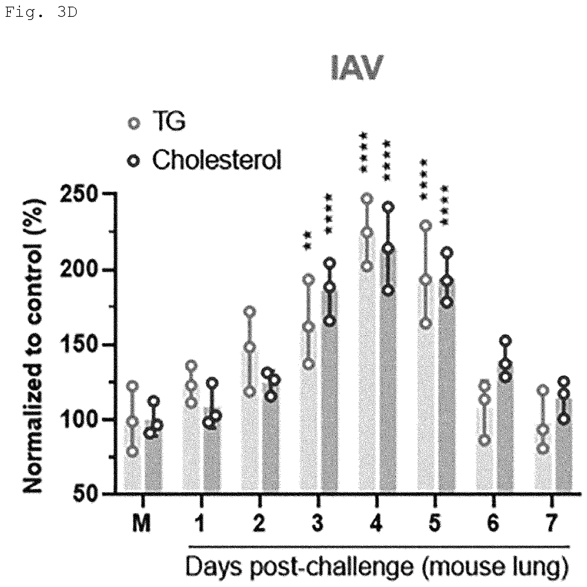

As seen in FIG. 3C and 3D, both triglyceride and cholesterol levels in the lungs of hamsters infected with SARS-CoV-2 increased from the onset of infection until day 4, then decreased onwards. Similarly, triglyceride and cholesterol levels in the lungs of mice infected with influenza A virus increased from the onset of infection until day 4, and then decreased thereafter.

### EXPERIMENTAL EXAMPLE 2: Changes in Cytoplasmic Hormone-Sensitive Lipase

### 2-1. Changes of cytoplasmic hormone-sensitive lipase in cultured cells due to SARS-CoV-2 and influenza A virus (IAV) infection

As described in Experimental Example 1, Vero E6 cells infected with the SARS-CoV-2 KCDC03 strain and A549 cells infected with the influenza A virus PR8 strain were prepared.

It was examined whether the late-stage activation of hormone-sensitive lipase (HSL) induced lipolysis in the lipid droplets formed by SARS-CoV-2. In this regard, Vero E6 cells monolayer cultured in 8-well chambers were infected with SARS-CoV-2 KCDC03 strain at a multiplicity of infection (MOI) of 0.1 ffu/cell, incubated at 37°C in a CO2 incubator for 4, 8, 12, 18, 24, 36, and 48 hours, and then fixed with 4% paraformaldehyde for immunofluorescence staining.

Similarly, to observe whether the late-stage activation of hormone-sensitive lipase induced lipolysis in lipid droplets formed by influenza A virus infection, A549 cells monolayer cultured in 8-well chambers were infected with the influenza A virus PR8 strain at an MOI of 1 ffu/cell, incubated at 37°C in a CO2 incubator for 1, 4, 8, 12, 18, 24, and 36 hours, and then fixed with 4% paraformaldehyde for immunofluorescence staining.

The fixed cells were washed with phosphate-buffered saline (PBS), blocked with 5% BSA at 20°C for 1 hour, and then incubated with primary antibodies against phosphorylated hormone-sensitive lipase at serine 563 (S563) and against lipid droplet-associated protein perilipin3 (PLIN3) at 4°C for 16 hours. The next day, the cells were washed with PBS and incubated with a secondary antibody conjugated with Alexa Fluor (AF) 488 or AF594 at 37°C for 1 hour. The cells were mounted using Slow-Fade Gold antifade reagent with DAPI for nucleus staining.

The changes in lipolysis over time after viral infection were analyzed in the same manner as in Experimental Example 1-1. Captured images were converted to pixel units using Adobe Photoshop PS6, and the area of colocalization signals of phosphorylated hormone-sensitive lipase/PLIN3 was calculated and expressed as the mean ± standard error for evaluation.

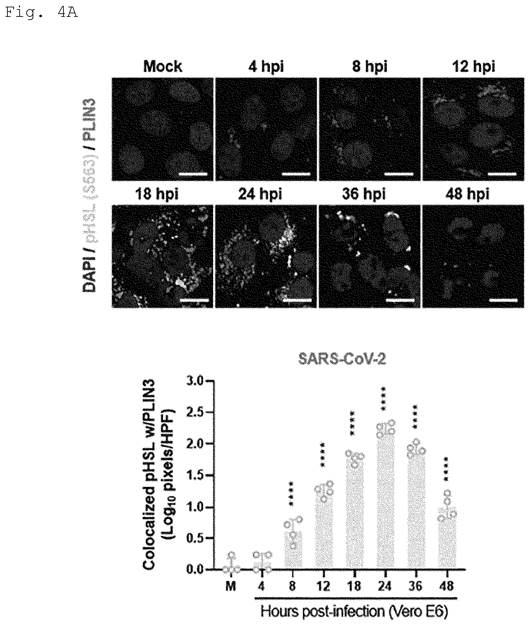

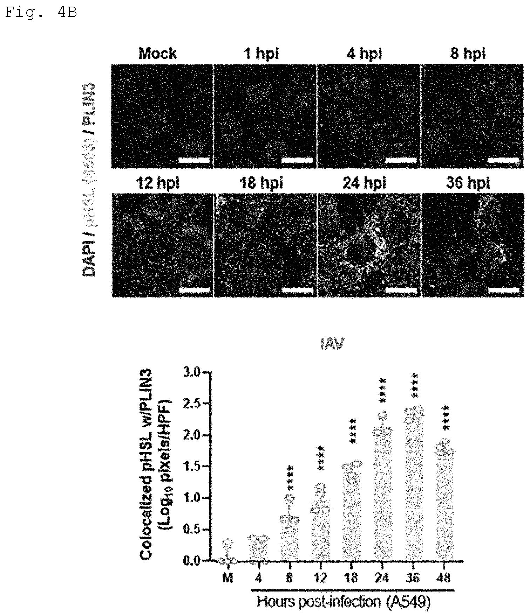

As seen in FIGS. 4a and 4b, phosphorylated HSL colocalized with lipid droplet surface marker PLIN3 in SARS-CoV-2-infected Vero E6 cells and influenza A virus-infected A549 cells was increased from the mid-stage of infection, indicating that phosphorylated HSL induces the breakdown of lipid droplets from the mid-stage of infection.

To determine the levels of cytoplasmic free fatty acids (FFA) and glycerol, monolayer-cultured Vero E6 cells in 100π culture dishes were infected with SARS-CoV-2 KCDC03 at an MOI of 0.1 ffu/cell and incubated in a 37°C CO2 incubator for 4, 8, 12, 18, 24, 36, and 48 hours, as described in the previous experiment. Cells were then detached using trypsin and harvested.

Similarly, monolayer-cultured A549 cells in 100π culture dishes were infected with influenza A virus PR8 at an MOI of 1 ffu/cell and incubated for 1, 4, 8, 12, 18, 24, and 36 hours before detachment and harvesting with trypsin. The harvested cells were quantified using a hemocytometer and 5 × 106 cells were aliquoted into each ep-tube. After centrifugation, the cell pellets were washed with phosphate-buffered saline and used for the analysis of free fatty acid and glycerol content.

The Fatty Acid Colorimetric Assay Kit was purchased from BioVision (Milpitas, CA, USA), and used according to the manufacturer's instructions. The cell pellet was added with 500 µL of chloroform and 1% Triton X-100 and homogeneously mixed by vortexing every 5 minutes. The organic phase was then collected, and air-dried, and the resulting pellet was dissolved in 400 µL of Fatty Acid Assay Buffer. After vigorous vortexing for 5 minutes, 50 µL of the extracted supernatant and 2 µL of ACS Reagent were mixed and transferred to the corresponding wells of a 96-well plate. As a positive control, 50 µL of the standard fatty acid solution provided and 2 µL of ACS Reagent were also mixed and transferred to the corresponding wells of the plate. Each well was then supplemented with 50 µL of Enzyme Mix and incubated at 37°C for 30 minutes. Absorbance was read at 570 on an ELISA reader. The fatty acid concentration in each sample was determined using a standard curve obtained from the standard fatty acid solution, according to the manufacturer's instructions.

The Glycerol Colorimetric Assay Kit was purchased from BioVision (Milpitas, CA, USA), and used according to the manufacturer's instructions. The cell pellet was mixed with Glycerol Assay Buffer and vigorously vortexed at 5-minute intervals for 20 minutes. After centrifugation, 50 µL of the supernatant from each sample was dispensed into the corresponding wells of 96-well plates. Additionally, 50 µL of the standard glycerol solution provided as a positive control was also dispensed into the corresponding wells of 96-well plates. Then, 50 µL of Reaction Mixture was added to each well containing the samples and the positive control and incubated at room temperature for 30 minutes. The absorbance was read at 570 nm on an ELISA reader, and the glycerol concentration in each sample was determined using a standard curve obtained from the standard glycerol solution, according to the manufacturer's instructions.

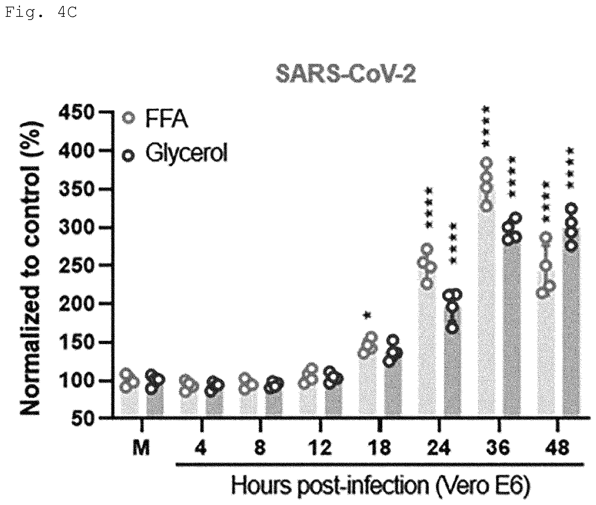

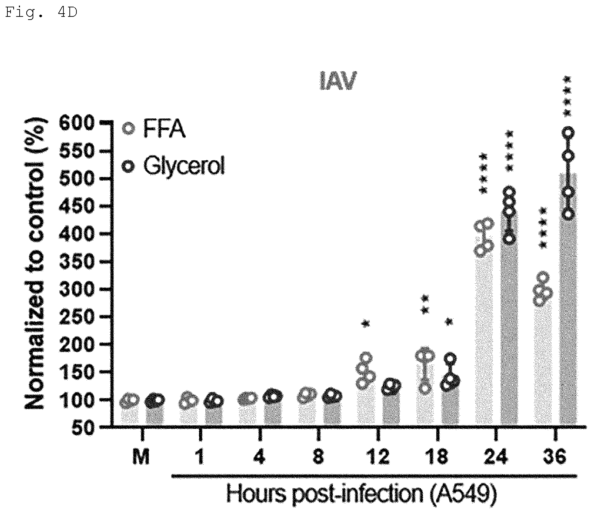

As shown in FIGS. 4c and 4d, the levels of cytoplasmic free fatty acids and glycerol in Vero E6 cells infected with SARS-CoV-2 significantly increased at 24, 36, and 48 hours post-infection. Similarly, the levels of cytoplasmic free fatty acids and glycerol in A549 cells infected with influenza A virus showed a significant increase at 24 and 36 hours post-infection. This suggests that the degradation of lipid droplets in the cytoplasm increased during the late stage of infection by SARS-CoV-2 and influenza A virus, leading to an increase in the final breakdown products of lipid droplets, namely free fatty acids and glycerol.

### 2-2. Changes of cytoplasmic hormone-sensitive lipase in cultured cells due to bovine coronavirus (BCoV), porcine epidemic diarrhea coronavirus (PEDV), bovine species A rotavirus (RVA), porcine reproductive and respiratory syndrome virus (PRRSV), and porcine sapovirus (PSaV) infection

To evaluate the changes in lipid droplets and hormone-sensitive lipase (HSL) following infection with the bovine coronavirus KWD20 strain, HRT-18G cells were monolayer cultured in 8-well chambers (SPL) and then washed with phosphate-buffered saline (PBS). The cells were then infected with the bovine coronavirus KWD20 strain at a multiplicity of infection (MOI) of 0.1 ffu/cell, as determined by immunofluorescence antibody assay. The virus was allowed to adsorb to the cells at 37°C for 1 hour and then washed with PBS. The cells were then incubated in DMEM supplemented with 5 µg/mL pancreatin and the aforementioned antibiotics at 37°C in a CO2 incubator for 18, 36, and 54 hours. Subsequently, the cells in the chambers were washed with PBS, fixed with 4% paraformaldehyde, and then permeabilized with 0.2% Triton-X at 20°C for 10 minutes. Thereafter, the cells were incubated with a primary antibody against phosphorylated hormone-sensitive lipase (S563) and a secondary antibody conjugated with AF647. Lipid droplets were stained with BODIPY, and the nuclei were stained with DAPI, followed by mounting.

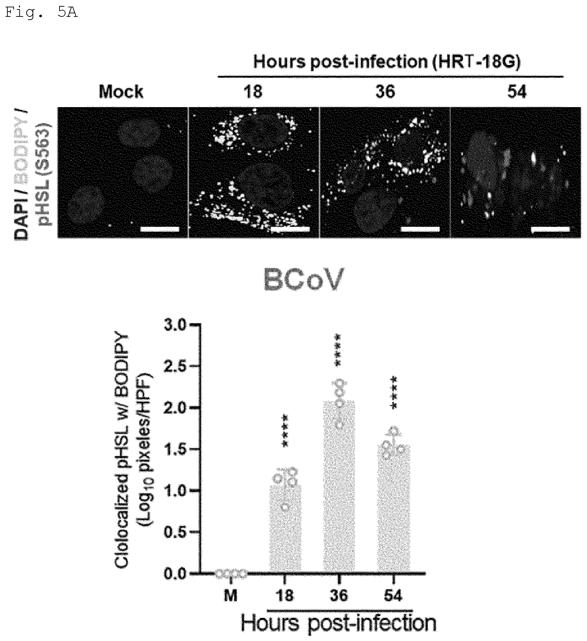

As shown in FIG. 5A, the number of lipid droplets in monocultured HRT-18G cells infected with or without the bovine coronavirus KWD20 strain decreased after 36 hours of infection. In contrast, the level of phosphorylated HSL peaked at 36 hours post-infection.

To evaluate the changes in lipid droplets and hormone-sensitive lipase (HSL) following infection with the porcine epidemic diarrhea coronavirus Q1401 strain, Vero E6 cells were monolayer cultured in 8-well chambers (SPL) and then washed with phosphate-buffered saline (PBS). The cells were then infected with the porcine epidemic diarrhea coronavirus Q1401 strain at a multiplicity of infection (MOI) of 0.1 ffu/cell, as determined by immunofluorescence antibody assay. The virus was allowed to adsorb to the cells at 37°C for 1 hour and then washed with PBS. The cells were then incubated in EMEM supplemented with 3 ug/mL porcine pancreatic trypsin and the aforementioned antibiotics at 37°C in a CO2 incubator for 18, 36, and 54 hours. Subsequently, the cells in the chambers were washed with PBS, fixed with 4% paraformaldehyde, and then permeabilized with 0.2% Triton-X at 20°C for 10 minutes. Thereafter, the cells were incubated with a primary antibody against phosphorylated hormone-sensitive lipase (S563) and a secondary antibody conjugated with AF647. Lipid droplets were stained with BODIPY, and the nuclei were stained with DAPI, followed by mounting.

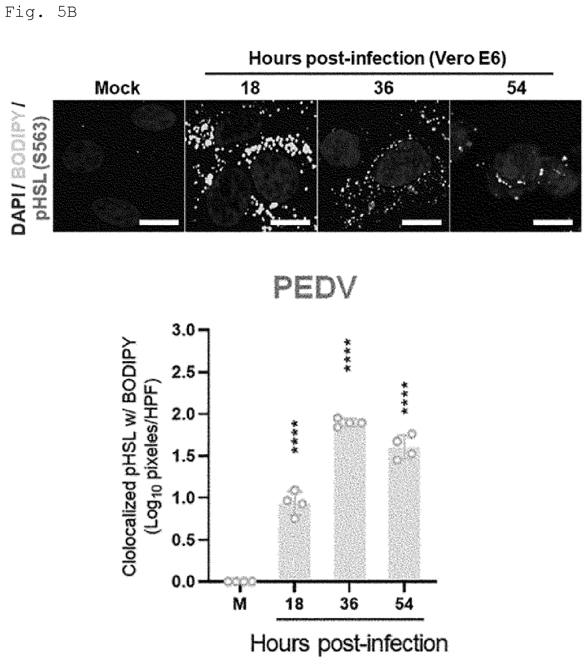

As shown in FIG. 5B, the number of lipid droplets in monocultured Vero E6 cells infected with or without the porcine epidemic diarrhea coronavirus QIAP1401 strain decreased after 36 hours of infection while the viral antigen continued to increase until 36 hours. In contrast, the level of phosphorylated HSL peaked at 36 hours post-infection.

To evaluate the changes in lipid droplets and hormone-sensitive lipase (HSL) following infection with the bovine species A rotavirus NCDVstrain, MA104 cells were monolayer cultured in 8-well chambers (SPL) and then washed with phosphate-buffered saline (PBS). The cells were then infected with the bovine species A rotavirus NCDV strain at a multiplicity of infection (MOI) of 0.1 ffu/cell, as determined by immunofluorescence antibody assay. The virus was allowed to adsorb to the cells at 37°C for 1 hour and then washed with PBS. The cells were then incubated in DMEM supplemented with 5 µg/mL pancreatin and the aforementioned antibiotics at 37°C in a CO2 incubator for 12, 24, and 36 hours. Subsequently, the cells in the chambers were washed with PBS, fixed with 4% paraformaldehyde, and then permeabilized with 0.2% Triton-X at 20°C for 10 minutes. Thereafter, the cells were incubated with a primary antibody against phosphorylated hormone-sensitive lipase (S563) and a secondary antibody conjugated with AF647. Lipid droplets were stained with BODIPY, and the nuclei were stained with DAPI, followed by mounting.

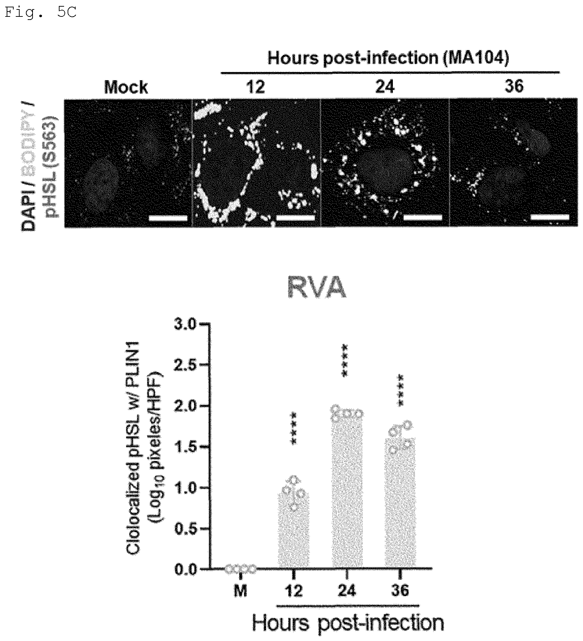

As shown in FIG. 5C, the number of lipid droplets in monocultured MA104 cells infected with or without the bovine species A rotavirus NCDV strain decreased after 24 hours of infection. In contrast, the level of phosphorylated HSL peaked at 24 hours post-infection.

To evaluate the changes in lipid droplets and hormone-sensitive lipase (HSL) following infection with the porcine reproductive and respiratory syndrome virus LYM strain, MARC-145cells were monolayer cultured in 8-well chambers (SPL) and then washed with phosphate-buffered saline (PBS). The cells were then infected with the porcine reproductive and respiratory syndrome virus LYM strain at a multiplicity of infection (MOI) of 0.1 ffu/cell, as determined by immunofluorescence antibody assay. The virus was allowed to adsorb to the cells at 37°C for 1 hour and then washed with PBS. The cells were then incubated in DMEM supplemented with the aforementioned antibiotics at 37°C in a CO2 incubator for 24, 48, and 72 hours. Subsequently, the cells in the chambers were washed with PBS, fixed with 4% paraformaldehyde, and then permeabilized with 0.2% Triton-X at 20°C for 10 minutes. Thereafter, the cells were incubated with a primary antibody against phosphorylated hormone-sensitive lipase (S563) and a secondary antibody conjugated with AF647. Lipid droplets were stained with BODIPY, and the nuclei were stained with DAPI, followed by mounting.

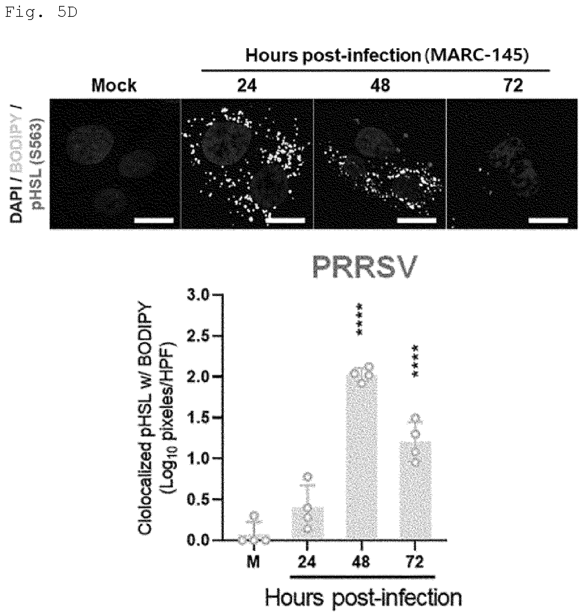

As shown in FIG. 5D, the number of lipid droplets in monocultured MARC-145 cells infected with or without the porcine reproductive and respiratory syndrome virus LYM strain decreased after 48 hours of infection. In contrast, the level of phosphorylated HSL peaked at 48 hours post-infection.

To evaluate the changes in lipid droplets and hormone-sensitive lipase (HSL) following infection with the porcine sapovirus Cowden strain, LLC-PK cells were monolayer cultured in 8-well chambers (SPL) and then washed with phosphate-buffered saline (PBS). The cells were then infected with the porcine sapovirus Cowden strain at a multiplicity of infection (MOI) of 0.1 ffu/cell, as determined by immunofluorescence antibody assay. The virus was allowed to adsorb to the cells at 37°C for 1 hour and then washed with PBS. The cells were then incubated in DMEM supplemented with 3 ug/mL porcine pancreatic trypsin, 200 µM glycochenodeoxycholic acid (GCDCA), and the aforementioned antibiotics at 37°C in a CO2 incubator for 18, 36, and 54 hours. Subsequently, the cells in the chambers were washed with PBS, fixed with 4% paraformaldehyde, and then permeabilized with 0.2% Triton-X at 20°C for 10 minutes. Thereafter, the cells were incubated with a primary antibody against phosphorylated hormone-sensitive lipase (S563) and a secondary antibody conjugated with AF647. Lipid droplets were stained with BODIPY, and the nuclei were stained with DAPI, followed by mounting.

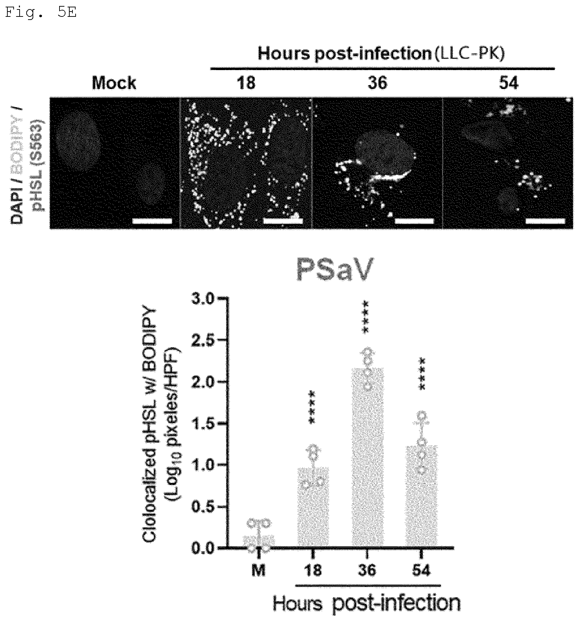

As shown in FIG. 5E, the number of lipid droplets in monocultured LLC-PK cells infected with or without the porcine sapovirus Cowden strain decreased after 36 hours of infection. In contrast, the level of phosphorylated HSL peaked at 36 hours post-infection.

The results of the immunofluorescence antibody assay for lipid droplets and hormone-sensitive lipase following each viral infection were evaluated using the same method as in Experimental Example 1-1. The images captured were converted to pixel units using Adobe Photoshop PS6, and the area of positive signals for colocalization of phosphorylated hormone-sensitive lipase (S563)/BODIPY was calculated and expressed as mean ± standard error.

In conclusion, similar to SARS-CoV-2 and influenza A virus, bovine coronavirus, porcine epidemic diarrhea coronavirus, bovine species A rotavirus, porcine reproductive and respiratory syndrome virus, and porcine sapovirus also showed an increase in the number of lipid droplets in cultured infected cells until the mid-stage of infection, followed by a decrease in the later stages. The decrease in the number of lipid droplets was due to the lipolytic enzyme, phosphorylated HSL, activated from the mid-stage of infection onwards.

### 2-3. Changes of hormone-Sensitive Lipase in alveolar epithelial cells of hamsters and bronchial epithelial cells of mice due to infection with SARS-CoV-2 and IAV

As described in the foregoing, hamsters inoculated with 10⁵ TCID50 of the SARS-CoV-2 KDCD03 strain were euthanized for autopsy on days 0 (negative control), 2, 4, 6, 8, and 10 post-infection, three animals each day. The left lobes of the extracted lungs were fixed at room temperature for one week in 4% paraformaldehyde for histopathological analysis and for the preparation of frozen tissue sections. The right anterior, middle, posterior, and accessory lobes were combined to create a homogenate, which was then used for the measurement of free fatty acids and glycerol in the cytoplasm, and assessment of SARS-CoV-2 Spike protein and hormone-sensitive lipase.

As described in the foregoing, mice inoculated with 1,000 PFU of influenza A virus were also euthanized for autopsy on days 0 (negative control), 1, 2, 3, 4, 5, 6, and 7 post-infection, four animals each day. The left lobes of the extracted lungs were fixed in 4% paraformaldehyde at room temperature for one week for histopathological analysis and the preparation of cryosections. The right anterior, middle, posterior, and accessory lobes were combined to form a homogenate which was then used for the measurement of cytoplasmic free fatty acids and glycerol and for the evaluation of influenza A virus PB1 protein and hormone-sensitive lipase.

For the assessment of phosphorylated hormone-sensitive lipase (S563) and the antigens of either SARS-CoV-2 or influenza A virus in the lung tissues, the fixed tissues were prepared into 3-µm thick cryosections. These sections were blocked with 5% BSA, incubated with primary antibodies against SARS-CoV-2 nucleocapsid (N) or influenza A virus M2 (membrane ion channel) antigen at 4°C for 16 hours, followed by a reaction with AF594-conjugated mouse IgG(H+L) secondary antibody at 20°C for one hour. The tissue sections were then washed with phosphate-buffered saline, reacted with 10 µM BODIPY for 15 minutes at 20°C for lipid droplet staining. After washing, the sections were and mounted with Slow-Fade Gold antifade reagent for nuclear staining.

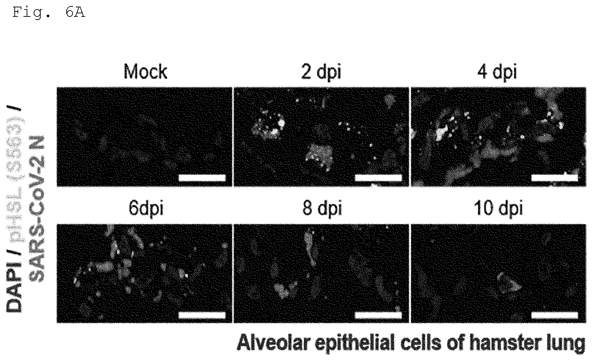

As can be seen in FIG. 6A, both phosphorylated hormone-sensitive lipase (HSL) and the SARS-CoV-2 nucleocapsid (N) in the alveolar epithelial cells of hamsters infected with SARS-CoV-2 significantly increased on days 2 and 4 post-infection, compared to the control group inoculated without the virus, and then decreased from day 6 post-infection.

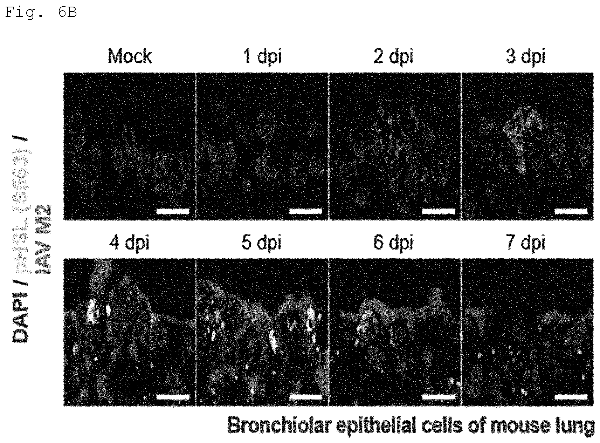

Additionally, as seen in FIG. 6B, both phosphorylated hormone-sensitive lipase and influenza A virus M2 protein in the bronchial epithelial cells from mice infected with influenza A virus markedly increased until days 5 and 6 post-infection compared to the virus-non-inoculated control group, and then decreased on day 7.

To perform a Western blot analysis for the examination of viral and cellular proteins, lung tissues from hamsters or mice (100 mg each) were lysed in RIPA buffer containing protease and phosphatase inhibitors. The lysate was added with beads and homogenized using a Precellys 23 instrument homogenizer, followed by centrifugation to collect the supernatant. The total protein content in the supernatant was quantified using a BCA Assay Kit (Thermo Scientific), and the quantified proteins were subjected to electrophoresis on an SDS-PAGE (sodium dodecyl sulfate-polyacrylamide gel).

The resolved proteins were transferred to a nitrocellulose membrane (GE Healthcare Life Sciences), and the membrane was blocked in 5% skim milk solution at room temperature for 1 hour, then washed with TBST, and incubated at 4°C for 16 hours with primary antibodies against SARS-CoV-2 Spike, influenza A virus PB1, phosphorylated hormone-sensitive lipase at serine 563 (S563), phosphorylated hormone-sensitive lipase at serine 660 (S660), total hormone-sensitive lipase, host GAPDH protein, or host β-actin protein.

On the following day, HRP (horse radish peroxidase)-conjugated secondary antibodies against mouse or rabbit IgG were applied at 20°C for one hour. The HRP enzymatic reaction was amplified using a chemiluminescence reaction kit (DoGen, Seoul, South Korea), and the reactions for each target protein were measured using a Davinch-Western Imaging System (Young Ltd., Seoul, South Korea). To ensure equal amounts of protein were analyzed, the transferred membranes were also probed with antibodies against GAPDH or β-actin, and the intensity of the target proteins was compared. Furthermore, the immunoreactivity intensity of the target phosphorylated hormone-sensitive lipase was quantified using ImageJ and compared with the intensity of hormone-sensitive lipase in a graphical representation.

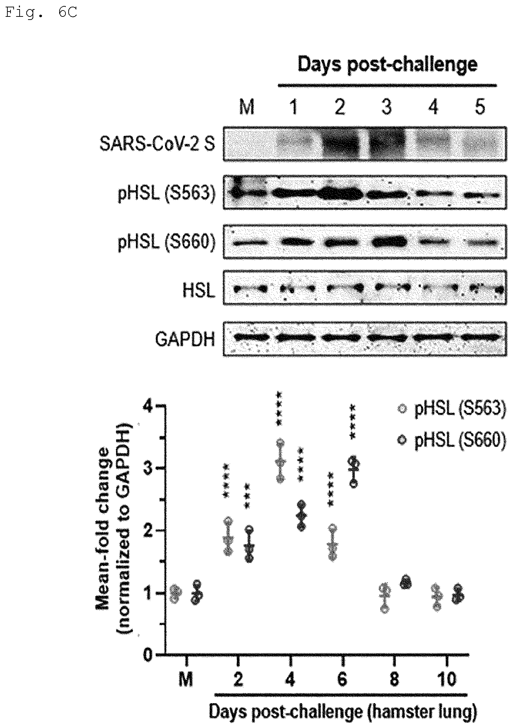

As shown in FIG. 6C, the expression levels of phosphorylated hormone-sensitive lipase and SARS-CoV-2 spike protein in alveolar epithelial cells of hamsters infected with SARS-CoV-2 rapidly increased from day 2 post-infection and were maintained highly until day 4.

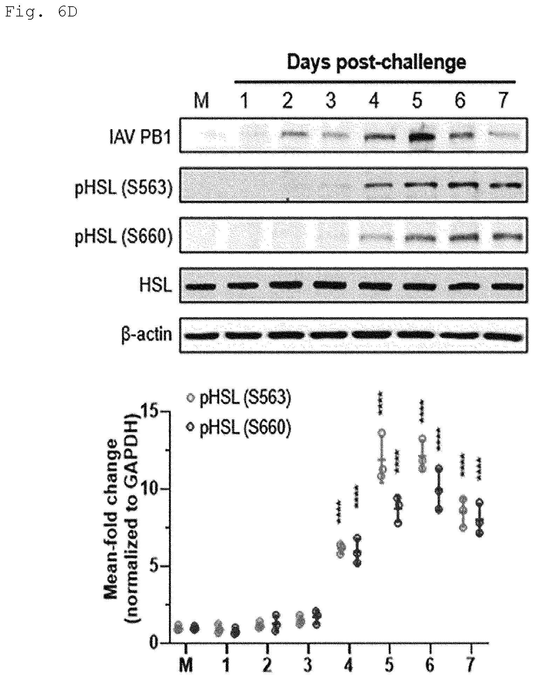

Similarly, as depicted in FIG. 6D, the expression levels of phosphorylated hormone-sensitive lipase and influenza A virus PB1 protein in bronchial epithelial cells of mice infected with influenza A virus showed a rapid increase from day 4 post-infection, remaining high until day 7.

To analyze free fatty acids in tissues collected from the hamsters and mice, the tissues were mixed with chloroform containing 1% Triton-X 100 and then processed in the same way as the intracellular free fatty acid assay. The absorbance was read at 570 nm on an ELISA reader.

For a glycerol assay, tissues collected from the hamsters and mice were mixed with glycerol assay buffer and processed in the same manner as in the intracellular glycerol assay. The absorbance was read at 570 nm on an ELISA reader.

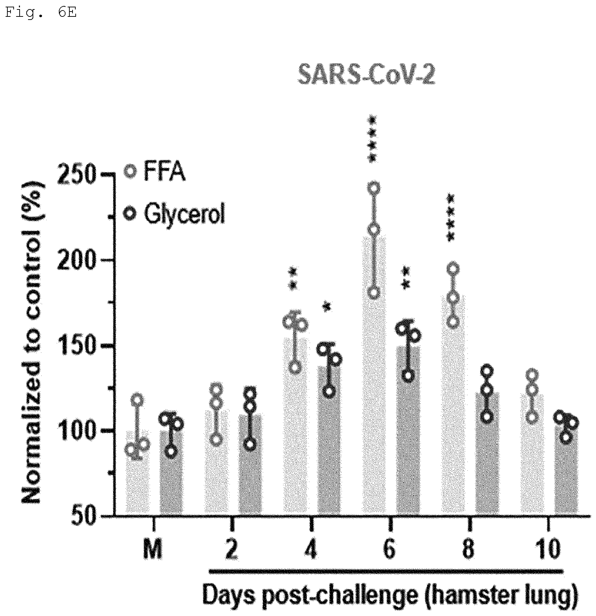

As shown in FIG. 6E, both free fatty acids and glycerol levels in the lungs of hamsters infected with SARS-CoV-2 increased until day 6 post-infection.

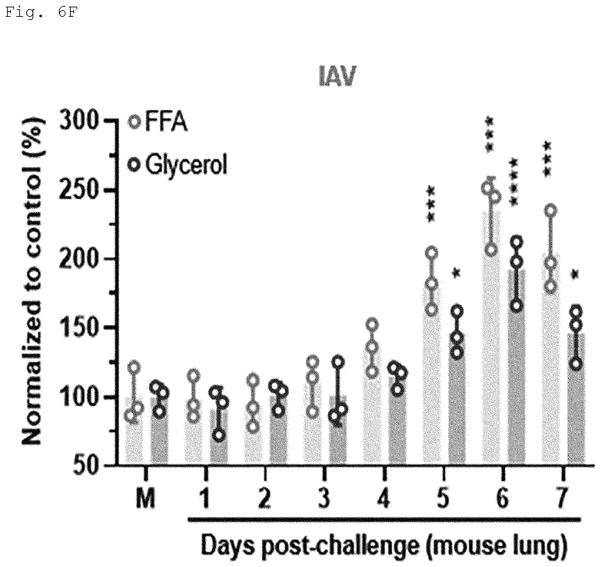

Similarly, as indicated in FIG. 6F, both free fatty acids and glycerol levels in the lungs of mice infected with influenza A virus increased until day 6 post-infection.

In conclusion, the decrease in lipid droplets in alveolar epithelial cells of hamsters infected with SARS-CoV-2 and in bronchial epithelial cells of mice infected with influenza A virus during the middle and late stages of infection was attributed to the activation of lipolytic enzymes such as phosphorylated HLS. This enzymatic breakdown of lipid droplets led to an increase in free fatty acids and glycerol.

### EXPERIMENTAL EXAMPLE 3: Changes in Cytoplasmic Free Fatty Acids and Glycerol in Hamster Alveolar Epithelial Cells Infected with Severe Acute Respiratory Syndrome Coronavirus 2 (SARS-CoV-2), and Cultured Cells Infected with Influenza A Virus (IAV), Bovine Coronavirus (BCoV), Porcine Epidemic Diarrhea Coronavirus (PEDV), Bovine Species A Rotavirus (RVA), Porcine Reproductive and Respiratory Syndrome Virus (PRRSV), and Porcine Sapovirus (PSaV).

To achieve the goal, Vero E6 cells were cultured in monolayers in 100π cell culture dishes and then washed with phosphate-buffered saline (PBS). The cells were then inoculated with SARS-CoV-2 KDCD03 strain at a multiplicity of infection (MOI) of 0.1 ffu/cell, as determined by immunofluorescence antibody assay. After infection, the cells were cultured for 4, 8, 12, 18, 24, 36, and 48 hours, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, A549 cells were cultured in monolayers in 100π cell culture dishes and then washed with phosphate-buffered saline (PBS). The cells were then inoculated with influenza A virus PR8 strain at a multiplicity of infection (MOI) of 1 ffu/cell, as determined by immunofluorescence antibody assay. After infection, the cells were cultured for 1, 4, 8, 12, 18, 24, and 36, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, HRT-18G cells were cultured in monolayers in 100π cell culture dishes and then washed with phosphate-buffered saline (PBS). The cells were then inoculated with bovine coronavirus KWD20 strain at a multiplicity of infection (MOI) of 0.1 ffu/cell, as determined by immunofluorescence antibody assay. After infection, the cells were cultured for 18, 36, and 54 hours, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, Vero E6 cells were cultured in monolayers in 100π cell culture dishes and then washed with phosphate-buffered saline (PBS). The cells were then inoculated with porcine epidemic diarrhea coronavirus Q1401 strain at a multiplicity of infection (MOI) of 0.1 ffu/cell, as determined by immunofluorescence antibody assay. After infection, the cells were cultured for 18, 36, and 54 hours, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, MA104 cells were cultured in monolayers in 100π cell culture dishes and then washed with phosphate-buffered saline (PBS). The cells were then inoculated with bovine species A rotavirus NCDV strain at a multiplicity of infection (MOI) of 0.1 ffu/cell, as determined by immunofluorescence antibody assay. After infection, the cells were cultured for 12, 24, and 36 hours, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, MARC-145 cells were cultured in monolayers in 100π cell culture dishes and then washed with phosphate-buffered saline (PBS). The cells were then inoculated with porcine reproductive and respiratory syndrome virus LMY strain at a multiplicity of infection (MOI) of 0.1 ffu/cell, as determined by immunofluorescence antibody assay. After infection, the cells were cultured for 24, 48, and 72 hours, and then detached with trypsin and harvested. The harvested cells were counted using a hemocytometer. An aliquot of 5 × 106 cells was used for each analysis. After centrifugation, the cell pellet was washed with PBS and stored at -20°C.

To achieve the goal, LLC-PK cells were cultured in monolayers in 100π cell culture dishes and then washed with phosphate-buffered saline (PBS). The cells were then inoculated with porcine sapovirus Cowden strain at a multiplicity of infection (MOI) of 0.1 ffu/cell, as determined by immunofluorescence antibody assay. After infection, the cells were cultured for 18, 36, and 54 hours, and then detached with trypsin and harvested. The harvested cells were counted using a hemocytometer. An aliquot of 5 × 106 cells was used for each analysis. After centrifugation, the cell pellet was washed with PBS and stored at -20°C.

For the analysis of intracellular free fatty acids following each viral infection, the Fatty Acid Colorimetric Assay Kit (BioVision) was used as described in the foregoing. According to the instructions, 500 µL of chloroform and 1% Triton-X were added to the cell pellet. The mixture was homogenized by vortexing every 5 minutes, and then the organic phase was collected and dried in air, and the formed pellet was dissolved in 400 µL of Fatty Acid Assay Buffer. After vigorously vortexing for 5 minutes, 50 µL of the extracted supernatant was mixed with 2 µL of ACS Reagent and transferred to corresponding wells of 96-well plates. Additionally, 50 µL of the standard fatty acid solution provided as a positive control and 2 µL of ACS Reagent were also transferred to corresponding wells of 96-well plates. Each well was then supplemented with 50 µL of Enzyme Mix and incubated at 37°C for 30 minutes. Absorbance at 570 nm was read on an ELISA reader. The concentration of fatty acids in each sample was determined using a calibration curve obtained with the standard fatty acid solution, according to the manufacturer's instructions.

For the analysis of intracellular glycerol following each viral infection, the Glycerol Colorimetric Assay Kit (BioVision) was used, as described above. According to the manufacturer's instructions, the cell pellet was mixed with Glycerol Assay Buffer and vortexed vigorously at 5-minute intervals for 20 minutes. After centrifugation, 50 µL of the obtained supernatant from each sample was dispensed into each well of 96-well plates. Also, 50 µL of the standard glycerol solution provided as a positive control was dispensed into each well of 96-well plates. Subsequently, 50 µL of the Reaction Mixture was added to each well containing samples and the positive control and incubated at room temperature for 30 minutes. Absorbance at 570 nm was measured using an ELISA reader. The concentration of glycerol in each sample was determined using a calibration curve obtained with the standard glycerol solution, according to the manufacturer's instructions.

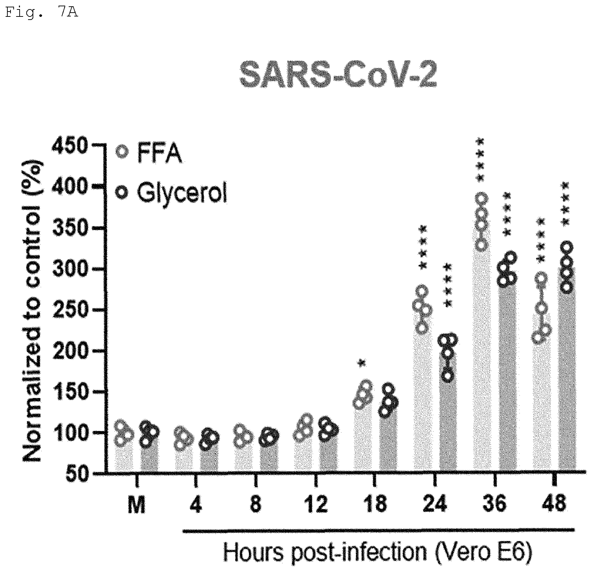

As can be seen in FIG. 7A, the levels of cytoplasmic free fatty acids and glycerol in Vero E6 cells infected with SARS-CoV-2 significantly increased at 24, 36, and 48 hours post-infection.

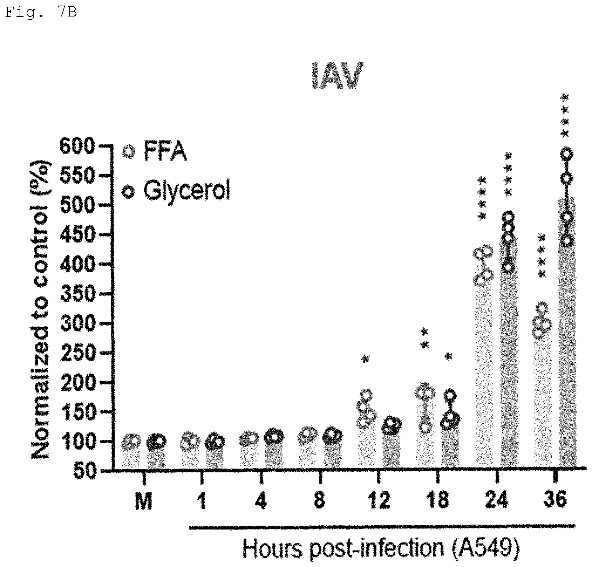

As shown in FIG. 7B, the levels of cytoplasmic free fatty acids and glycerol in A549 cells infected with influenza A virus markedly increased at 24 and 36 hours post-infection.

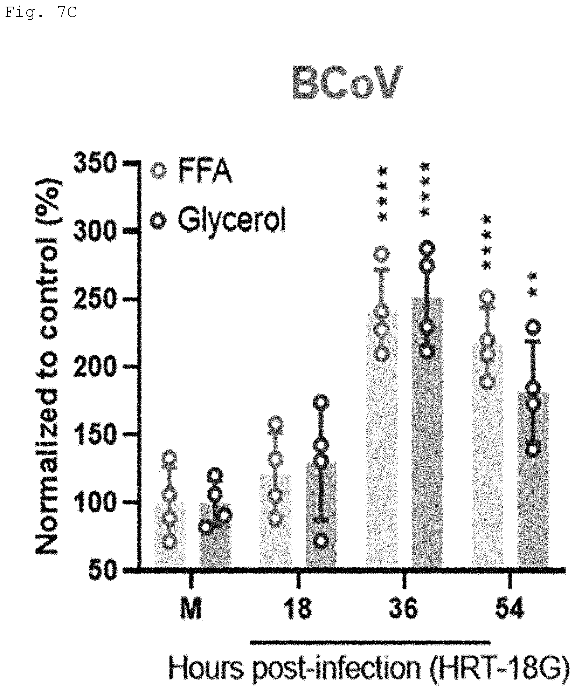

As shown in FIG. 7C, there was a notable increase in the levels of cytoplasmic free fatty acids and glycerol in HRT-18G cells infected with bovine coronavirus at 36 and 54 hours post-infection.

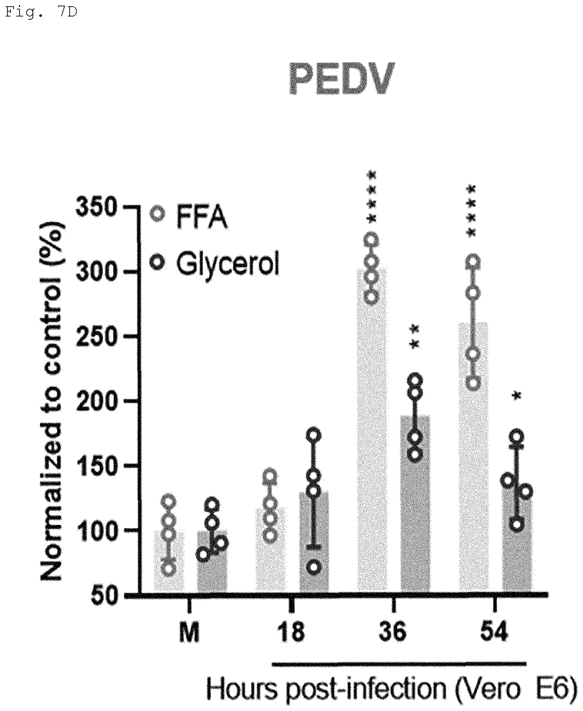

FIG. 7D indicates a significant increase in the levels of cytoplasmic free fatty acids and glycerol in Vero E6 cells infected with porcine epidemic diarrhea coronavirus at 36 and 54 hours post-infection.

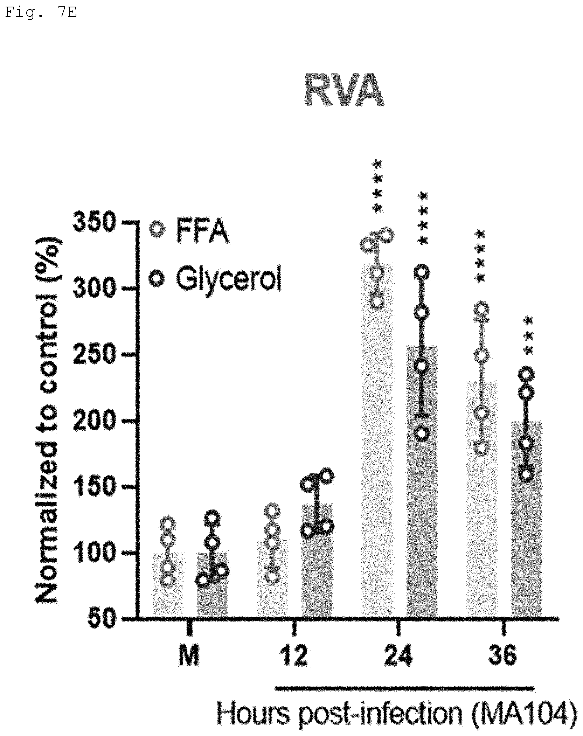

As shown in FIG. 7E, the levels of cytoplasmic free fatty acids and glycerol in MA104 cells infected with bovine species A rotavirus sharply increased at 24 and 36 hours post-infection.

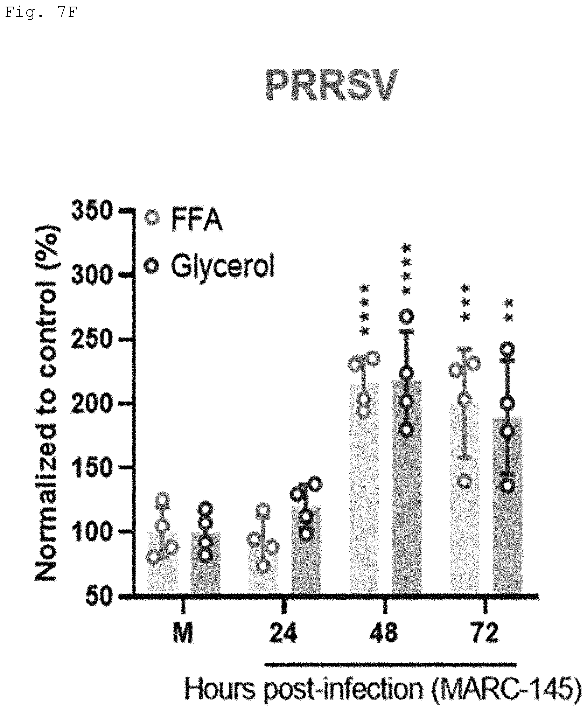

Additionally, FIG. 7F reveals a significant increase in the levels of cytoplasmic free fatty acids and glycerol in MARC-145 cells infected with porcine reproductive and respiratory syndrome virus at 48 and 72 hours post-infection.

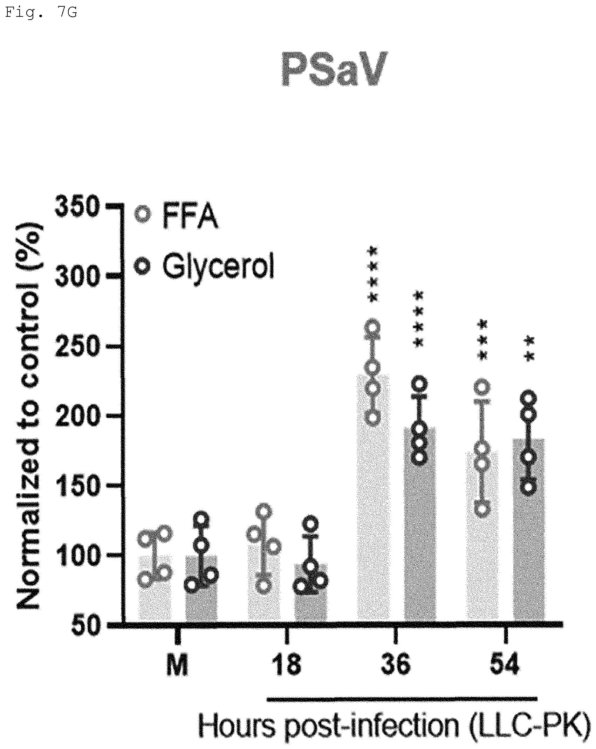

Lastly, as can be seen in FIG. 7G, the levels of cytoplasmic free fatty acids and glycerol in LLC-PK cells infected with porcine sapovirus significantly increased at 36 and 54 hours post-infection.

In conclusion, the observed increase in cytoplasmic free fatty acids and glycerol in the later stages of infection in cells infected with SARS-CoV-2, influenza A virus, bovine coronavirus, porcine epidemic diarrhea coronavirus, bovine species A rotavirus, porcine reproductive and respiratory syndrome virus, and porcine sapovirus was attributed to degradation of the lipid droplets.

### EXPERIMENTAL EXAMPLE 4: Inhibition of Atglistatin and CAY10499 against Proliferation of SARS-CoV-2 and Influenza A Virus and against Palmitoylation of SARS-CoV-2 Spike Protein and Influenza A Virus Hemagglutinin (HA) and M2 Proteins

To verify the antiviral effects due to inhibition against lipases, the adipose triglyceride lipase inhibitor atglistatin (MedChemExpress; CAS Number: 1469924-27-3) and the non-selective lipase inhibitor CAY10499 (Cayman Chemicals; CAS Number: 359714-55-9) were each prepared at a concentration of 20 mM in DMSO.

To achieve the goal, the monolayer of Vero E6 cells in 6-well plates were infected with SARS-CoV-2 strain KCDC03 at a multiplicity of infection (MOI) of 0.1 ffu/cell. The virus was allowed to adsorb to the cells for 1 hour in a 37°C CO2 incubator. Subsequently, the cells were washed with phosphate-buffered saline and cultured for 18 hours in DMEM supplemented with 1 ug/ml TPCK-treated trypsin, 100 u/ml penicillin, and 100 ug/ml streptomycin, in a 37°C CO2 incubator. Then, the wells were washed three times with phosphate-buffered saline, and the medium was replaced with a medium containing a final concentration of 20 µM atglistatin and CAY10499. After an additional 18 hours of incubation, the supernatant was used for determining titers of progeny viruses using cell culture immunofluorescence assay (CCIF assay), and RNA was extracted to measure the number of viral genome copies.

To examine the number of viral genome copies, RNA was extracted from cell pellets with the aid of the RNeasy Mini Kit (Qiagen) according to the manufacturer's instructions. The extracted RNA's concentration and purity were measured, and primary cDNA was synthesized using the TOPscript^{™} cDNA Synthesis kit (Enzynomics) . The synthesized cDNA was used to perform real-time PCR with the TOPreal^{™} qPCR 2X PreMix (Enzynomics) in the presence of primers for the SARS-CoV-2 nucleocapsid gene and an internal control primer for the 60S ribosomal protein L32. The CT values were measured using the LineGene 9600 Plus Real-time PCR detection system (Bioer, Hangzhou, China). The expression level of the nucleocapsid gene relative to the internal control was measured, and the data were presented as mean ± standard error compared to the negative control group.

To perform the Cell Culture Immunofluorescence Antibody Method (CCIF assay), virus supernatant was serially diluted by tenfold in EMEM, leaving only virus-free medium in the last well. The viral dilutions and the negative control were each inoculated in an amount of 100 µL into the monolayer of Vero E6 cells in 96-well plates and incubated for 1 hour at 37°C. Then, 100 µL of EMEM containing 1 µg/mL TPCK trypsin was added to each well and cultured for 18 hours. After removing the culture medium, the cells were fixed for 10 minutes at 20°C with 4% paraformaldehyde. Subsequently, Cell Culture Immunofluorescence Antibody assay was performed using antibodies against the nucleocapsid (N) protein of SARS-CoV-2.

To determine the levels of free fatty acids and glycerol in the cytoplasm, SARS-CoV-2 KCDC03 strain was inoculated at a multiplicity of infection of 0.1 ffu/cell into Vero E6 cells monolayer-cultured in 100π cell culture dishes. After 18 hours, atglistatin and CAY10499 were dropwise added to the medium to achieve a final concentration of 20 µM. After an additional 18 hours of incubation, the cells were harvested using trypsin, and counted using a hemocytometer. An aliquot of 5 × 106 cells was added to each ep-tube. Following centrifugation, the cell pellet was washed with phosphate-buffered saline and used for the analysis of free fatty acids and glycerol content.

To achieve the goal, the monolayer of Vero E6 cells in 8-well chambers was inoculated with SARS-CoV-2 KCDC03 strain at a multiplicity of infection of 0.1 ffu/cell. The cells were then incubated with the virus for 1 hour at 37°C in a CO2 incubator to allow viral adsorption. After washing with phosphate-buffered saline, DMEM supplemented with 1 ug/ml TPCK-treated trypsin, 100 u/ml penicillin, and 100 ug/ml streptomycin was added, followed by incubation for 18 hours at 37°C in a CO2 incubator. The cells were then washed three times with phosphate-buffered saline and the medium was replaced with one containing atglistatin and CAY10499 at a final concentration of 20 µM. After an additional 18 hours of incubation, the cells were fixed for 10 minutes at 20°C with 4% paraformaldehyde. Subsequently, an immunofluorescence antibody assay was performed using antibodies against the SARS-CoV-2 spike protein and lipid droplet marker BODIPY staining.

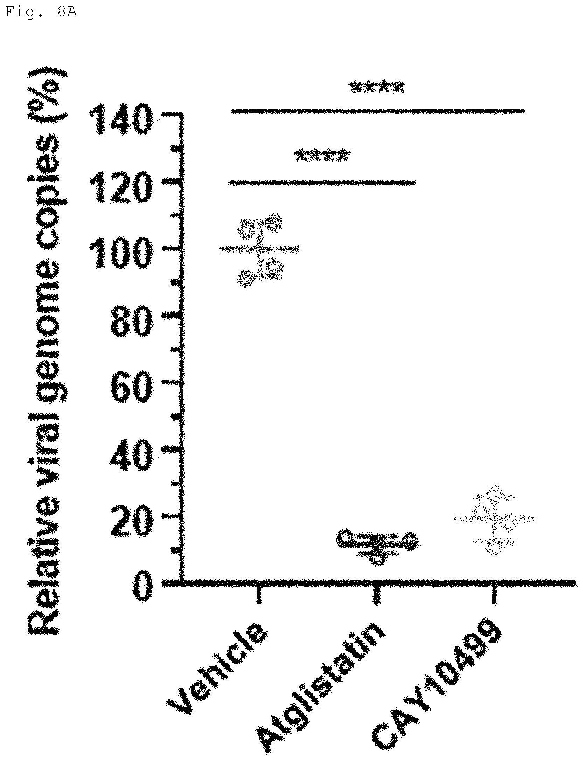

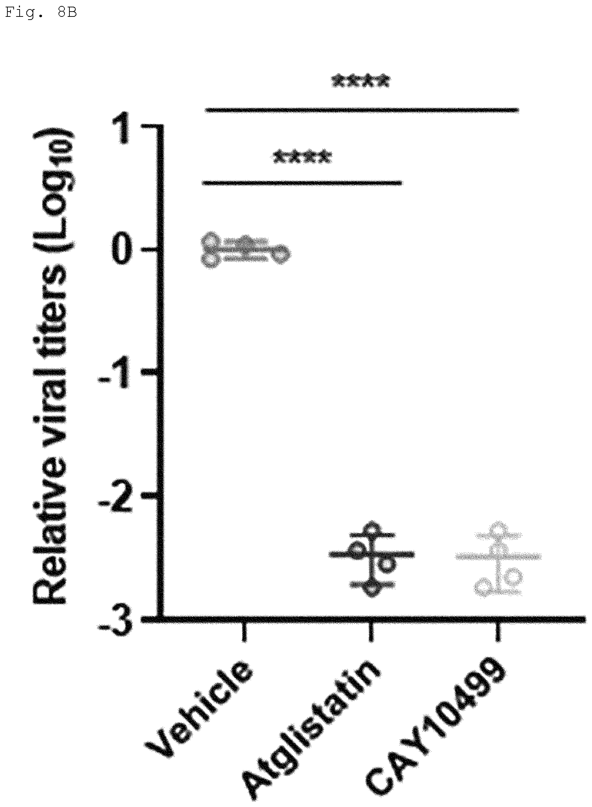

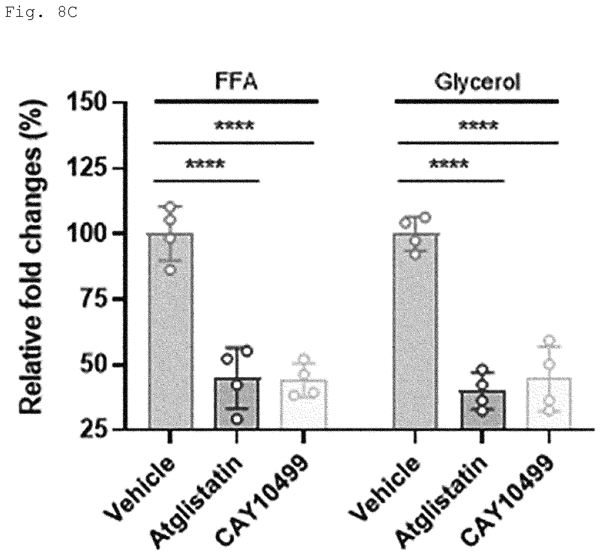

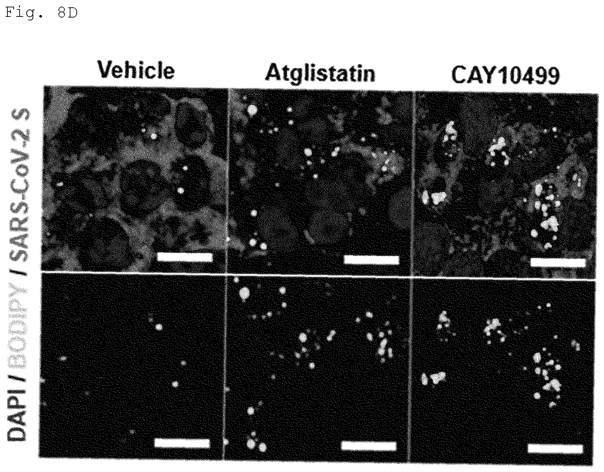

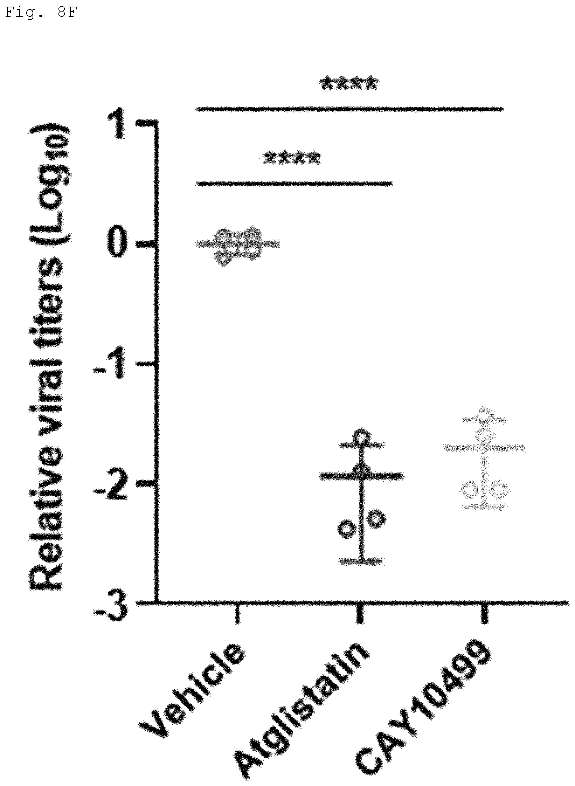

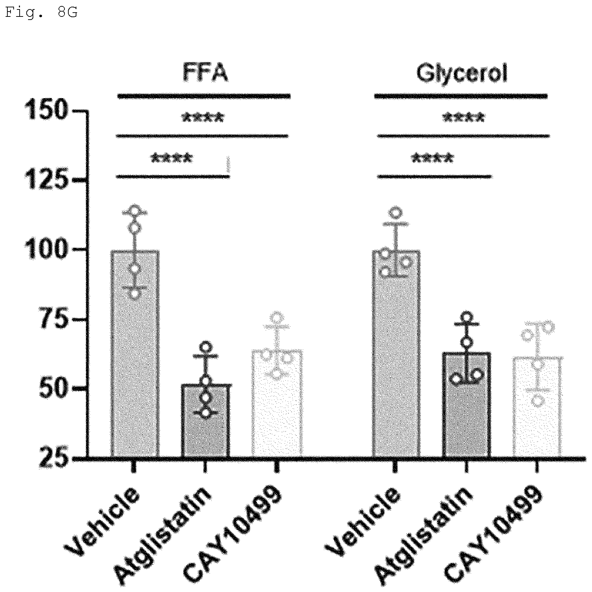

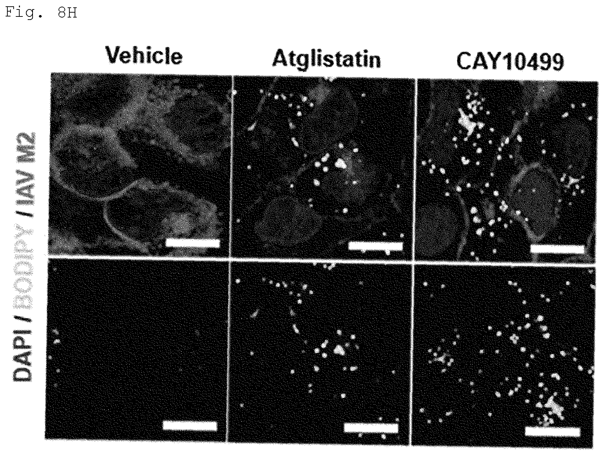

As can be seen in FIGS. 8a to 8d, after inoculation with SARS-CoV-2, the groups treated with atglistatin and CAY10499 showed a significant decrease in viral gene count (a), progeny virus count (b), free fatty acids and glycerol in the cytoplasm (c), and virus spike protein synthesis (d), compared to the vehicle-treated group. However, the number of lipid droplets did not decrease significantly (d).

To achieve the goal, the monolayer of A549 cells in 6-well plates were inoculated with influenza A virus PR8 strain at a multiplicity of infection of 1 ffu/cell. The cells were then incubated with the virus for 1 hour at 37°C in a CO2 incubator to allow viral adsorption. After washing with phosphate-buffered saline, DMEM supplemented with 1 ug/ml TPCK-treated trypsin, 100 u/ml penicillin, and 100 ug/ml streptomycin was added, followed by incubation for 12 hours at 37°C in a CO2 incubator. The cells were then washed three times with phosphate-buffered saline and the medium was replaced with one containing atglistatin and CAY10499 at a final concentration of 20 µM. After an additional 12 hours of incubation, the titer of progeny viruses in the medium supernatant were determined using a cell culture plaque assay and RNA was extracted for counting viral genome copies.

Viral genome copies were determined in the same manner as described above, but real-time PCR was performed using primers specific for the influenza A virus PB1 gene.

For the plaque assay, virus supernatant was serially diluted tenfold using DMEM, with the final dilution being virus-free medium. The viral dilutions and the negative control inoculum were each added in an amount of 200 µL to the monolayer of MDCK cells in a 6-well plate and incubated at 37°C for 1 hour. To each well was added 4 mL of DMEM containing 1 ug/mL TPCK trypsin and 0.5% ultrapure agarose (Thermo Scientific), followed by incubation for 3 days. After removing the culture medium, the cells were fixed for 10 minutes at 20°C with 4% paraformaldehyde. The cells were then washed with phosphate-buffered saline and stained with 0.1 mg/mL crystal violet at 20°C for 30 minutes for plaque analysis.

To determine the levels of free fatty acids and glycerol in the cytoplasm, the monolayer of A549 cells in a 100π cell culture dish were inoculated with influenza A virus strain PR8 at a multiplicity of infection of 1 ffu/cell. After 12 hours, atglistatin and CAY10499 were added to the medium to achieve a final concentration of 20 µM. After a further 12 hours of incubation, the cells were harvested using trypsin and counted using a hemocytometer. An aliquot of 5 × 106 cells was added to each ep-tube. Following centrifugation, the cell pellet was washed with phosphate-buffered saline and used for the analysis of free fatty acids and glycerol content.

For the analysis of viral antigens and lipid droplets, the monolayer of A549 cells in an 8-well chamber were inoculated with influenza A virus PR8 strain at a multiplicity of infection of 1 ffu/cell. The virus was incubated at 37°C for 12 hours, and each well was washed three times with phosphate-buffered saline before replacement with a medium containing 20 µM atglistatin and CAY10499. After an additional 12 hours of incubation, the cells were fixed with 4% paraformaldehyde at 20°C for 10 minutes. Then, immunofluorescence assay was performed using antibodies against influenza A virus M2 protein and BODIPY staining for lipid droplets.

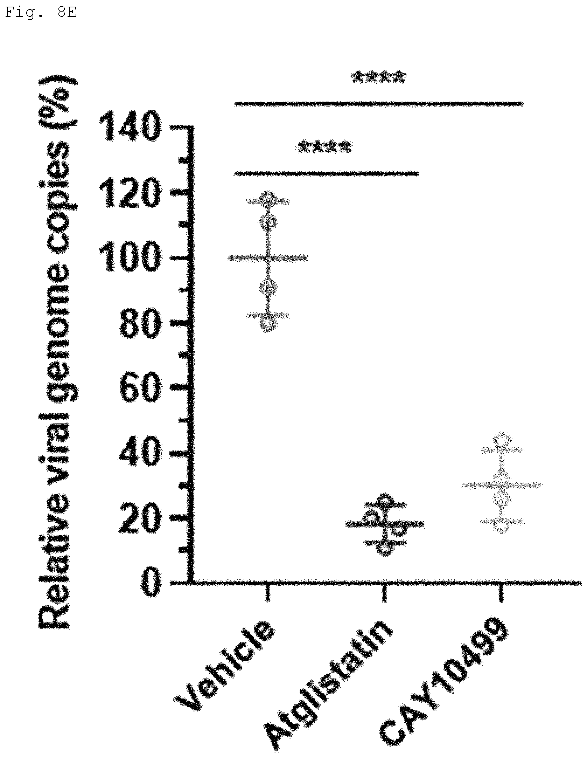

As can be seen in FIGS. 8e to 8h, after inoculation with influenza A virus, the groups treated with atglistatin and CAY10499 showed a significant decrease in viral genome copy (e), progeny virus titer (f), levels of free fatty acids and glycerol in the cytoplasm (g), and production of virus M2 protein (h), but did not significantly decrease in the number of lipid droplets (h), compared to the vehicle-treated group.

The palmitoylation, which refers to the covalent attachment of free fatty acids (FFA) on the spike protein of SARS-CoV-2, the hemagglutinin protein of influenza A virus, and the M2 protein of influenza A virus, is important for the fusion of the virus envelope with host cells, virus assembly and release, and pathogenicity.

For use in verifying that the inhibition of lipase leads to inhibition against the palmitoylation of SARS-CoV-2 spike protein, influenza A virus hemagglutinin protein, and influenza A virus membrane protein, atglistatin and CAY10499 were prepared in DMSO at a concentration of 20 mM.

To achieve the goal, Vero E6 cells were inoculated with SARS-CoV-2 KCDC03 strain at a multiplicity of infection of 0.1 ffu/cell, and the virus was allowed to adsorb onto the cells for 1 hour in a 37°C CO2 incubator. The cells were then washed with phosphate-buffered saline, and DMEM supplemented with 1 ug/ml TPCK-treated trypsin, 100 u/ml penicillin, and 100 ug/ml streptomycin was added. Afterward, the cells were incubated in a 37°C CO2 incubator for 18 hours. Subsequently, each well was washed three times with phosphate-buffered saline, and the medium was replaced with one containing 20 µM atglistatin and CAY10499. Following an additional 18-hour incubation, the cells were washed with phosphate-buffered saline, treated with trypsin, harvested, and centrifuged. The palmitoylation assay (Badrilla, Leeds, UK) was then conducted on these cells according to the manufacturer's instructions.

To achieve the goal, A549 cells were inoculated with influenza A virus PR8 strain at a multiplicity of infection of 1 ffu/cell, and the virus was allowed to adsorb onto the cells for 1 hour in a 37°C CO2 incubator. The cells were then washed with phosphate-buffered saline, and DMEM supplemented with 1 ug/ml TPCK-treated trypsin, 100 u/ml penicillin, and 100 ug/ml streptomycin was added. Afterward, the cells were incubated in a 37°C CO2 incubator for 12 hours. Subsequently, each well was washed three times with phosphate-buffered saline, and the medium was replaced with one containing 20 µM atglistatin and CAY10499. Following an additional 12-hour incubation, the cells were washed with phosphate-buffered saline, treated with trypsin, harvested, and centrifuged. The palmitoylation assay was then conducted on these cells according to the manufacturer's instructions.

The cell pellet was washed with phosphate-buffered saline and then mixed with Buffer A containing Thiol Blocking Reagent. The mixture was incubated at 40°C for 4 hours with continuous stirring. Subsequently, 70% acetone was added to precipitate the proteins, and the supernatant was discarded. The acetone addition process was repeated five times. The pellet was thoroughly washed, followed by elution in a binding buffer at 40°C for 3 hours. A portion of the eluted supernatant was mixed with Laemmli Sample Buffer and used as an input control for total protein, while the remainder underwent a purification process for palmitoylated proteins.

Initially, the total protein supernatant was mixed with CAPTUREome^{™} Capture Resin and Thio Cleavage Reagent, and the mixture was incubated on a rotary mixer at 20°C for 3 hours. After obtaining and washing the resin, the resin-bound palmitoylated proteins were eluted from the resin using Laemmli Sample Buffer. Both the total protein control and the palmitoylated proteins were analyzed by Western blotting using antibodies against SARS-CoV-2 Spike protein, influenza A virus hemagglutinin, and M2 protein.

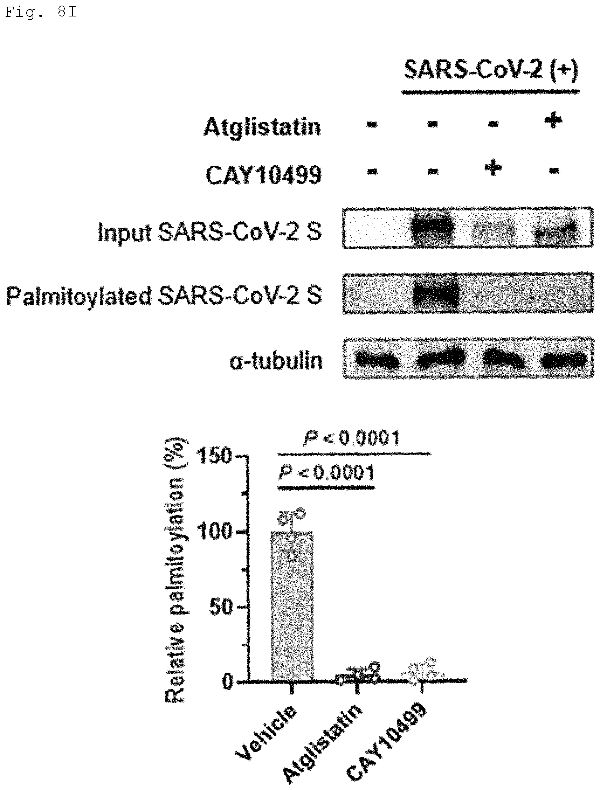

As demonstrated in FIG. 8I, the treatment groups with atglistatin and CAY10499 significantly reduced the palmitoylation of the spike (S) protein of SARS-CoV-2 following infection, compared to the vehicle-treated group.

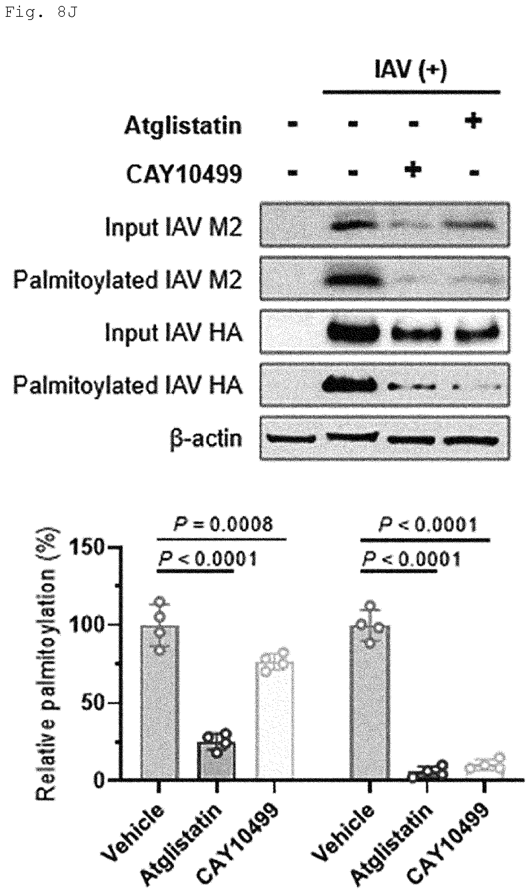

Additionally, as shown in FIG. 8J, post-infection with influenza A virus, the groups treated with atglistatin and CAY10499 significantly decreased the palmitoylation of both hemagglutinin (H) and M2 proteins compared to the vehicle-treated group.

In conclusion, lipid droplets induced by infection with SARS-CoV-2 and influenza A virus are degraded in the later stages of infection, and the free fatty acids released as a result cause palmitoylation of the SARS-CoV-2 spike protein, influenza A virus hemagglutinin protein, and influenza A virus M2 protein. However, atglistatin and CAY10499 inhibit the degradation of lipid droplets to prevent the formation of free fatty acids, thereby suppressing the palmitoylation of viral proteins, which in turn inhibits viral proliferation.

### EXPERIMENTAL EXAMPLE 5: Half-Maximal Cytotoxic Concentration, Half-Maximal Viral Inhibitory Concentration and Selectivity Index of Lipase Inhibitors Atglistatin and CAY10499

To determine the half-maximal cytotoxic concentration (CC50) of the lipase inhibitors atglistatin and CAY10499, Vero E6, A549, HRT-18G, MA104, MARC-145, and LLC-PK cells were prepared in monolayers in 96-well plates. Furthermore, culture media with a concentration gradient of atglistatin serially diluted two-fold starting from 160 µM (160, 80, 40, 20, 10, 5, 2.5, 1.25, 0.625, 0.373, 0.186, 0.093, 0.046 µM), and with a concentration gradient of CAY10499 serially diluted two-fold starting from 80 µM (80, 40, 20, 10, 5, 2.5, 1.25, 0.625, 0.373, 0.186, 0.093, 0.046 µM), were prepared.

The cells were treated with the drugs for 24 hours, and the culture media was removed before addition of 200 µL of 3-(4,5-dimethylthiazol-2-yl)-2,5-diphenyl tetrazolium bromide (MTT) solution to each well. The plates were then incubated for 4 hours in a 5% CO2 incubator at 36°C. Subsequently, 150 µL of DMSO solution was added to each well and incubated at 20°C. The optical density (OD) was measured at 570 nm wavelength using an enzyme-linked immunosorbent assay (ELISA) reader.

To achieve the goal, the monolayer of Vero E6 cells in 96-well plates were infected with SARS-CoV-2 KCDC03 strain at a multiplicity of infection (MOI) of 0.01 ffu/cell, A549 cells with influenza A virus PR8 strain at MOI 0.01 ffu/cell, HRT-18G cells with bovine coronavirus KWD20 strain at MOI 0.01 ffu/cell, Vero E6 cells with porcine epidemic diarrhea coronavirus QIAP1401 strain at MOI 0.01 ffu/cell, MA104 cells with bovine species A rotavirus NCDV strain at MOI 0.01 ffu/cell, MARC-145 cells with porcine reproductive and respiratory syndrome virus LMY strain at MOI 0.01 ffu/cell, and LLC-PK cells with porcine sapovirus Cowden strain at MOI 0.01 ffu/cell. After allowing the virus to adsorb for 1 hour, the wells were washed three times with phosphate-buffered saline and then the media was replaced with the following respective virus culture media.

**TABLE 6**

| Half-maximal cytotoxic concentration of atglistatin and CAY10499 (µM) | | |
|---|---|---|
| **Cell line** | CC₅₀ **(µM)** | |
| | Atglistatin | CAY10499 |
| A549 | 205.9 | 105.7 |
| Vero E6 | 226.4 | 101.8 |
| MDCK | 268.6 | 110.0 |
| HRT18G | 182.5 | 103.5 |
| MA104 | 214.5 | 107.1 |
| MARC | 211.6 | 101.4 |
| LLC-PK | 224.2 | 108.9 |

As seen in Table 6, the half-maximal cytotoxic concentrations (CC50) of atglistatin for A549, Vero E6, MDCK, HRT18G, MA04, MARC, and LLC-PK cells were high, ranging between 182.5 to 268.6, while the CC50 of CAY10499 was also high, ranging between 101.4 to 110.0, though slightly lower than that of atglistatin.

To test the half-maximal inhibitory concentration (IC50) of Atglistatin and CAY10499 against various viruses, SARS-CoV-2 was cultured for 18 hours, influenza A virus for 12 hours, bovine coronavirus for 18 hours, porcine epidemic diarrhea coronavirus for 18 hours, bovine species A rotavirus for 12 hours, porcine reproductive and respiratory syndrome virus for 18 hours, and porcine sapovirus for 18 hours. Subsequently, the culture media were replaced with media containing 160, 80, 40, 20, 10, 5, 2.5, 1.25, 0.625, 0.373, 0.186, 0.093, and 0.046 µM atglistatin, and 80, 40, 20, 10, 5, 2.5, 1.25, 0.625, 0.373, 0.186, 0.093, and 0.046 µM CAY10499. The cultures were then incubated for an additional 18 hours for SARS-CoV-2, 12 hours for influenza A virus, 18 hours for bovine coronavirus, 18 hours for porcine epidemic diarrhea coronavirus, 12 hours for bovine species A rotavirus, 18 hours for porcine reproductive and respiratory syndrome virus, and 18 hours for porcine sapovirus. After this incubation, the supernatants were removed, and the cells were fixed for 15 minutes in a 4% paraformaldehyde solution.

Once fixed, the cells were washed three times in each well with phosphate-buffered saline and then treated with 0.2% Triton X-100 (Sigma Aldrich) for 10 minutes at 20°C. After three washes with phosphate-buffered saline, 100- to 200-fold diluted primary antibodies against SARS-CoV-2 spike protein, influenza A virus nucleoprotein, bovine coronavirus spike protein, porcine epidemic diarrhea coronavirus nucleocapsid protein, bovine species A rotavirus VP6 protein, porcine reproductive and respiratory syndrome virus matrix protein, and porcine sapovirus VPg protein were added to each well and incubated for 12 hours in a refrigerator at 4°C.

After removing the supernatant, the cells in each well were washed three times with phosphate-buffered saline (PBS) and then treated with a 100- to 200-fold diluted secondary antibody conjugated with Alexa Fluor 488 (AF488) or fluorescein isothiocyanate (FITC). The cells were incubated at room temperature for 2 hours. Then, the supernatant was removed again, and the nuclei were stained with a propidium iodide solution. Subsequently, a 60% glycerol solution was added to each well, and the virus-positive cells were examined under a fluorescence microscope. Based on these experiments, the CC50 and IC50 values were calculated, and the selective index (SI = CC50/IC50) was determined by dividing the CC50 value by the IC50 value.

**TABLE 7**

| Half-maximal inhibitory concentration and selective index of atglistatin and CAY10499 | | | | | |
|---|---|---|---|---|---|
| Virus family | Virus (strain) | IC₅₀ (µM) | | SI (CC₅₀/IC₅₀)^{a} | |
| | | Atglist atin | CAY10499 | Atglist atin | CAY10499 |
| *Orthomyxov iridae* | Influenza A virus (H1N1) (PR8) | 1.07 | 4.13 | 251 | 27 |
| *Coronaviri dae* | SARS-CoV-2 (KCDC03) | 1.47 | 1.54 | 154 | 62 |
| *Coronaviri dae* | Bovine coronavirus (KWD20) | 1.20 | 7.30 | 152 | 11 |
| *Coronaviri dae* | Porcine epidemic diarrhea coronavirus (QIAP1401) | 1.96 | 3.19 | 116 | 30 |
| *Reoviridae* | Species A Rotavirus (NCDV) | 6.70 | 20.85 | 32 | 4 |
| *Arteriviri dae* | Porcine respiratory reproductive syndrome virus (LMY) | 2.53 | 4.21 | 84 | 21 |
| *Caliciviri dae* | Porcine sapovirus (Cowden) | 3.11 | 11.76 | 72 | 8 |

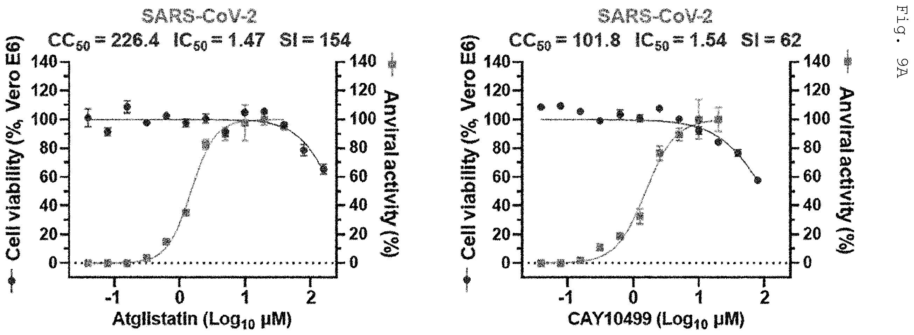

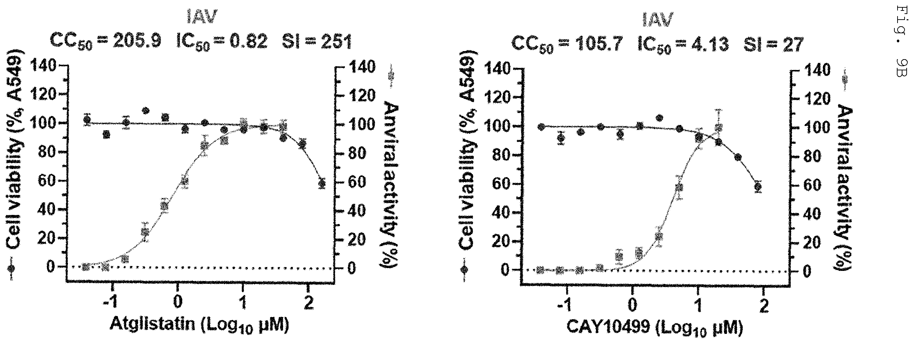

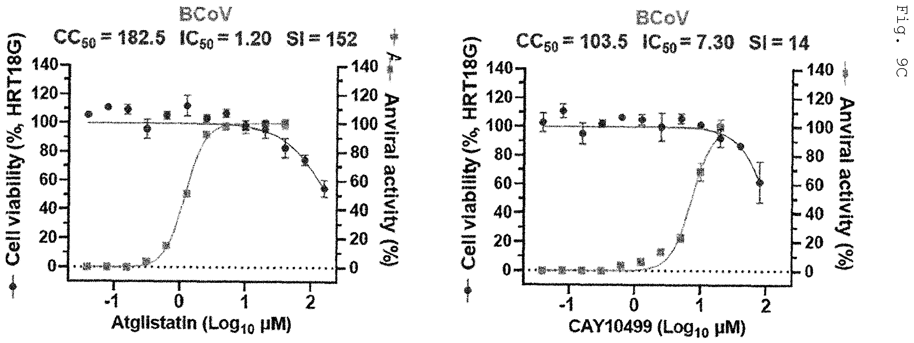

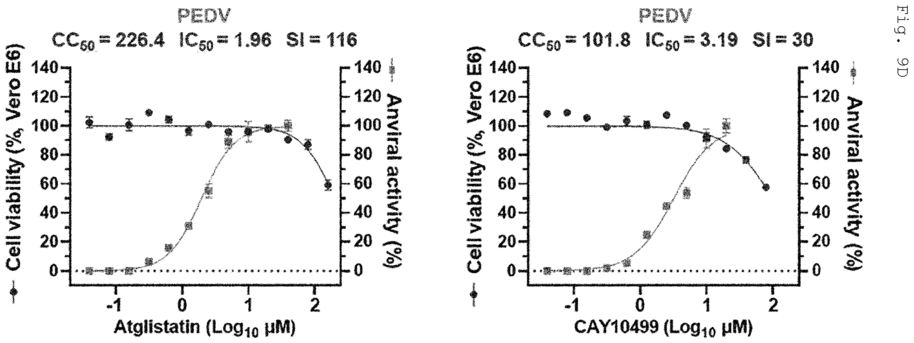

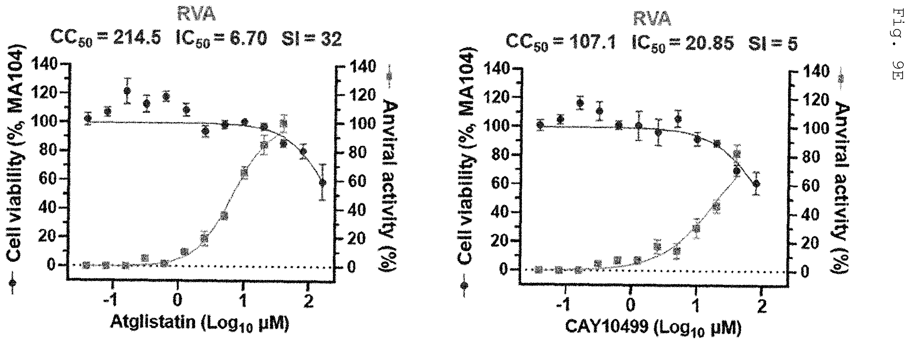

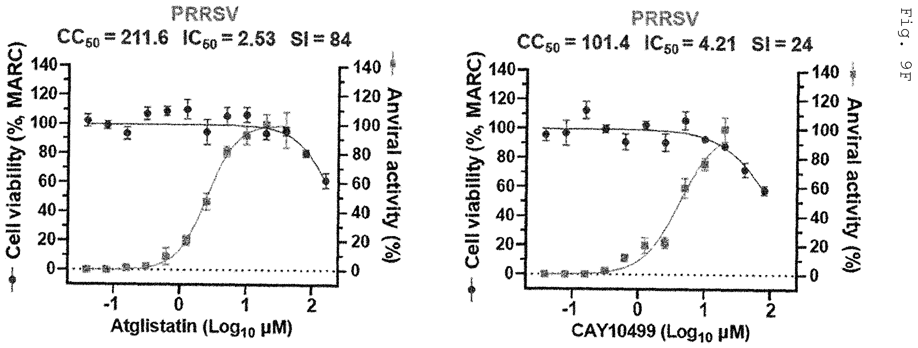

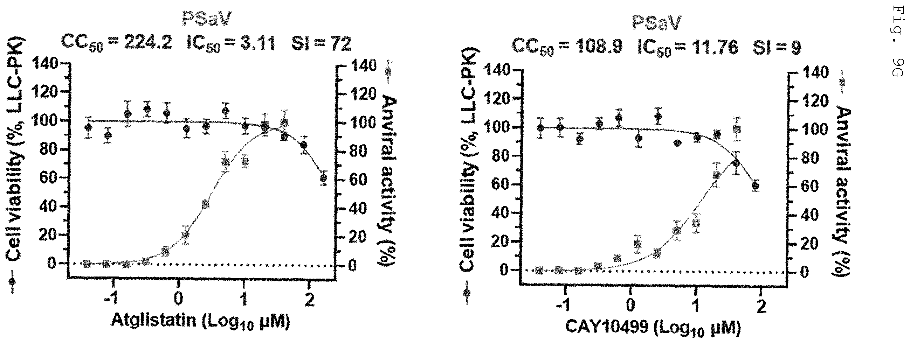

As shown in Table 7 and FIGS. 9a to 9g, the half-maximal inhibitory concentration (IC50) of atglistatin ranged from 1.07 to 6.70, while for CAY10499, it ranged from 1.54 to 20.85. Notably, against the globally impactful influenza A virus and SARS-CoV-2, the IC50 of atglistatin was 0.82 and 1.47, respectively, while for CAY10499, it was 4.13 and 1.54. This indicates that atglistatin and CAY10499 have high inhibitory capabilities at low concentrations against influenza A virus and SARS-CoV-2.

Furthermore, in terms of the selective index (SI), which shows the balance between cell toxicity and infection inhibition, the SI for SARS-CoV-2 KCDC03 strain, influenza A virus PR8 strain, bovine coronavirus KWD20 strain, porcine epidemic diarrhea coronavirus QIAP1401 strain, bovine species A rotavirus NCDV strain, porcine reproductive and respiratory syndrome virus LMY strain, and porcine sapovirus Cowden strain were 154, 251, 152, 116, 32, 84, and 72 for atglistatin, and 62, 27, 14, 30, 5, 24, and 9 for CAY10499, respectively. This demonstrates that both atglistatin and CAY10499 exhibit low cytotoxicity and high infection inhibition capability.

### EXPERIMENTAL EXAMPLE 6: Inhibitory Effects of Lipase Inhibitors Atglistatin and CAY10499 on Viral Genome Copy and Progeny Virus Production

To detect the inhibitory effects of atglistatin and CAY10499 on the copies of various viral genomes and the production of progeny viruses, the monolayer of Vero E6 cells in 12-well plates were infected with SARS-CoV-2 KCDC03 strain at an infectious multiplicity of 0.01 ffu/cell, A549 cells with influenza A virus (IAV) PR8 strain at 0.01 ffu/cell, HRT-18G cells with bovine coronavirus (BCoV) KWD20 strain at 0.01 ffu/cell, Vero E6 cells with porcine epidemic diarrhea coronavirus (PEDV) QIAP1401 strain at 0.01 ffu/cell, MA104 cells with bovine species A rotavirus (RVA) NCDV strain at 0.01 ffu/cell, MARC0145 cells with porcine reproductive and respiratory syndrome virus (PRRSV) LMY strain at 0.01 ffu/cell, and LLC-PK cells with porcine sapovirus Cowden strain at 0.01 ffu/cell, followed by adsorbing the viruses for one hour.

Subsequently, each well was washed three times with phosphate-buffered saline, followed by the addition of the respective viral culture media. Incubation was made for 18 hours for SARS-CoV-2, 12 hours for influenza A virus, 18 hours for bovine coronavirus, 18 hours for porcine epidemic diarrhea coronavirus, 12 hours for bovine species A rotavirus, 18 hours for porcine reproductive and respiratory syndrome virus, and 18 hours for porcine sapovirus.

Thereafter, the culture media were replaced with media containing 1, 10, and 20 µM of atglistatin and CAY10499. Additional incubation was made of the plates infected with SARS-CoV-2 for 18 hours, influenza A virus for 12 hours, bovine coronavirus for 18 hours, porcine epidemic diarrhea coronavirus for 18 hours, bovine species A rotavirus for 12 hours, porcine reproductive and respiratory syndrome virus for 18 hours, and porcine sapovirus for 18 hours. Subsequently, each plate underwent three cycles of freezing and thawing to detach the cells from each well for subsequent experiments.

To analyze the inhibitory effects of atglistatin and CAY10499 on the genome copies of each virus, RNA was extracted using the RNeasy mini kit (Qiagen). The extracted RNA was then used to synthesize cDNA using the TOPscript^{™} cDNA Synthesis kit (Enzynomics), which includes random hexamer and poly(rA)-oligo(dT). Afterward, real-time PCR was performed on the synthesized cDNA in the TOPreal^{™} qPCR 2X PreMix (Enzynomics) solution in the presence of specific primers for SARS-CoV-2 nucleocapsid (N), influenza A virus PB1, bovine coronavirus spike (S), porcine epidemic diarrhea coronavirus nucleocapsid (N), bovine species A rotavirus VP6, porcine reproductive and respiratory syndrome virus matrix (M), and porcine sapovirus VPg, using the LineGene 9600 Plus Real-time PCR detection system (Bioer, Hangzhou, China).

To analyze the inhibitory effects of atglistatin and CAY10499 on the production of progenies of the viruses, the supernatant obtained after three cycles of freezing and thawing was serially ten-fold diluted. Subsequently, the supernatant of SARS-CoV-2 KCDC03 strain was adsorbed onto the monolayer of Vero E6 cells in a 96-well plate, the supernatant of influenza A virus PR8 strain onto A549 cells, the supernatant of bovine coronavirus KWD20 strain onto HRT-18G cells, the supernatant of porcine epidemic diarrhea coronavirus QIAP1401 strain onto Vero E6 cells, the supernatant of bovine species A rotavirus NCDV strain onto MA104 cells, the supernatant of porcine reproductive and respiratory syndrome virus LMY strain onto MARC-145 cells, and the supernatant of porcine sapovirus strain Cowden onto LLC-PK cells. After adsorption, each well was washed twice with phosphate-buffered saline (pH 7.4), followed by the addition of the respective culture media.

Additional incubation was made of SARS-CoV-2 for 12 hours, influenza A virus for 8 hours, bovine coronavirus for 12 hours, porcine epidemic diarrhea coronavirus for 12 hours, bovine species A rotavirus for 8 hours, porcine reproductive and respiratory syndrome virus for 18 hours, and porcine sapovirus for 12 hours. Then, after removal of the supernatants, the cells were fixed with 4% paraformaldehyde for 15 minutes and each well was washed thrice with phosphate-buffered saline and treated with 0.2% Triton X-100 at 20°C for 10 minutes.

After three washes with phosphate-buffered saline, the wells were treated with 100- to 200-fold diluted primary antibodies to the spike protein of SARS-CoV-2, the nucleoprotein protein of influenza A virus, the spike protein of bovine coronavirus, the nucleocapsid protein of porcine epidemic diarrhea coronavirus, the VP6 protein of bovine species A rotavirus, the matrix protein of porcine reproductive and respiratory syndrome virus, and the VPg protein of porcine sapovirus, respectively, and applied to a refrigerator at 4°C for 12 hours.

Subsequently, the supernatant was removed, and each well was washed thrice with phosphate-buffered saline, followed by incubation with a 100- to 200-fold dilution of a secondary antibody conjugated with Alexa Fluor 488 or fluorescein isothiocyanate at room temperature for 2 hours. The supernatant was removed, and the nuclei were stained with a propidium iodide solution. A 60% glycerol solution was added to each well that was then examined for virus-positive cells under a fluorescence microscope.

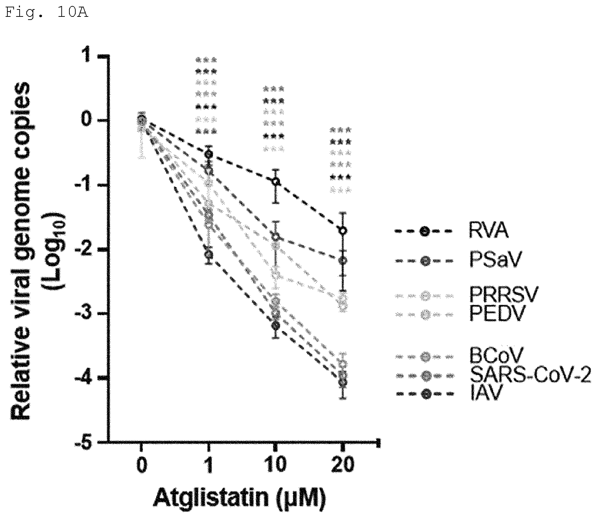

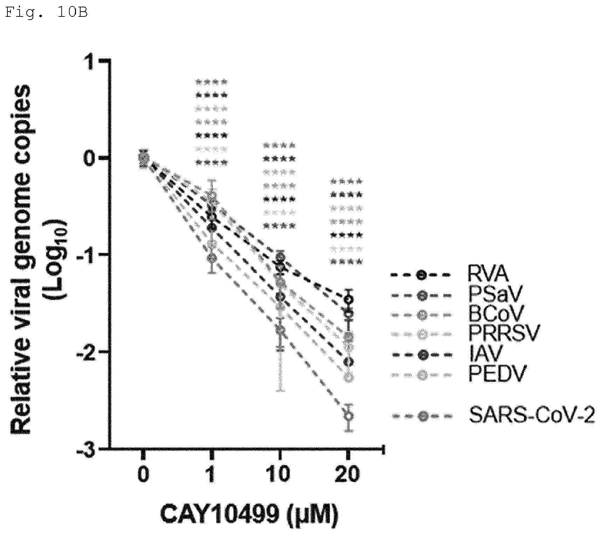

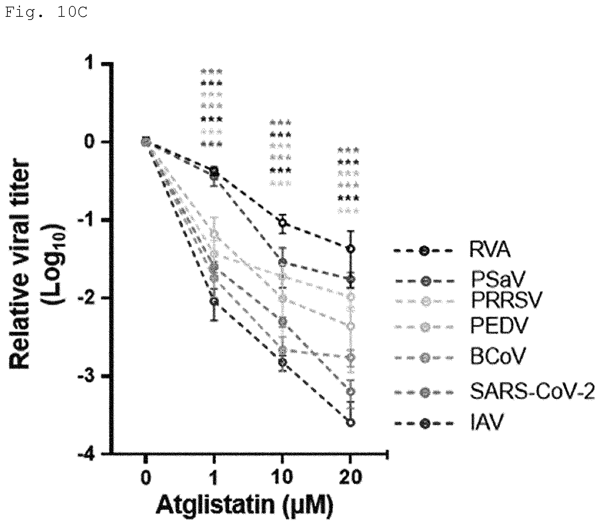

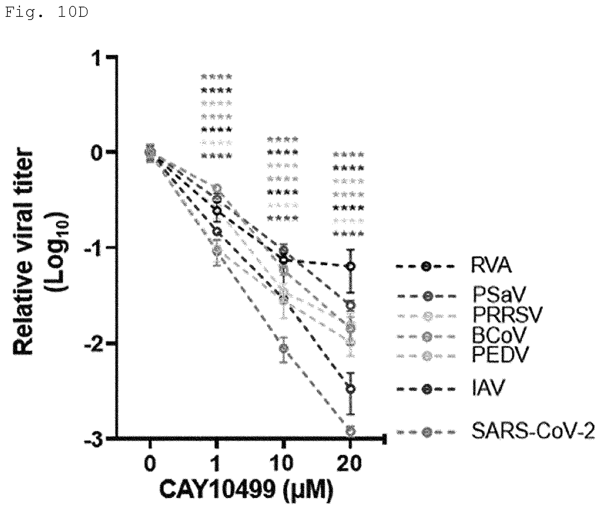

As can be seen in FIGS. 10a to 10d, atglistatin and CAY10499 reduced the copies of genomes and the production of progenies of each virus at all concentrations of 1, 10, and 20 µM in a dose-dependent manner with significance. Therefore, it was demonstrated that the use of lipase inhibitors exhibited an antiviral effect against a broad spectrum of RNA viruses.

### EXPERIMENTAL EXAMPLE 7: Effects of Lipase Inhibitors Atglistatin and CAY10499 on Survival Rate, Weight Loss, Clinical Symptoms, Virus Proliferation, and Lesion in Mice Infected with Influenza A Virus (IAV)

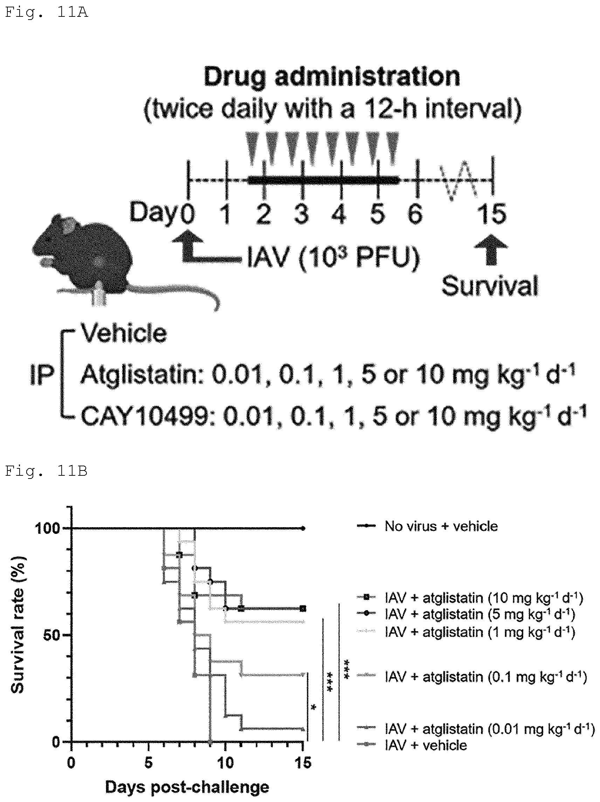

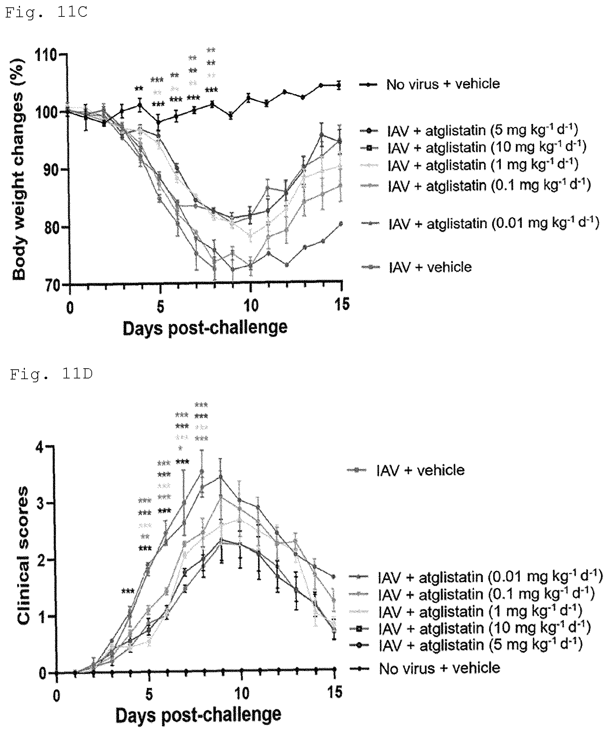

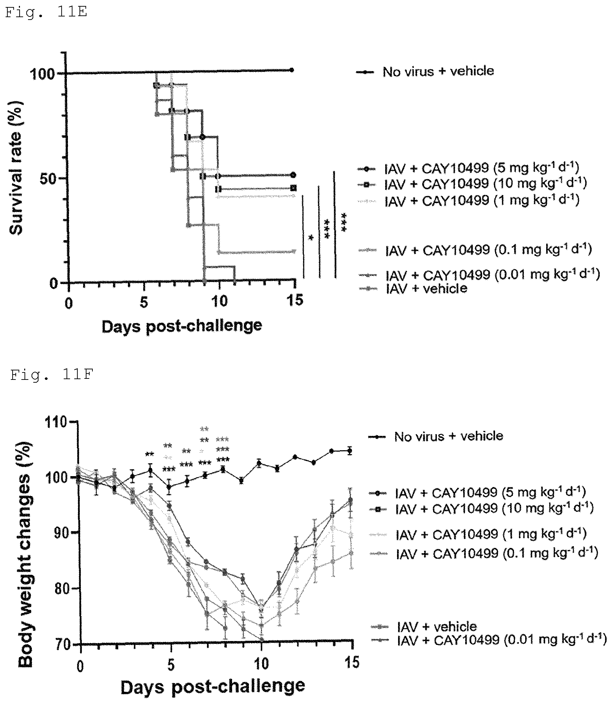

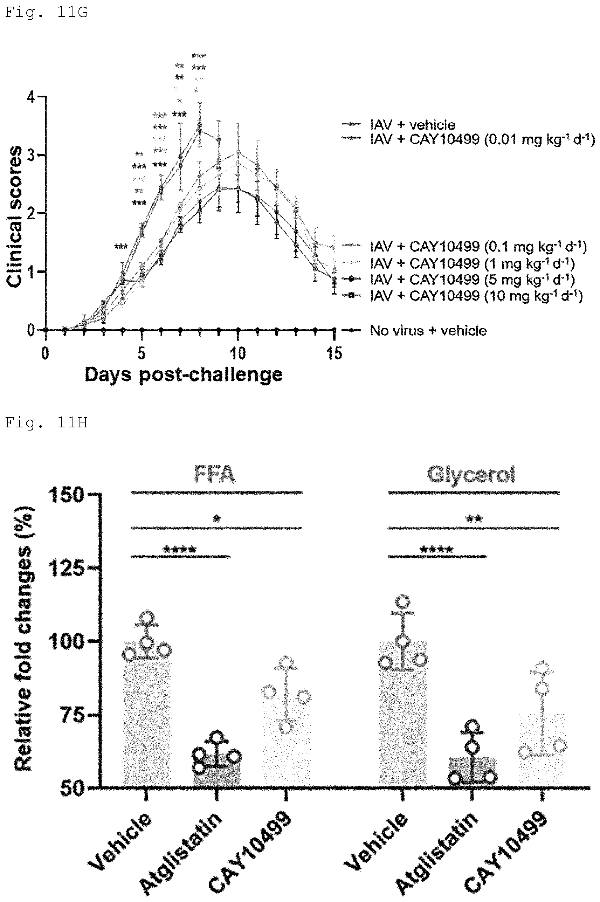

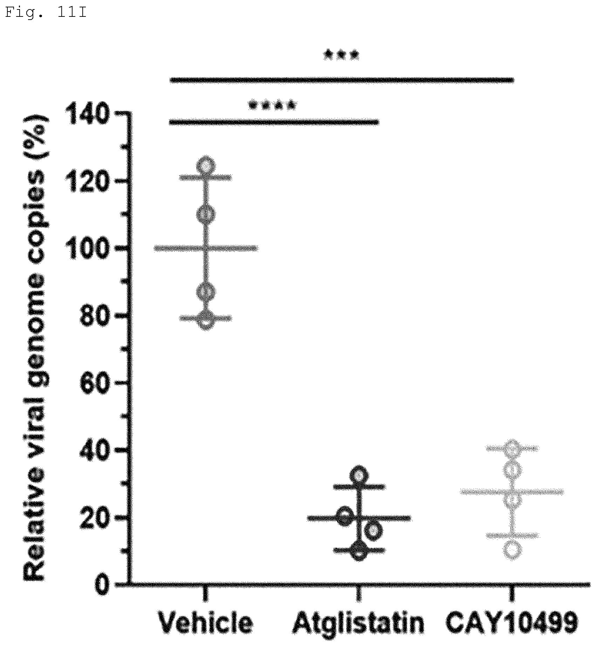

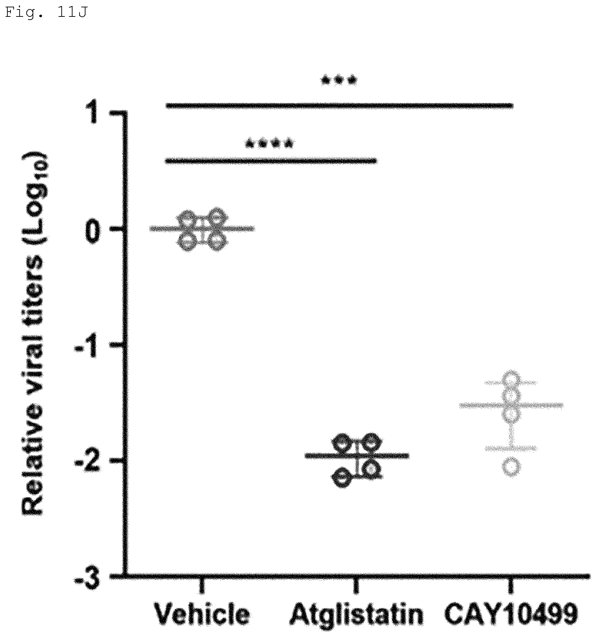

For use in verifying the antiviral effect of lipase inhibition in animals infected with influenza A virus, 150 mg/ml of atglistatin and 150 mg/ml of CAY10499 were dissolved in DMSO. To evaluate the antiviral effect depending on the concentration, the drugs were prepared at concentrations of 1.000, 0.500, 0.100, 0.010, and 0.001 mg/ml. In this regard, a mixture of DMSO:40% PEG:H₂O at a ratio of 1:4:5 was used as a solvent. The drugs were completely dissolved by vortexing. When administered to mice, 200 µL of the solutions accounted for doses of 10.00, 5.00, 1.00, 0.10, and 0.01 mg/kg/day, respectively (see FIG. 11A).

For an assay for the antiviral efficacy of atglistatin, a total of seven experimental groups were established as follows: a non-injected and untreated group (No virus + vehicle), a group injected with influenza A virus PR8 strain and untreated (IAV + vehicle), a group injected with influenza A virus PR8 strain and treated with 0.01 mg/kg/day atglistatin (IAV + atglistatin (0.01 mg kg-¹ day⁻¹)), a group injected with the virus and treated with 0.1 mg/kg/day atglistatin (IAV + atglistatin (0.1 mg kg-¹ day⁻¹)), a group injected with the virus and treated with 1 mg/kg/day atglistatin (IAV + atglistatin (1 mg kg⁻¹ day⁻¹)), a group injected with the virus and treated with 5 mg/kg/day atglistatin (IAV + atglistatin (5 mg kg⁻¹ day⁻¹)), and a group injected with the virus and treated with 10 mg/kg/day atglistatin (IAV + atglistatin (10 mg kg-¹ day⁻¹)). Each group of 16 female wild-type C57BL/6J mice, each seven weeks old, was acclimatized for one week under the described conditions and used in the experiment at eight weeks of age. For evaluating the antiviral efficacy of CAY10499, mouse groups were prepared in the same manner with the exception of using CAY10499 instead of atglistatin.

Influenza A virus inoculation was carried out on anesthetized mice via an intranasal route at 1,000 PFU per mouse, following the same procedure as above. In cases where the virus was not injected, only phosphate-buffered saline was administered. Observations were made for 15 days, during which survival rates, body weight, and clinical symptoms were monitored and recorded daily. Mice that showed severe weight loss (more than 30%) were euthanized and recorded as deaths due to viral infection.

Drug treatment began two days after the inoculation of influenza A virus. The drugs were administered intraperitoneally using a syringe, twice daily at 12-hour intervals in the morning and afternoon, at the consistent certain time each day, for a total of four days, with each dose being 100 µL. For the untreated group, a mixture of the drug diluent DMSO/40% PEG/H₂O was administered intraperitoneally (see FIG. 11A).

The survival rate, which was 100% with a 0% mortality rate on day 0 of the infection, was measured daily over 15 days, and compared with the survival rate of the virus-injected and untreated group. Statistical significance was analyzed using the Log Rank Test, with a p value of 0.05 or higher considered significant.

As seen in FIGS. 11b to 11g, administration of the lipase inhibitors atglistatin and CAY10499 to influenza A virus-infected mice resulted in an improvement in survival rate, weight loss, and clinical symptoms in a dose-dependent manner. Notably, a survival rate of 60% was achieved in the experimental group treated with atglistatin at 10 mg kg⁻¹ day⁻¹ for four days.

Next, examination was made of the effects of atglistatin or CAY10499 treatment on free fatty acids and glycerol in the lung tissues, viral genome copies, and progeny virus numbers. Additionally, to assess how much influenza A virus-induced pneumonia could be alleviated, immunofluorescence staining for virus antigen and lipid droplets in mouse lung tissues, histopathological analysis, and immunohistochemical staining for virus antigens were performed as follows.

Experimental groups were established: a non-injected and untreated group (No virus + vehicle), a group injected with influenza A virus PR8 strain and untreated (IAV + vehicle), a group injected with influenza A virus PR8 strain and treated with 5 mg/kg/day atglistatin (IAV + atglistatin (5 mg kg⁻¹ day⁻¹)), and a group injected with influenza A virus PR8 strain and treated with 5 mg/kg/day CAY10499 (IAV + CAY10499 (5 mg kg⁻¹ day⁻¹)). Each group consisted of four seven-week-old female wild-type C57BL/6J mice, acclimatized for one week, and used in the experiment at eight weeks of age.

Mice were inoculated with influenza A virus PR8 strain in the same manner as described above, and drug treatment was conducted for 4 days, starting from the second day post-infection. On the sixth day post-infection, the mice were euthanized for autopsy. The left lobe of the excised lung was fixed at 4°C for one week in 4% paraformaldehyde for histopathological analysis and preparation of frozen sections. The right cranial, middle, caudal, and accessory lobes were combined and finely chopped together for analysis of free fatty acids and glycerol in the cytosol, as well as for assessing viral genome copies and progeny virus titers.

These tissues were assayed for free fatty acid levels. In this regard, the sample was mixed with chloroform containing 1% Triton-X 100 and assayed in a similar manner to that for cellular free fatty acid analysis. The absorbance was read at 570 nm on an ELISA reader.

For a glycerol assay, the samples were mixed with Glycerol Assay Buffer and analyzed in the same manner as for the cellular glycerol assay, with the absorbance read at 570 nm on an ELISA reader.

The inhibitory effect of the drugs on the virus genome was evaluated by performing real-time PCR for the influenza A virus PB1 gene and the host β-actin gene, to quantify the viral gene numbers.

For the plaque assay, 100 mg of mouse lung tissue was homogenized using a Precellys 23 instrument homogenizer from Bertin Technologies (Montigny-le-Bretonneux, France) with beads. After centrifugation of the homogenate, the supernatant was collected and serially diluted, and a plaque assay was performed on monolayer-cultured MDCK cells in 6-well plates, as previously described, to evaluate the inhibitory effect of each drug against the proliferation of progeny viruses.

As shown in FIGS. 11h to 11j, administration of the lipase inhibitors Atglistatin and CAY10499 to influenza A virus-infected mice resulted in a significant decrease in free fatty acids and glycerol concentrations (h), virus gene numbers (i), and progeny virus numbers (j) in lung tissues, with higher effectiveness with atglistatin than CAY10499.

To evaluate lipid droplets and influenza A virus antigen in the lung tissues, the fixed tissues were prepared into 3-um-thick frozen sections. These sections were blocked with 5% BSA and incubated with a primary antibody against influenza A virus M2 antigen at 4°C for 16 hours and then with AF594-conjugated mouse IgG(H+L) secondary antibody at 20°C for one hour. The tissue sections were washed with phosphate-buffered saline and reacted with 10 µM BODIPY for 15 minutes at 20°C for lipid droplet staining. After washing, the sections were mounted with Slow-Fade Gold antifade reagent for nucleus staining.

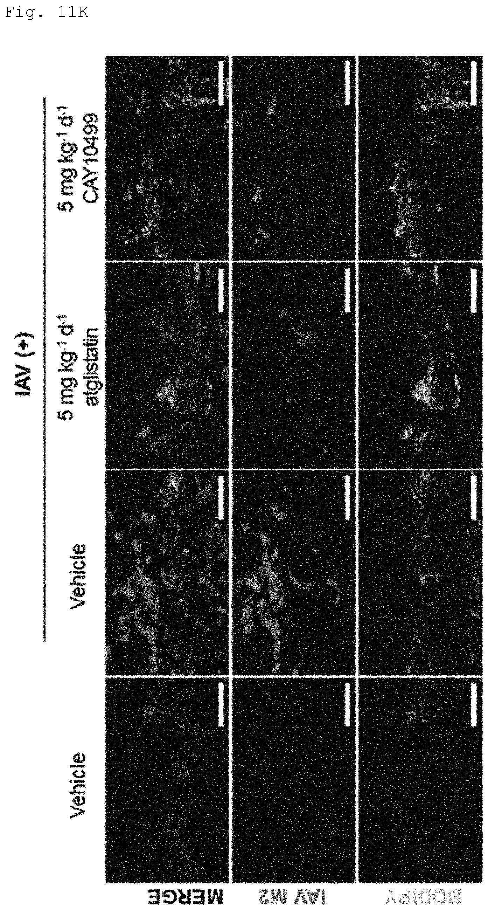

As shown in FIGS. 11k, administration of the lipase inhibitors Atglistatin and CAY10499 to influenza A virus-infected mice resulted in a significant reduction in viral antigen in the bronchiolar epithelial cells of mice treated with the drugs post virus inoculation compared to the untreated virus-inoculated group. However, the number of lipid droplets did not decrease significantly.

For histopathological analysis of the lung tissues, the fixed tissues were dehydrated through graded alcohols, cleared in xylene, and embedded in paraffin. The sections were then finely cut at 3 um thickness, deparaffinized in xylene, and rehydrated through graded alcohols and water. The rehydrated sections were stained with Hematoxylin & Eosin, dehydrated through graded alcohols, cleared in xylene, and mounted. Histological examination was then performed using an optical microscope.

Immunohistochemistry was performed for influenza A virus antigens in the lung tissue. Paraffin-embedded tissue sections, each 3 um thick, were deparaffinized, washed, rehydrated, and autoclaved in 1 mM citrate solution, pH 6.0 for antigen retrieval. Afterwards, the sections were treated with 3% H₂O₂ to quench endogenous peroxidase activity, blocked with 5% BSA, and then incubated for 16 hours at 4°C with a primary antibody against IAV virion, purchased from Virostat (Portland, ME, USA). The next day, after washing with phosphate-buffered saline, the sections were incubated for one hour with the secondary antibody HRP-conjugated goat IgG (H+L) and subjected to chromogenesis with DAB.

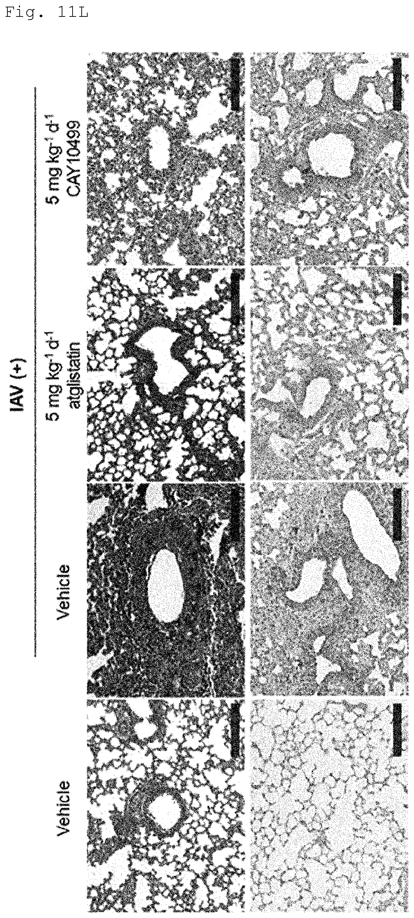

As shown in FIG. 11L, administration of the lipase inhibitors atglistatin and CAY10499 to influenza A virus-infected mice resulted in the alleviation and reduction of lung lesions, including interstitial pneumonia, and the number of virus-positive cells, compared to the mice in the untreated virus-inoculated group. Notably, atglistatin was more effective than CAY10499.

Consequently, the lipase inhibitors atglistatin and CAY10499 were effective in protecting mice infected with influenza A virus. When administered at a dose of 10 mg kg-¹ day⁻¹, atglistatin and CAY10499 increased the survival rate to 62.5% and 50%, respectively, compared to a 100% mortality rate in the control group. Notably, atglistatin showed a higher viral inhibition capability than CAY10499.

### EXPERIMENT EXAMPLE 8: Effects of Atglistatin Treatment on Severity of Pneumonia, Virus Proliferation, Weight Loss, and Lesion in SARS-CoV-2-Infected Hamsters

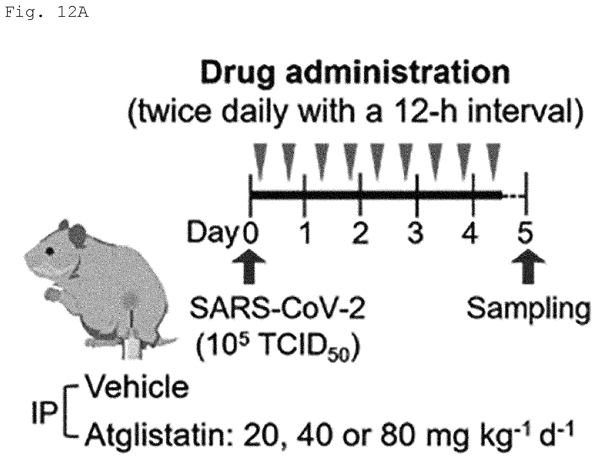

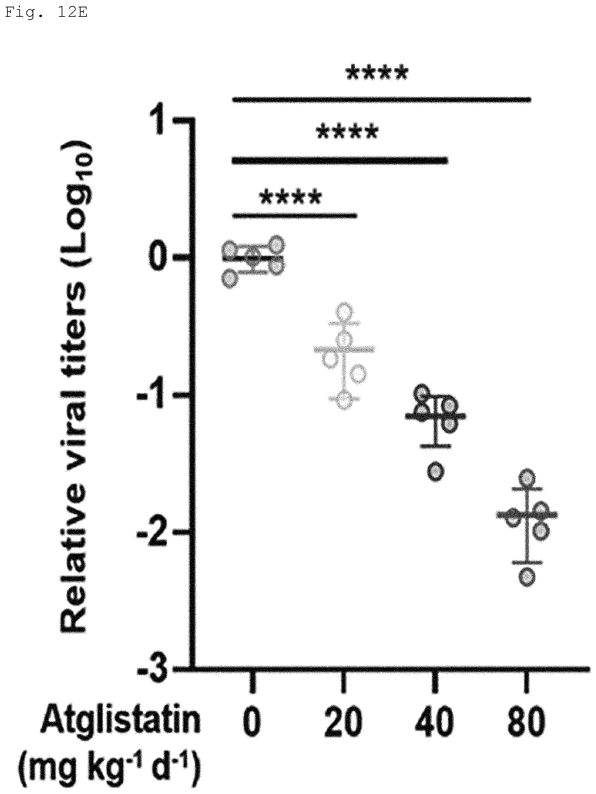

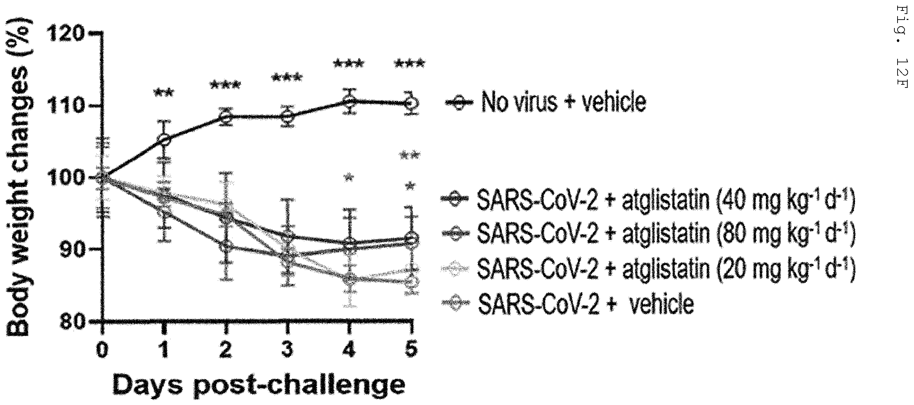

For use in evaluating the antiviral effects of lipase inhibition in animals infected with SARS-CoV-2, atglistatin was dissolved in DMSO at a concentration of 150 mg/ml. To assess the antiviral efficacy at different concentrations, the drug was prepared at concentrations of 15, 7.5, and 3.75 mg/ml. In this regard, a mixture of DMSO:40% PEG:H₂O at a ratio of 1:4:5 was used as a solvent. The drug was completely dissolved by vortexing. When administered to hamsters, 800 µL of the solutions accounted for doses of 80, 40, and 20 mg kg-¹ day⁻¹, respectively (FIG. 12A).

For an assay for the antiviral efficacy of atglistatin, a total of five experimental groups were established as follows: a non-injected and untreated group (No virus + vehicle), a group injected with SARS-CoV-2 KCDC03 strain and untreated (SARS-CoV-2 + vehicle), a group injected with SARS-CoV-2 KCDC03 strain and treated with 20 mg/kg/day atglistatin (SARS-CoV-2 + atglistatin (20 mg kg⁻¹ day⁻¹)), a group injected with SARS-CoV-2 KCDC03 strain and treated with 40 mg/kg/day atglistatin (SARS-CoV-2 + atglistatin (40 mg kg⁻¹ day⁻¹)), and a group injected with SARS-CoV-2 KCDC03 strain and treated with 80 mg/kg/day atglistatin (SARS-CoV-2 + atglistatin (80 mg kg-¹ day-¹)). Each group of five female Golden Syrian hamsters, each 12 weeks old, was acclimatized for one week under the described conditions and used in the experiment at 13 weeks of age (FIG. 12A).

Inoculation of the SARS-CoV-2 KCDC03 strain was performed in the same manner as previous experiments, with an intratracheal administration of 100,000 TCID50 per hamster under anesthesia. The non-virus-inoculated group received only phosphate-buffered saline. Subsequently, the hamsters were observed for five days, with daily monitoring and recording of survival rates, body weight, and clinical symptoms.

Drug treatment began immediately after the inoculation of SARS-CoV-2. The drugs were injected intraperitoneally using a syringe, twice daily at 12-hour intervals in the morning and afternoon, at the consistent certain time each day, for a total of four days, with each dose being 400 µL. For the untreated group, a mixture of the drug diluent DMSO/40% PEG/H₂O was administered intraperitoneally .

Necropsy was performed on the fifth day post-infection. Firstly, the entire lung tissue was photographed with a camera. The left lobe of the excised lung was fixed at 4°C for one week in 4% paraformaldehyde for histopathological analysis, followed by preparation into frozen sections. The right cranial, middle, caudal, and accessory lobes were all minced together for the assessment of cytoplasmic free fatty acids and glycerol, viral genome level, and progeny virus production.

To perform the free fatty acid assay in the tissues, they were mixed with chloroform containing 1% Triton-X 100, followed by a process identical to the previously mentioned intracellular free fatty acid assay. Absorbance was read at 570 nm on an ELISA reader.

To conduct the glycerol assay, the tissue mixture was combined with Glycerol Assay Buffer and then processed in the same manner as the intracellular glycerol assay, with absorbance measured at 570 nm using an ELISA reader.

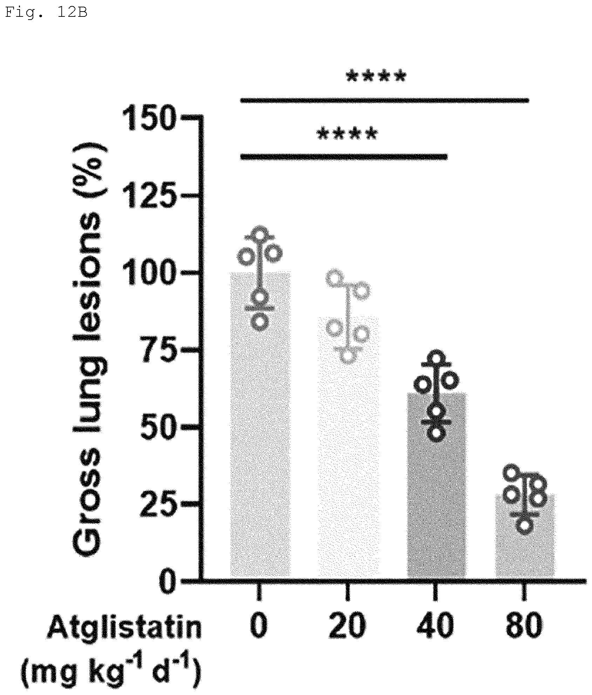

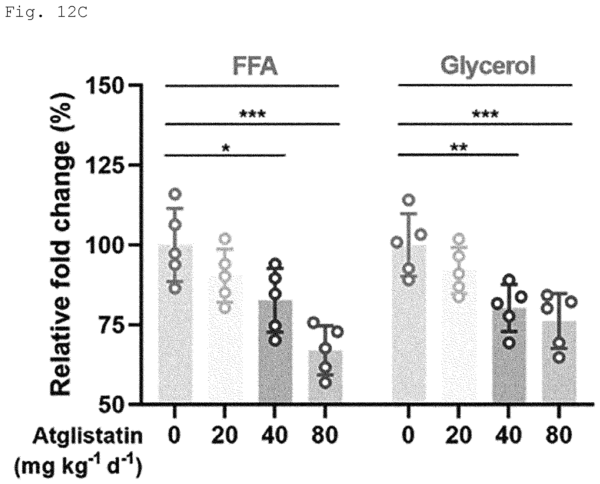

As shown in FIGS. 12b and 12c, the administration of the lipase inhibitor atglistatin to hamsters infected with SARS-CoV-2 resulted in alleviating pneumonia severity in a dose-dependent manner and significantly reducing the production of free fatty acids and glycerol. Notably, the rate of alleviating pneumonia severity reached 73% in the group treated with atglistatin at a dose of 80 mg kg⁻¹ day⁻¹ for 4.5 days.

Evaluation was made of the influence of atglistatin treatment on the number of viral genes and progeny viruses in lung tissue. Additionally, the alleviative effect of atglistatin on pneumonia caused by SARS-CoV-2 infection was assessed through histopathological analysis of hamster lung tissue, immunohistochemical staining analysis for viral antigens, and immunofluorescence staining analysis for viral antigens and lipid droplets, as described below.

The inhibitory effect of the drug on viral gene replication was evaluated by performing real-time PCR for the nucleocapsid gene of SARS-CoV-2 and the β-actin gene of the host to quantitate the viral gene.

For use in a cell culture immunofluorescence antibody assay, 100 mg of the hamster lung tissue was homogenized using a Precellys 23 homogenizer purchased from Bertin Technologies (Montigny-le-Bretonneux, France) with beads, followed by centrifugation to collect the supernatant. The obtained supernatant was serially diluted and inoculated onto monolayer-cultured Vero E6 cells in a 96-well plate. After 12 hours of incubation, an immunofluorescence antibody assay was performed using an antibody against the nucleocapsid of SARS-CoV-2 to evaluate the inhibitory effect of each drug on the proliferation of progeny viruses.

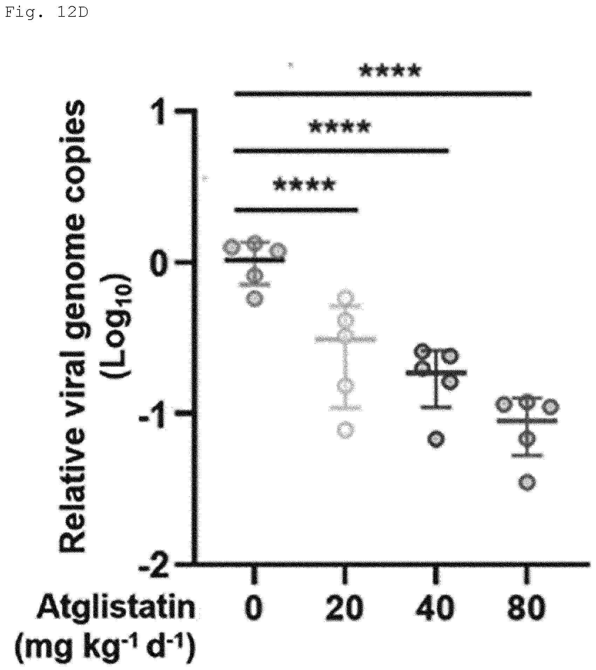

As shown in FIGS. 12d to 12f, administration of the lipase inhibitor atglistatin to hamsters infected with SARS-CoV-2 resulted in a significant decrease in the number of viral genes (d) and progeny viruses (e) in lung tissue, ultimately ceasing weight loss (f) in hamsters.

To evaluate lipid droplets and SARS-CoV-2 antigens in the lung tissue, 3-um cryosections were prepared from the fixed tissue. After blocking with 5% BSA, the sections were incubated with a primary antibody against the SARS-CoV-2 spike antigen at 4°C for 16 hours and then with AF594-conjugated mouse IgG(H+L) secondary antibody at 20°C for one hour. The tissue sections were washed with phosphate-buffered saline and then stained with 10 µM BODIPY at 20°C for 15 minutes for lipid droplet staining, followed by washing and mounting with Slow-Fade Gold antifade reagent for nuclear staining.

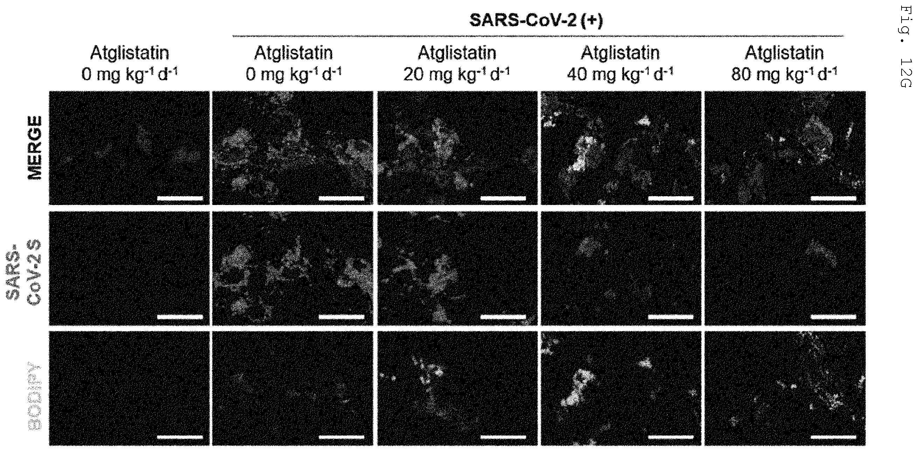

As can be seen in FIG. 12G, the administration of the lipase inhibitor atglistatin to hamsters infected with SARS-CoV-2 resulted in a significant decrease in the level of viral antigens in the alveolar epithelial cells of the hamsters in the experimental group treated with the drug post-infection, compared to the untreated virus-inoculated group. However, the number of lipid droplets did not decrease significantly.

For histopathological analysis of the lung tissue, the fixed tissue was dehydrated through graded alcohols, cleared in xylene, and embedded in paraffin before being sectioned at 3 um thickness. The tissue sections were deparaffinized in xylene, rehydrated through graded alcohols and water, and then stained with Hematoxylin & Eosin. After dehydration through graded alcohols and clearing in xylene, the tissue sections were mounted. Histological examination was then performed under an optical microscope

Immunohistochemistry was performed for SARS-CoV-2 nucleocapsid antigens in the lung tissue. Paraffin-embedded tissue sections, each 3 um thick, were deparaffinized, washed with water, rehydrated, and autoclaved in 1 mM citrate solution, pH 6.0 for antigen retrieval. Afterwards, the sections were treated with 3% H₂O₂ to quench endogenous peroxidase activity, blocked with 5% BSA, and then incubated for 16 hours at 4°C with a primary antibody against SARS-CoV-2 nucleocapsid (N), purchased from Prosci (San Diego, CA, USA). The next day, after washing with phosphate-buffered saline, the sections were incubated with the secondary antibody HRP-conjugated goat IgG (H+L) and subjected to chromogenesis with DAB.

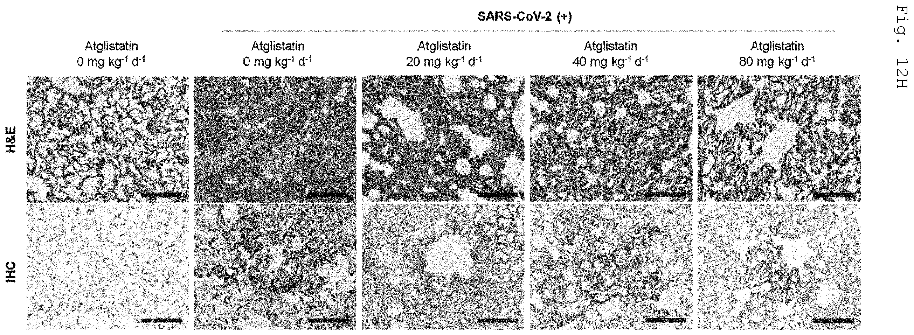

As shown in FIG. 12H, administration of the lipase inhibitor atglistatin to SARS-CoV-2-infected hamsters resulted in alleviating and reducing lung lesions, including interstitial pneumonia, and the number of virus-positive cells, compared to the mice in the untreated, virus-inoculated group.

Consequently, the lipase inhibitor atglistatin was effective in protecting hamsters infected with SARS-CoV-2. When administered at a dose of 80 mg kg⁻¹ day⁻¹, atglistatin the severity of pneumonia by 73%. These results indicate that atglistatin blocks the degradation of lipid droplets to suppress the formation of free fatty acids, which leads to inhibiting viral proliferation.

### EXPERIMENT EXAMPLE 9: Effects of Administration of Atglistatin and Anti-Influenza A Virus (IAV) Agent Either Individually or in Combination on Survival Rate, Weight Loss, Clinical Symptoms, Virus Proliferation, and Lesion in Infected Mice and Effects of Administration of Atglistatin and Anti-SARS-CoV-2 Agent Either Individually or in Combination on Survival Rate, Weight Loss, Clinical Symptoms, Virus Proliferation, and Lesion in Infected Hamsters

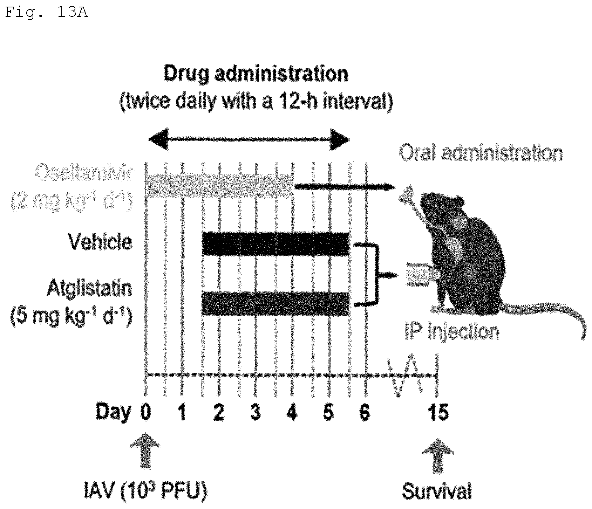

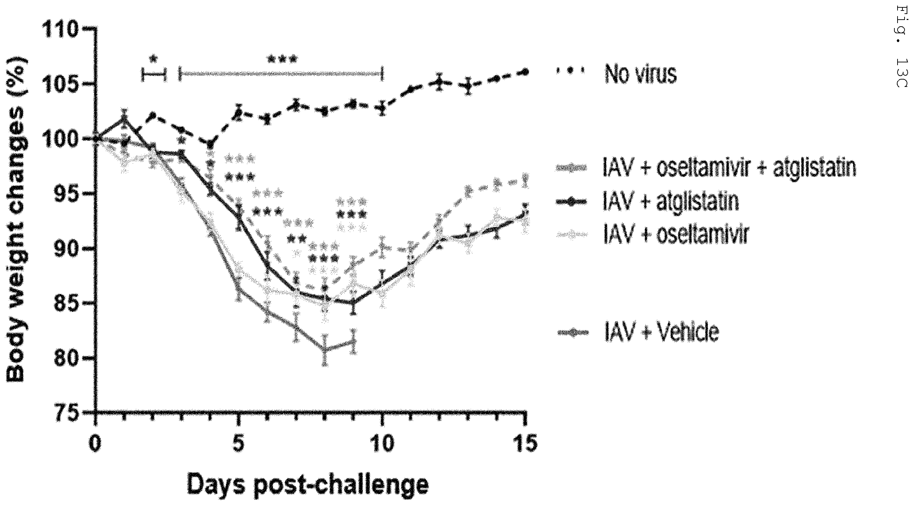

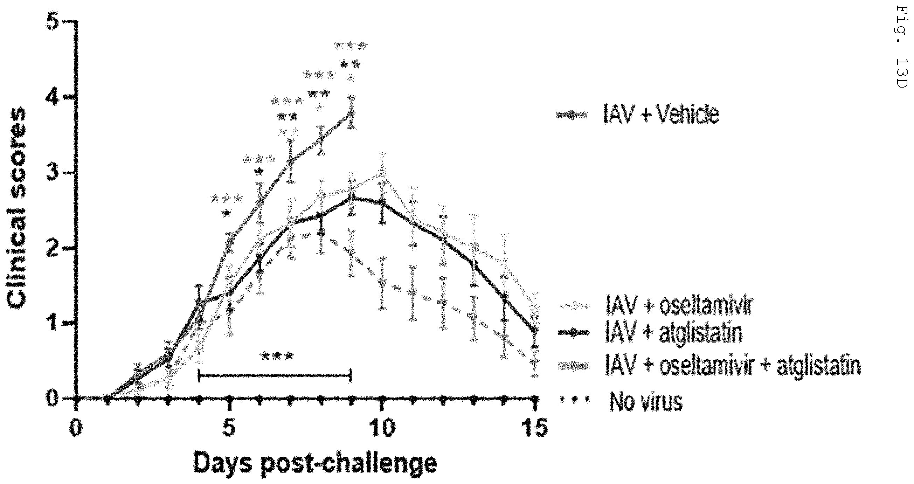

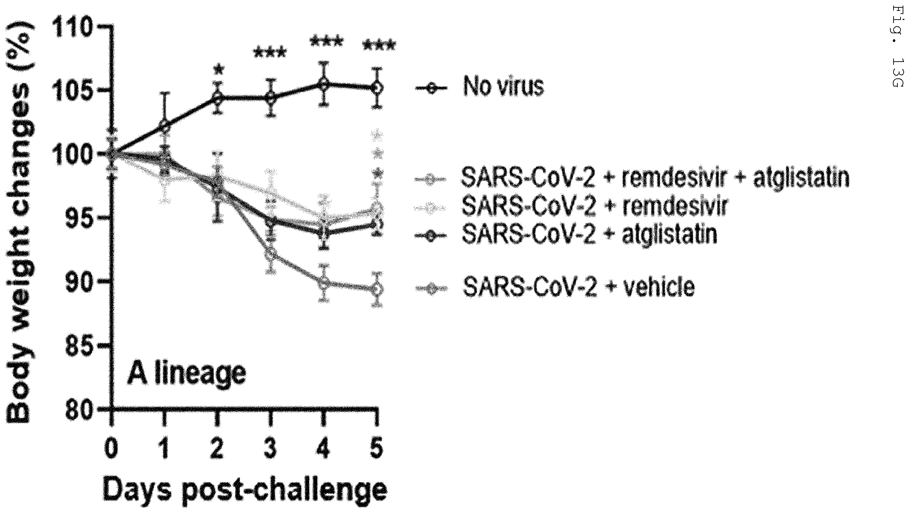

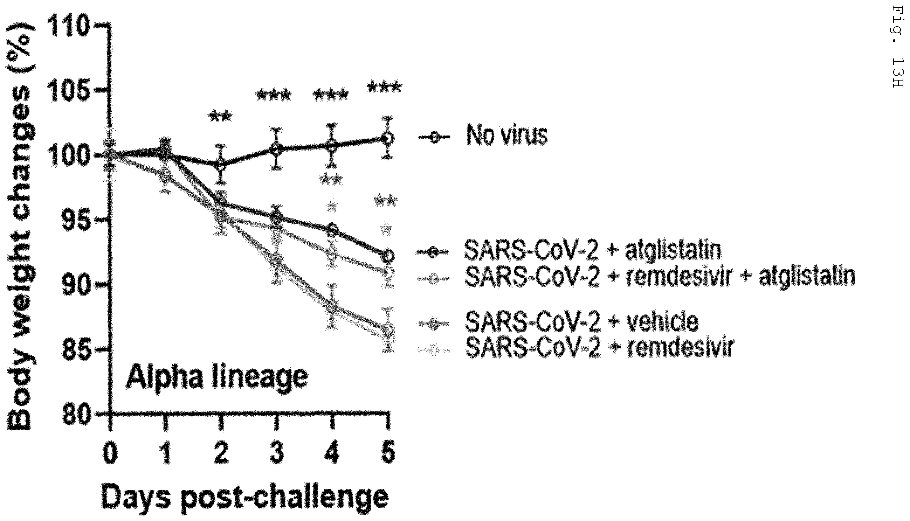

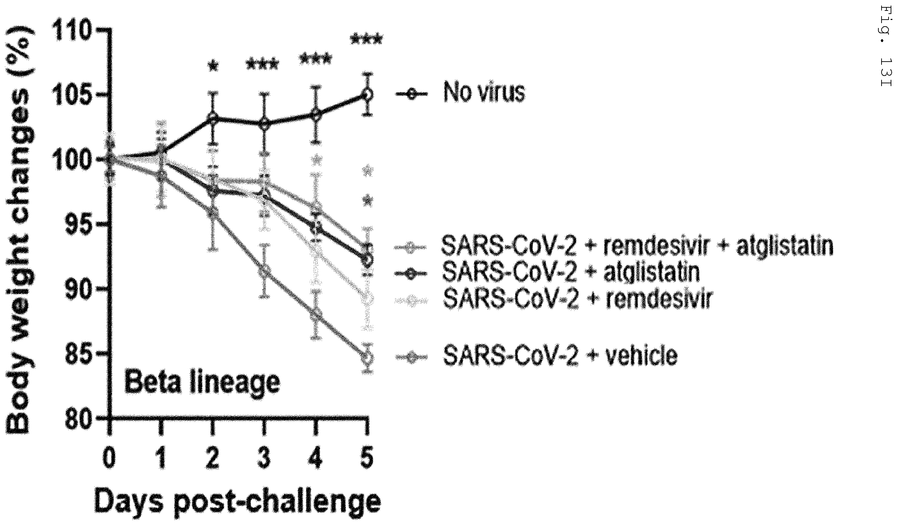

For use in verifying the antiviral effect when administering the lipase inhibitor and the antiviral agent oseltamivir either individually or in combination in influenza A virus-infected animals, 150 mg/ml of atglistatin and 100 mg/ml of oseltamivir were dissolved in DMSO and H₂O, respectively. To evaluate the antiviral effect depending on the concentration, 0.5 mg/ml atglistatin and 0.2 mg/ml oseltamivir were prepared. In this regard, a mix of DMSO:40% PEG:H₂O at a ratio of 1:4:5 or H₂O was used as a solvent. When administered to mice, 200 µL of the solutions accounted for doses of 5 mg kg-¹ day⁻¹ for atglistatin and 2 mg kg-¹ day⁻¹ for oseltamivir (see FIG. 13A).

For an assay for antiviral efficacy when administering atglistatin and oseltamivir either individually or in combination, a total of five experimental groups were established as follows: a non-injected and untreated group (No virus + vehicle), a group injected with influenza A virus PR8 strain and untreated (IAV + vehicle), a group injected with influenza A virus PR8 strain and treated with 2 mg/kg/day oseltamivir (IAV + oseltamivir), a group injected with influenza A virus PR8 strain and treated with 5 mg/kg/day atglistatin (IAV + atglistatin), and a group injected with influenza A virus PR8 strain and treated with 2 mg/kg/day oseltamivir and 5 mg/kg/day atglistatin (IAV + oseltamivir + atglistatin). Each group of 16 female wild-type C57BL/6J mice, each seven weeks old, was acclimatized for one week under the described conditions and used in the experiment at eight weeks of age.

Influenza A virus inoculation was carried out on anesthetized mice via an intranasal route at 1,000 PFU per mouse, following the same procedure as above. In cases where the virus was not injected, only phosphate-buffered saline was administered. Observations were made for 15 days, during which survival rates, body weight, and clinical symptoms were monitored and recorded daily. Mice that showed severe weight loss (more than 30%) were euthanized and recorded as deaths due to viral infection.

Oseltamivir treatment began immediately after the inoculation of influenza A virus. The drugs were orally administered using an oral zonde, twice daily at 12-hour intervals in the morning and afternoon, at the consistent certain time each day, for a total of four days, with each dose being 100 µL. Atglistatin treatment commenced two days after inoculation. The lipase inhibitor was intraperitoneally injected at a dose of 100 µL, twice daily at 12-hour intervals in the morning and afternoon at the consistent certain time each day, for a total of four days, with the aid of a syringe. For the untreated group, a mixture of the drug diluent DMSO/40% PEG/H₂O was administered intraperitoneally (see FIG. 13A).

The survival rate, which was 100% with a 0% mortality rate on day 0 of the infection, was measured daily over 15 days, and compared with the survival rate of the virus-injected and untreated group. Statistical significance was analyzed using the Log Rank Test, with a p value of 0.05 or higher considered significant.

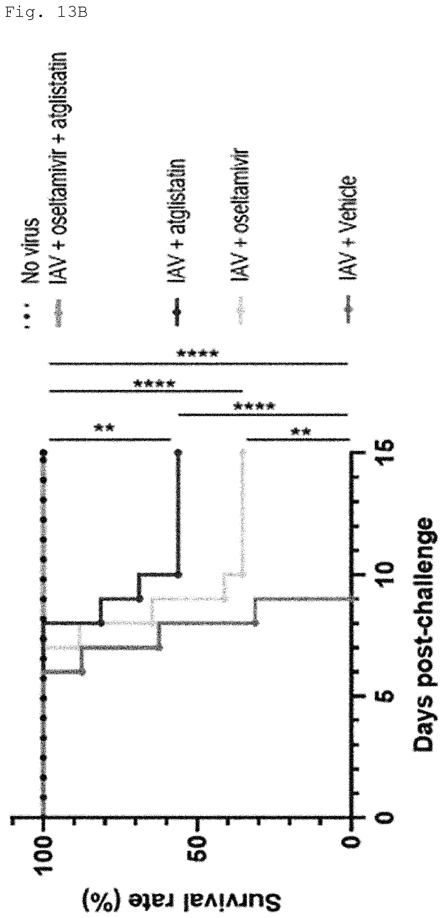

As seen in FIGS. 13b to 13d, administration of oseltamivir and atglistatin in combination exhibited 100% survival rate and resulted in an improvement in survival rate, weight loss, and clinical symptoms, compared to administration of either oseltamivir or atglistatin, with a notable therapeutic effect on influenza A virus infections.

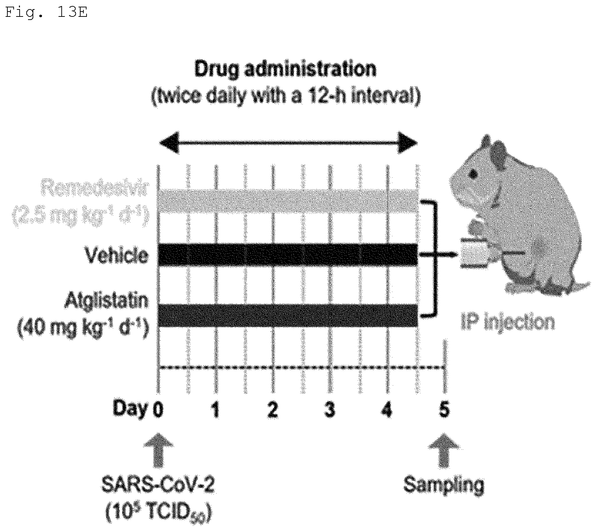

For use in verifying the antiviral effect when administering the lipase inhibitor atglistatin and the antiviral agent remdesivir individually or in combination in animals infected with SARS-CoV-2 KCDC03 strain (A lineage), KDCA51463 strain (Alpha lineage), or KDCA55905 strain (Beta lineage), 150 mg/ml of atglistatin and 50 mg/ml of remdesivir were dissolved in DMSO and H₂O, respectively. To evaluate the antiviral effect depending on the concentration, 7.5 mg/ml atglistatin and 0.47 mg/ml remdesivir were prepared. In this regard, a mix of DMSO:40% PEG:H₂O at a ratio of 1:4:5 or a mix of DMSO: Corn oil at a ratio of 1:9 was used as a solvent. When administered to mice, 800 µL of the solutions accounted for doses of 40 mg kg⁻¹ day⁻¹ for atglistatin and 2.5 mg kg⁻¹ day⁻¹ for remdesivir (see FIG. 13E).

For an assay for the antiviral efficacy when administering remdesivir and atglistatin individually or in combination against SARS-CoV-2 KCDC03 strain (A lineage), KDCA51463 strain (Alpha lineage), and KDCA55905 strain (Beta lineage), a total of five experimental groups were established as follows: a non-injected and untreated group (No virus + vehicle), a group injected with SARS-CoV-2 and untreated (SARS-CoV-2 + vehicle), a group injected with SARS-CoV-2 strain and treated with 2.5 mg/kg/day remdesivir (SARS-CoV-2 + remdesivir), a group injected with SARS-CoV-2 and treated with 40 mg/kg/day atglistatin (SARS-CoV-2 + atglistatin), and a group injected with SARS-CoV-2 and treated with 2.5 mg/kg/day remdesivir and 40 mg/kg/day atglistatin (SARS-CoV-2 + remdesivir + atglistatin). Each group of 5 Golden Syrian hamsters, each 11 weeks old, was acclimatized for one week under the described conditions before use in the experiment.

The inoculation of SARS-CoV-2 KCDC03 strain (A lineage), KDCA51463 strain (Alpha lineage), and KDCA55905 strain (Beta lineage) was performed in the same manner as previous experiments, with an intratracheal administration of 100,000 TCID50 per hamster under anesthesia. The non-virus-inoculated group received only phosphate-buffered saline. Subsequently, the hamsters were observed for five days, with daily monitoring and recording of survival rates, body weight, and clinical symptoms.

Treatment with remdesivir and atglistatin began immediately after the inoculation of SARS-CoV-2. The drugs were injected intraperitoneally using a syringe, twice daily at 12-hour intervals in the morning and afternoon, at the consistent certain time each day, for a total of 4.5 days, with each dose being 400 µL. For the untreated group, a mixture of the drug diluent DMSO/40% PEG/H₂O was administered intraperitoneally.

Necropsy was performed on the fifth day post-infection. Firstly, the entire lung tissue was photographed with a camera. The left lobe of the excised lung was fixed at 4°C for one week in 4% paraformaldehyde for histopathological analysis, followed by preparation into cryosections. The right cranial, middle, caudal, and accessory lobes were all minced together for the assessment of cytoplasmic free fatty acids and glycerol, viral genome level, and progeny virus production.

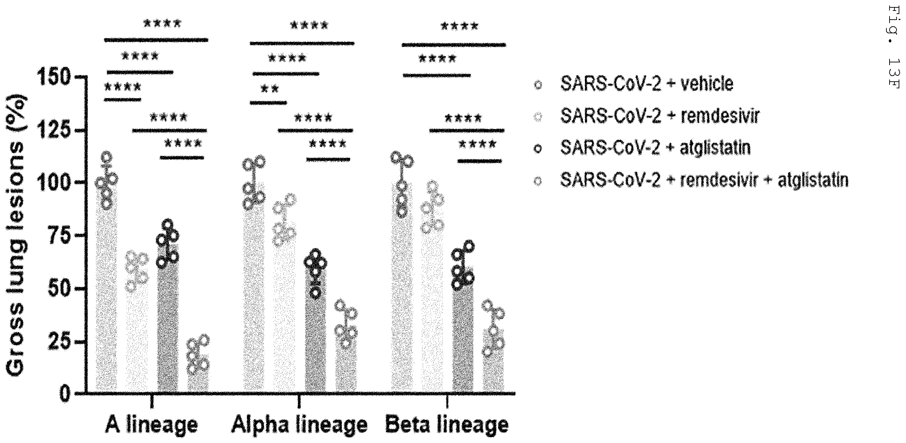

As shown in FIGS. 13f to 13i, the combined administration of remdesivir and atglistatin significantly improved pneumonia severity by 83% in SARS-CoV-2 lineage A infection, by 67% in SARS-CoV-2 lineage Alpha infection, and by 69% in SARS-CoV-2 lineage Beta infection. The combination of remdesivir and atglistatin was more effective in reducing pneumonia severity and weight loss compared to the administration of either remdesivir or atglistatin alone, showing significant therapeutic effects across various variants of SARS-CoV-2.

In conclusion, the lipase inhibitor atglistatin, when combined with oseltamivir in treating influenza A virus infections, showed a significantly greater therapeutic effect than oseltamivir alone. Similarly, when used for treating the infections of SARS-CoV-2 including various variants, a combination of the lipase inhibitor and remdesivir demonstrated a more significant therapeutic effect, compared to the administration of remdesivir alone.

### EXPERIMENTAL EXAMPLE 10: Structure and Preparation of the Lipase Inhibitor Atglistatin and Derivatives thereof

CAY10499, which is a hormone-sensitive lipase (HSL) inhibitor, was purchased from Cayman Chemicals (Ann Arbor, Michigan, USA), and atglistatin, which is an adipose triglyceride lipase (ATGL) inhibitor, was purchased from MedChemExpress (Monmouth Junction, NJ, USA).

The atglistatin derivatives 3-(4'-dimethylamino)-(1,1'-biphenyl)-3-yl)-1-ethyl-1-methylurea3-(4'-(Dimethylamino)-[1,1'-biphenyl]-3-yl)-1-ethyl-1-methylurea [DABPU-EM] and 3-(4'-dimethylamino)-(1,1'-biphenyl)-3-yl)-1,1-diethylurea 3-(4'-(Dimethylamino)-[1,1'-biphenyl]-3-yl)-1,1-diethylurea[DABPU-DE] were synthesized as follows.

### 10-1. Synthesis of N,N-dialkylcarbamic chloride

In 5.0 mL of water, 3-bromoaniline (172 mg), 4-(dimethylamino)phenyl)boronic acid (182 mg), potassium phosphate tribasic (K3PO4) (445 mg), and palladium acetate (Pd(OAc)2) (4.5 mg) were stirred together at 100°C for 2 hours. To the reaction mixture was added excess water and ethyl acetate, and the ethyl acetate layer was separated using a separatory funnel. The organic layer was dried over magnesium sulfate (MgSO4) and concentrated to obtain an intermediate which was then used for subsequent reactions in a new reaction vessel, without additional separation and purification. N,N-Dialkylcarbamic chloride or N,N-diarylcarbamic chloride (2.0 mmol) was mixed with 5.0 mL of pyridine at 40°C for 4 hours while stirring. The reaction mixture was added with water and ethyl acetate. The organic layer was extracted and concentrated. The concentrate was purified by silica gel column chromatography to afford the derivative compound.

### 10-2. Synthesis of 3-(4'-(dimethylamino)-(1,1'-biphenyl)-3-yl)-1,1-dimethylurea [DABPU-DM]

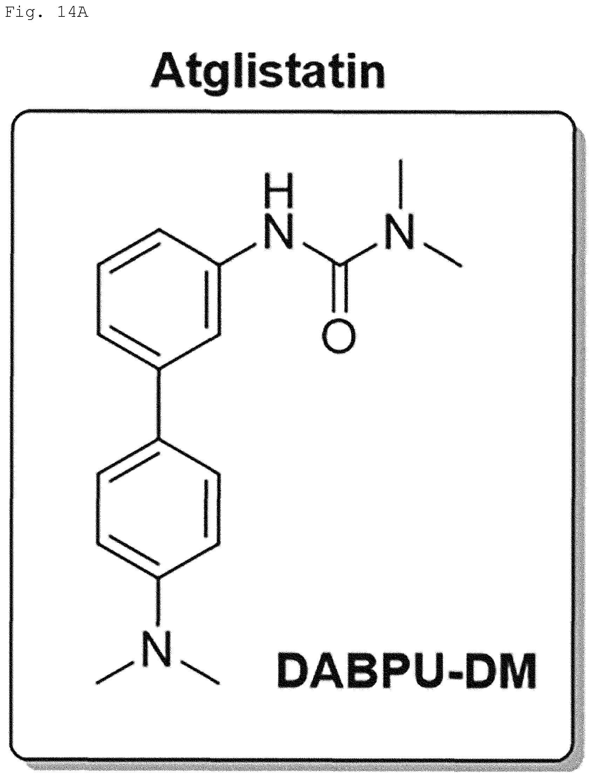

The same procedure as in Experimental Example 10-1, with the exception of using dimethylcarbamic chloride (214 mg) instead of N,N-dialkylcarbamic chloride, to afford 96 mg of the final product DABPU-DM represented in FIG. 14A (yield 34%). DABPU-DM is an abbreviation for 3-(4`-(dimethylamino)-(1,1'-biphenyl)-3-yl)-1,1-dimethylurea. Analysis data for the product is as follows.

1H NMR (500 MHz, CDCl3) δ 7.58-7.55 (m, 1H), 7.53-7.48 (m, 2H), 7.31-7.28 (m, 2H), 7.25-7.21 (m, 1H), 6.80-6.75 (m, 2H), 6.42 (s, 1H), 3.03 (s, 6H), 2.98 (s, 6H); 13C NMR (126 MHz, CDCl3) δ 155.9, 150.1, 142.0, 139.6, 129.2, 129.0, 127.8, 121.1, 117.8, 117.7, 112.8, 40.7, 36.6; MS (ESI, m/z): 283.1 [M]+

### 10-3. Synthesis of 3-(4'-(dimethylamino)-(1,1'-biphenyl)-3-yl)-1-ethyl-1-methylurea [DABPU-EM]

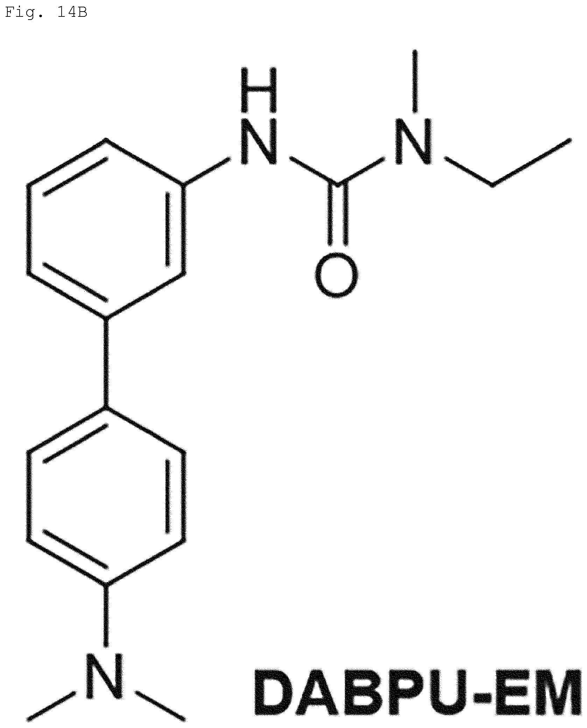

The same procedure as in Experimental Example 10-1, with the exception of using ethyl(methyl)carbamic chloride) (243 mg) instead of N,N-dialkylcarbamic chloride, to afford 122 mg of the final product DABPU-EM represented in FIG. 14B (yield 41%). DABPU-EM is an abbreviation for 3-(4'-dimethylamino)-(1,1'-biphenyl)-3-yl)-1-ethyl-1-methylurea. Analysis data for the product is as follows.

1H NMR (500 MHz, CDCl3) δ 7.59-7.57 (m, 1H), 7.53-7.48 (m, 2H), 7.33-7.27 (m, 2H), 7.24-7.21 (m, 1H), 6.80-6.74 (m, 2H), 6.45 (s, 1H), 3.41 (q, J = 7.2 Hz, 2H), 2.99 (s, 3H), 2.98 (s, 6H), 1.18 (t, J = 7.2 Hz, 3H); 13C NMR (126 MHz, CDCl3) δ 155.4, 150.1, 141.9, 139.6, 129.2, 129.0, 127.8, 121.0, 117.8, 117.7, 112.8, 43.7, 40.7, 34.0, 13.1; HRMS (FD) cacld. for C18H23N3O [M]+: 297.1841, found:297.1839.

### 10-4. Synthesis of 3-(4'-dimethylamino)-(1,1'-biphenyl)-3-yl)-1,1-diethylurea [DABPU-DE]

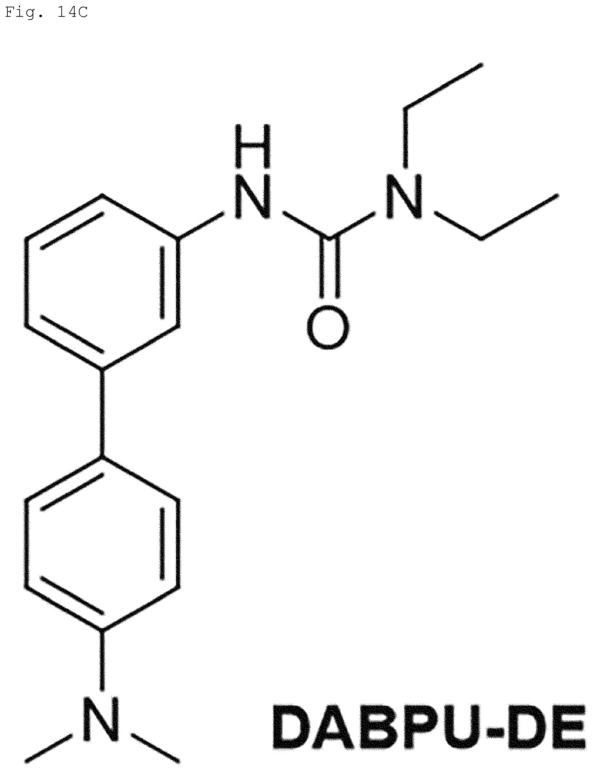

The same procedure as in Experimental Example 10-1, with the exception of using diethylcarbamic chloride) (271 mg) instead of N,N-dialkylcarbamic chloride, to afford 97 mg of the final product DABPU-DE represented in FIG. 14C (yield 31 %). DABPU-DE is an abbreviation for 3-(4'-dimethylamino)-(1,1'-biphenyl)-3-yl)-1,1-diethylurea. Analysis data for the product is as follows.

1H NMR (500 MHz, CDCl3) δ 7.59 (s, 1H), 7.50 (d, J = 8.8 Hz, 2H), 7.33-7.25 (m, 2H), 7.24-7.19 (m, 1H), 6.75 (d, J = 8.8 Hz, 2H), 6.43 (s, 1H), 3.36 (q, J = 7.1 Hz, 4H), 2.96 (s, 6H), 1.20 (t, J = 7.1 Hz, 6H); 13C NMR (126 MHz, CDCl3) δ 154.7, 150.0, 141.8, 139.7, 129.1, 129.0, 127.8, 120.8, 117.7, 117.6, 112.7, 41.7, 40.6, 14.0; HRMS (FD) cacld. for C19H25N3O [M]+: 311.1998, found:311.2001.

### 10-5. Synthesis of 3-(4'-dimethylamino)-(1,1'-biphenyl)-3-yl)-1,1-diphenylurea [DABPU-DPh]

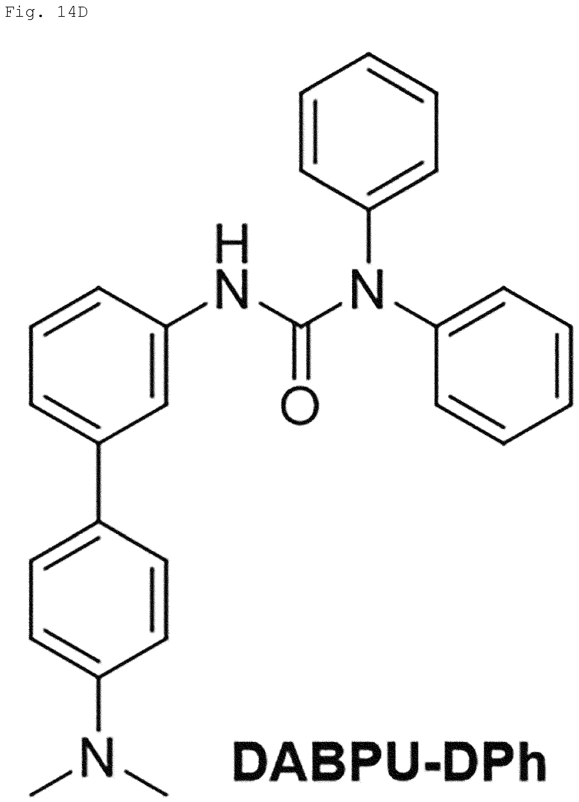

The same procedure as in Experimental Example 10-1, with the exception of using diphenylcarboamic chloride (462 mg) instead of N,N-diarylcarbamic chloride, to afford 102 mg of the final product DABPU-DPh represented in FIG. 14D (yield 25%). DABPU-DPh is an abbreviation for 3-(4'-dimethylamino)-(1,1'-biphenyl)-3-yl)-1,1-diphenylurea. Analysis data for the product is as follows.

1H NMR (500 MHz, CDCl3) δ 7.56 (t, J = 1.8 Hz, 1H), 7.50-7.46 (m, 2H), 7.43-7.39 (m, 4H), 7.38-7.34 (m, 4H), 7.31-7.26 (m, 3H), 7.25-7.20 (m, 2H), 6.76 (d, J = 8.8 Hz, 2H), 6.50 (s, 1H), 2.98 (s, 6H); 13C NMR (126 MHz, CDCl3) δ 153.7, 150.1, 142.5, 142.1, 138.9, 129.7, 129.3(2C), 127.9, 127.7, 126.8, 121.4, 117.3, 117.1, 112.8, 40.7; HRMS (FD) cacld. for C27H25N3O [M]+: 407.1998, found: 407.1974

### 10-6. Synthesis of N-(4'-dimethylamino)-(1,1'-biphenyl)-3-yl)pyrrolidine-1-carboxamide [DABPU-Pyrrol]

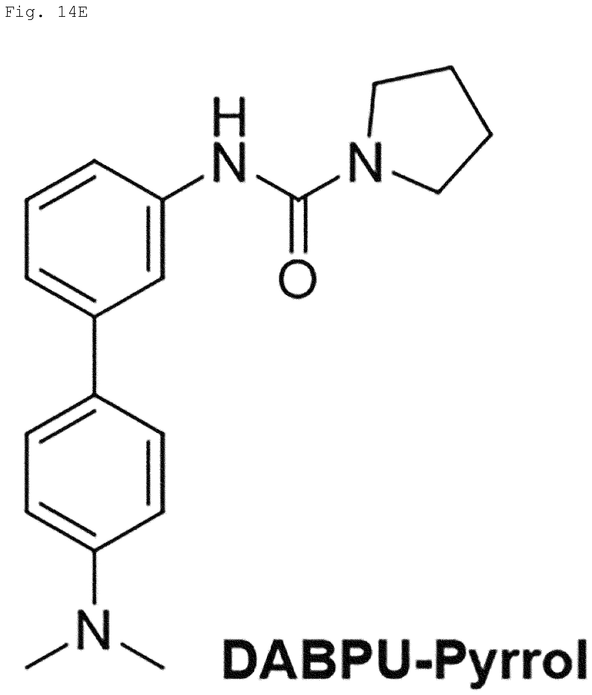

The same procedure as in Experimental Example 10-1, with the exception of using Pyrrolidine-1-carbonyl chloride)(266 mg) instead of N,N-dialkylcarbamic chloride, to afford 108 mg of the final product DABPU-Pyrrol represented in FIG. 14E (yield 35%). DABPU-Pyrrol is an abbreviation for N-(4'-dimethylamino)-(1,1'-biphenyl)-3-yl)pyrolidine-1-carboxamide. Analysis data for the product is as follows.

1H NMR (500 MHz, CDCl3) δ 7.61 (t, J = 1.8 Hz, 1H), 7.55-7.47 (m, 2H), 7.36-7.27 (m, 2H), 7.24-7.19 (m, 1H), 6.82-6.72 (m, 2H), 6.26 (s, 1H), 3.47 (t, J = 6.7 Hz, 4H), 2.98 (s, 6H), 1.99-1.92 (m, 4H); 13C NMR (126 MHz, CDCl3) δ 154.1, 150.1, 142.0, 139.6, 129.2, 129.1, 127.8, 120.9, 117.5, 117.4, 112.8, 45.9, 40.7, 25.7; HRMS (FD) cacld. for C19H23N3O [M]+: 309.1841 found: 309.1843

### 10-7. Synthesis of N-(4'-(dimethylamino)-(1,1'-biphenyl)-3-yl)morpholine-4-carboxamide [DABPU-Morph]

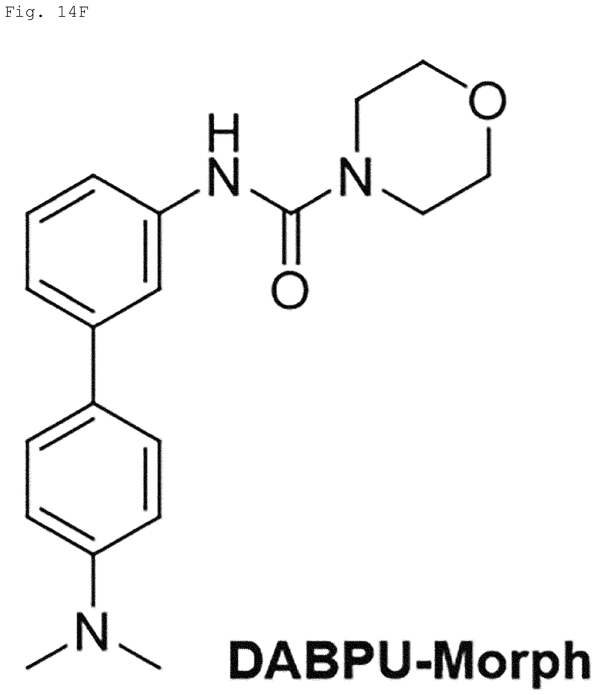

The same procedure as in Experimental Example 10-1, with the exception of using morpholine-4-carbonyl chloride (299 mg) instead of N,N-dialkylcarbamic chloride, to afford 140 mg of the final product DABPU-Morph represented in FIG. 14F (yield 43%) . DABPU-Morph is an abbreviation for N-(4'-(dimethylamino)-(1,1'-biphenyl)-3-yl)morpholine-4-carboxamide. Analysis data for the product is as follows.

1H NMR (500 MHz, CDCl3) δ 7.53 (t, J = 1.8 Hz, 1H), 7.51-7.46 (m, 2H), 7.33-7.28 (m, 1H), 7.28-7.26 (m, 1H), 7.26-7.23 (m, 1H), 6.82-6.70 (m, 2H), 6.59 (s, 1H), 3.69 (t, J = 4.9 Hz, 4H), 3.46 (t, J = 4.9 Hz, 4H), 2.98 (s, 6H); 13C NMR (126 MHz, CDCl3) δ 155.4, 150.1, 142.1, 139.2, 129.3, 128.8, 127.8, 121.5, 118.2, 118.1, 112.8, 66.6, 44.4, 40.7; MS (ESI, m/z): 325.2 [M]+

### EXPERIMENTAL EXAMPLE 11: Inhibition of Atglistatin (DABPU-DM) Derivatives DABPU-EM and DABPU-DE against Proliferation of SARS-CoV-2, Influenza A Virus (IAV), Bovine Coronavirus (BCoV), Porcine Epidemic Diarrhea Coronavirus (PEDV), Bovine Species A Rotavirus (RVA), Porcine Reproductive and Respiratory Syndrome Virus (PRRSV), and Porcine sapovirus (PSaV)

For use in verifying that antiviral activity against SARS-CoV-2, influenza A virus, bovine coronavirus, porcine epidemic diarrhea coronavirus, bovine species A rotavirus, porcine reproductive and respiratory syndrome virus, and porcine sapovirus is induced by inhibiting lipases, CAY10499 and atglistatin were each prepared at concentrations of 1 mM, 10 mM, and 20 mM in DMSO.

To achieve the goal, the monolayer of Vero E6 cells in 6-well plates and 8-well chambers were inoculated with SARS CoV-2 KCDC03 strain at a multiplicity of infection of 0.1 ffu/cell. The virus was incubated at 37°C for 18 hours, after which the medium was changed with a medium containing DABPU-EM or DABPU-DE at a final concentration of 1 µM, 10 µM, or 20 µM. Following an additional 18 hours of incubation, the Vero E6 cells in 6-well plates were washed with phosphate-buffered saline, collected by scaping, and centrifuged. The cell pellet thus obtained was subjected to Western blot analysis. The intensity of the SARS-CoV-2 spike protein detected was assessed by comparison with the relative positive signals of the internal control beta-actin using the ImageJ software. Separately, the Vero E6 cells in 8-well chambers were washed with phosphate-buffered saline, fixed with 4% paraformaldehyde, and then subjected to immunofluorescence staining for SARS-CoV-2 spike protein in the same manner as described in the foregoing.

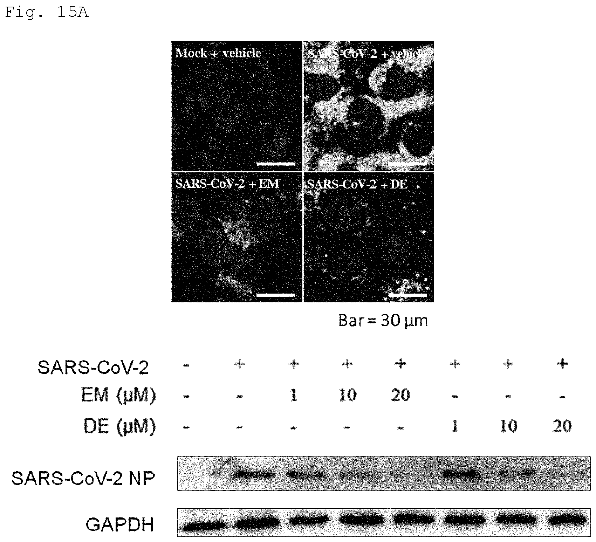

As can be seen in FIG. 15A, the expression levels of the viral antigen in infected cells demonstrated the inhibitory activity of DABPU-EM or DABPU-DE in a dose-dependent manner against SARS-CoV-2, with higher inhibitory effects of DABPU-DE than DABPU-EM.

To achieve the goal, the monolayer of A549 cells in 6-well plates and 8-well chambers were inoculated with influenza A virus PR8 strain at a multiplicity of infection of 0.1 ffu/cell. The virus was incubated at 37°C for 12 hours, after which the medium was changed with a medium containing DABPU-EM or DABPU-DE at a final concentration of 1 µM, 10 µM, or 20 µM. Following an additional 12 hours of incubation, the A549 cells in 6-well plates were washed with phosphate-buffered saline, collected by scaping, and centrifuged. The cell pellet thus obtained was subjected to Western blot analysis. The intensity of the influenza A virus M2 protein detected was assessed by comparison with the relative positive signals of the internal control beta-actin using the ImageJ software. Separately, the A549 cells in 8-well chambers were washed with phosphate-buffered saline, fixed with 4% paraformaldehyde, and then subjected to immunofluorescence staining for influenza A virus M2 protein in the same manner as described in the foregoing.

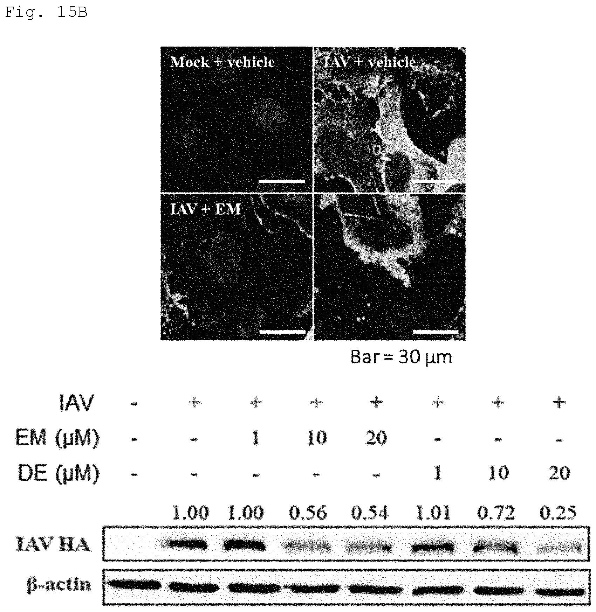

As can be seen in FIG. 15B, the expression levels of the viral antigen in infected cells demonstrated the inhibitory activity of DABPU-EM or DABPU-DE in a dose-dependent manner against influenza A virus, with higher inhibitory effects of DABPU-DE than DABPU-EM.

To achieve the goal, the monolayer of HRT-18G cells in 6-well plates and 8-well chambers were inoculated with bovine coronavirus KWD20 strain at a multiplicity of infection of 0.1 ffu/cell. The virus was incubated at 37°C for 18 hours, after which the medium was changed with a medium containing DABPU-EM or DABPU-DE at a final concentration of 1 µM, 10 µM, or 20 µM. Following an additional 18 hours of incubation, the HRT-18G cells in 6-well plates were washed with phosphate-buffered saline, collected by scaping, and centrifuged. The cell pellet thus obtained was subjected to Western blot analysis. The intensity of the bovine coronavirus spike protein detected was assessed by comparison with the relative positive signals of the internal control beta-actin using the ImageJ software. Separately, the HRT-18G cells in 8-well chambers were washed with phosphate-buffered saline, fixed with 4% paraformaldehyde, and then subjected to immunofluorescence staining for bovine coronavirus KWD20 spike protein in the same manner as described in the foregoing.

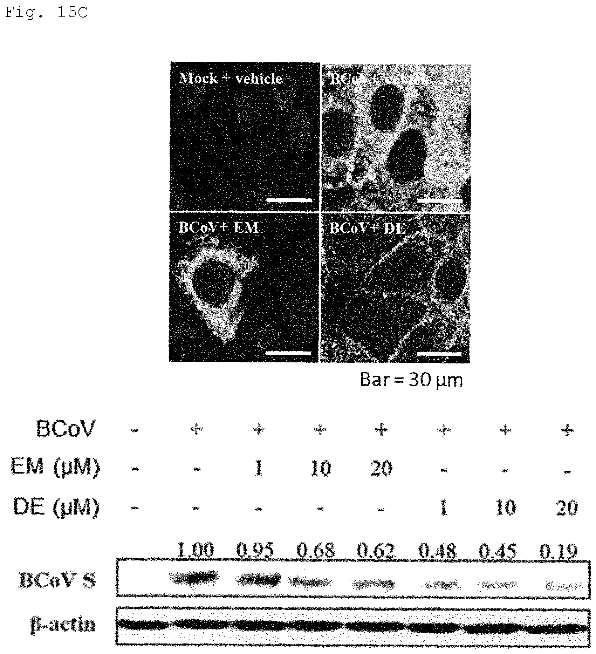

As can be seen in FIG. 15C, the expression levels of the viral antigen in infected cells demonstrated the inhibitory activity of DABPU-EM or DABPU-DE in a dose-dependent manner against bovine coronavirus, with higher inhibitory effects of DABPU-DE than DABPU-EM.

To achieve the goal, the monolayer of Vero E6 cells in 6-well plates and 8-well chambers were inoculated with porcine epidemic diarrhea coronavirus QIAP1401 strain at a multiplicity of infection of 0.1 ffu/cell. The virus was incubated at 37°C for 18 hours, after which the medium was changed with a medium containing DABPU-EM or DABPU-DE at a final concentration of 1 µM, 10 µM, or 20 µM. Following an additional 18 hours of incubation, the Vero E6 cells in 6-well plates were washed with phosphate-buffered saline, collected by scaping, and centrifuged. The cell pellet thus obtained was subjected to Western blot analysis. The intensity of the porcine epidemic diarrhea coronavirus nucleocapsid protein detected was assessed by comparison with the relative positive signals of the internal control beta-actin using the ImageJ software. Separately, the Vero E6 cells in 8-well chambers were washed with phosphate-buffered saline, fixed with 4% paraformaldehyde, and then subjected to immunofluorescence staining for porcine epidemic diarrhea coronavirus nucleocapsid protein in the same manner as described in the foregoing.

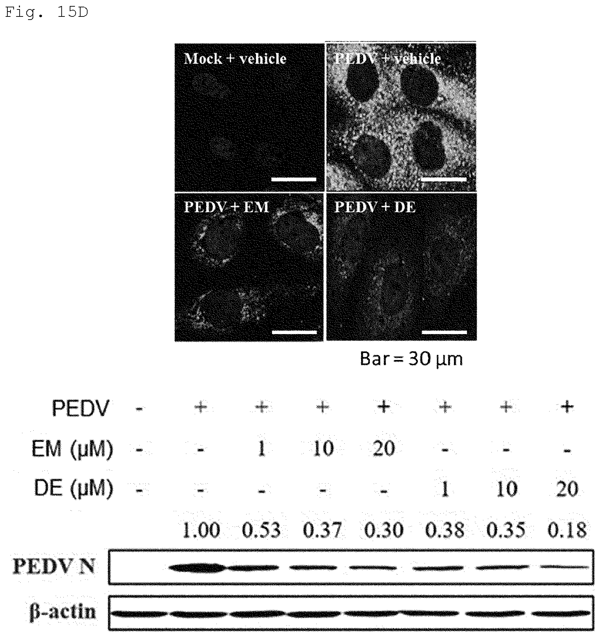

As can be seen in FIG. 15D, the expression levels of the viral antigen in infected cells demonstrated the inhibitory activity of DABPU-EM or DABPU-DE in a dose-dependent manner against porcine epidemic diarrhea coronavirus, with higher inhibitory effects of DABPU-DE than DABPU-EM.

To achieve the goal, the monolayer of MA104 cells in 6-well plates and 8-well chambers were inoculated with bovine species A rotavirus NCDV strain at a multiplicity of infection of 0.1 ffu/cell. The virus was incubated at 37°C for 12 hours, after which the medium was changed with a medium containing DABPU-EM or DABPU-DE at a final concentration of 1 µM, 10 µM, or 20 µM. Following an additional 12 hours of incubation, the MA104 cells in 6-well plates were washed with phosphate-buffered saline, collected by scaping, and centrifuged. The cell pellet thus obtained was subjected to Western blot analysis. The intensity of the bovine species A rotavirus VP6 protein detected was assessed by comparison with the relative positive signals of the internal control beta-actin using the ImageJ software. Separately, the MA104 cells in 8-well chambers were washed with phosphate-buffered saline, fixed with 4% paraformaldehyde, and then subjected to immunofluorescence staining for bovine species A rotavirus VP6 protein in the same manner as described in the foregoing.

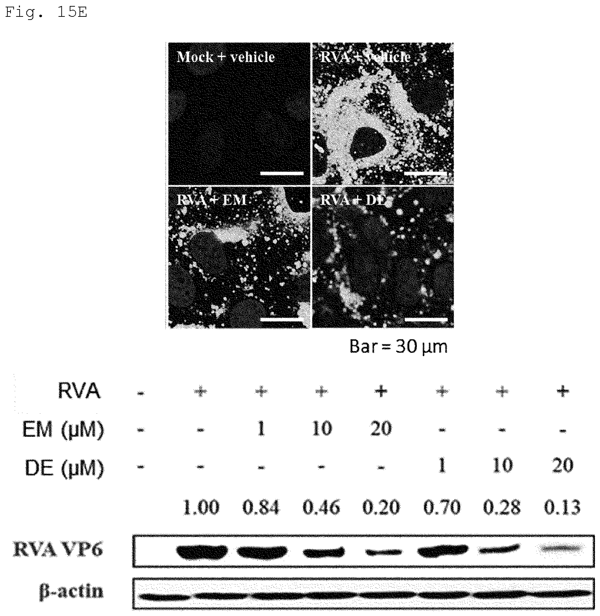

As can be seen in FIG. 15E, the expression levels of the viral antigen in infected cells demonstrated the inhibitory activity of DABPU-EM or DABPU-DE in a dose-dependent manner against bovine species A rotavirus, with higher inhibitory effects of DABPU-DE than DABPU-EM.

To achieve the goal, the monolayer of MARC-145 cells in 6-well plates and 8-well chambers were inoculated with porcine reproductive and respiratory syndrome virus LMY strain at a multiplicity of infection of 0.1 ffu/cell. The virus was incubated at 37°C for 24 hours, after which the medium was changed with a medium containing DABPU-EM or DABPU-DE at a final concentration of 1 µM, 10 µM, or 20 µM. Following an additional 24 hours of incubation, the MARC-145 cells in 6-well plates were washed with phosphate-buffered saline, collected by scaping, and centrifuged. The cell pellet thus obtained was subjected to Western blot analysis. The intensity of the porcine reproductive and respiratory syndrome virus matrix protein detected was assessed by comparison with the relative positive signals of the internal control beta-actin using the ImageJ software. Separately, the MARC-145 cells in 8-well chambers were washed with phosphate-buffered saline, fixed with 4% paraformaldehyde, and then subjected to immunofluorescence staining for porcine reproductive and respiratory syndrome virus matrix protein in the same manner as described in the foregoing.

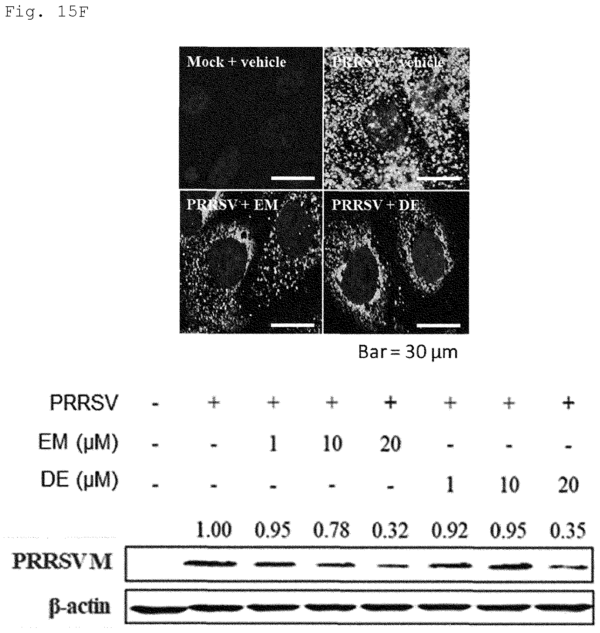

As can be seen in FIG. 15F, the expression levels of the viral antigen in infected cells demonstrated the inhibitory activity of DABPU-EM or DABPU-DE in a dose-dependent manner against bovine species A rotavirus, with higher inhibitory effects of DABPU-DE than DABPU-EM.

To achieve the goal, the monolayer of LLC-PK cells in 6-well plates and 8-well chambers were inoculated with porcine sapovirus Cowden strain at a multiplicity of infection of 0.1 ffu/cell. The virus was incubated at 37°C for 24 hours, after which the medium was changed with a medium containing DABPU-EM or DABPU-DE at a final concentration of 1 µM, 10 µM, or 20 µM. Following an additional 18 hours of incubation, the LLC-PK cells in 6-well plates were washed with phosphate-buffered saline, collected by scaping, and centrifuged. The cell pellet thus obtained was subjected to Western blot analysis. The intensity of the porcine sapovirus VPg protein detected was assessed by comparison with the relative positive signals of the internal control beta-actin using the ImageJ software. Separately, the LLC-PK cells in 8-well chambers were washed with phosphate-buffered saline, fixed with 4% paraformaldehyde, and then subjected to immunofluorescence staining for porcine sapovirus VPg protein in the same manner as described in the foregoing.

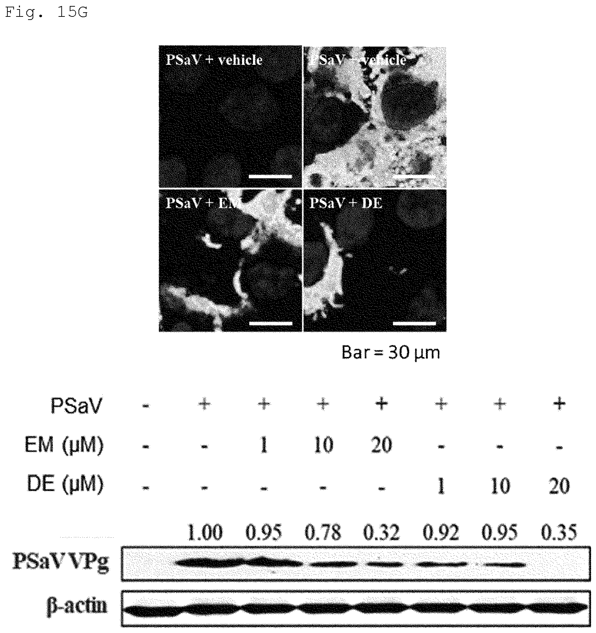

As can be seen in FIG. 15G, the expression levels of the viral antigen in infected cells demonstrated the inhibitory activity of DABPU-EM or DABPU-DE in a dose-dependent manner against porcine sapovirus, with higher inhibitory effects of DABPU-DE than DABPU-EM.

### EXPERIMENTAL EXAMPLE 12: Half-Maximal Cytotoxic Concentration, Half-Maximal Inhibitory Concentration, and Selective Index of Lipase Inhibitor Atglistatin (DABPU-DM) Derivatives DABPU-EM and DABPU-DE

The atglistatin derivatives DABPU-EM and DABPU-DE were examined for half-maximal cytotoxic concentration.

**TABLE 8**

| Half-maximal cytotoxic concentration (CC50) of atglistatin derivatives DABPU-EM and DABPU-DE | | |
|---|---|---|
| Cell line | CC₅₀ (µM) | |
| | DABPU-EM | DABPU-DE |
| A549 | 206.3 | 213.3 |
| Vero E6 | 209.7 | 222.2 |
| HRT18G | 186.1 | 177.4 |
| MA104 | 223.3 | 208.8 |
| MARC | 209.7 | 216.5 |
| LLC-PK | 231.2 | 225.5 |

As seen in Table 8, DABPU-EM was measured to have half-maximal cytotoxic concentrations of as high as 186.1 to 231.2 against A549, Vero E6, MDCK, HRT18G, MA04, MARC, and LLC-PK cells. The half-maximal cytotoxic concentration of DABPU-DE against A549. Vero E6, MDCK, HRT18G, MA04, MARC, and LLC-PK cells were slightly lower than that of DABPU-EM, ranging from 177.4 to 225.5.

**TABLE 9**

| Half-maximal inhibitory concentration and selective index of atglistatin inhibitors DABPU-EM and DABPU-DE | | | | | |
|---|---|---|---|---|---|
| Virus family | Virus (strain) | IC₅₀ (µM) | | SI (CC₅₀/IC₅₀)^{a} | |
| | | DABPU-EM | DABPU-DE | DABPU-EM | DABPU-DE |
| *Orthomyxovi ridae* | Influenza A virus (H1N1) (PR8) | 1.63 | 1.03 | 127 | 207 |
| *Coronavirid ae* | SARS-CoV-2 (KCDC03) | 1.71 | 1.20 | 122 | 185 |
| *Coronavirid ae* | Bovine coronavirus (KWD20) | 3.44 | 1.12 | 54 | 158 |
| *Coronavirid ae* | Porcine epidemic diarrhea coronavirus (QIAP1401) | 1.94 | 1.62 | 108 | 128 |
| *Reoviridae* | Species A Rotavirus (NCDV) | 7.08 | 5.22 | 32 | 36 |
| *Arterivirid ae* | Porcine respiratory reproductive syndrome virus (LMY) | 2.70 | 2.29 | 78 | 95 |
| *Calicivirid ae* | Porcine sapovirus (Cowden) | 3.70 | 2.77 | 62 | 81 |

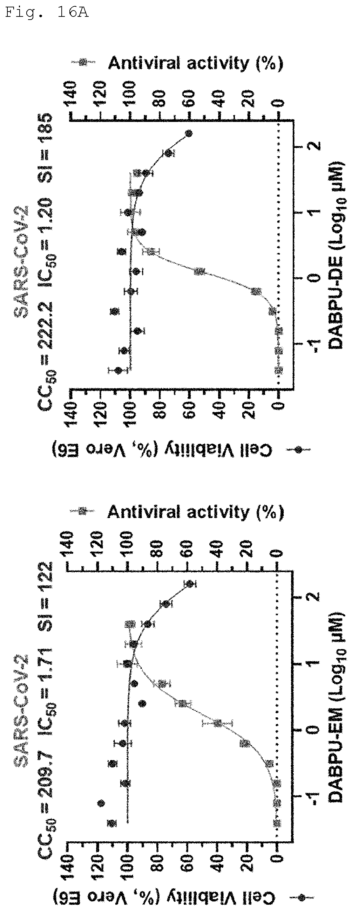

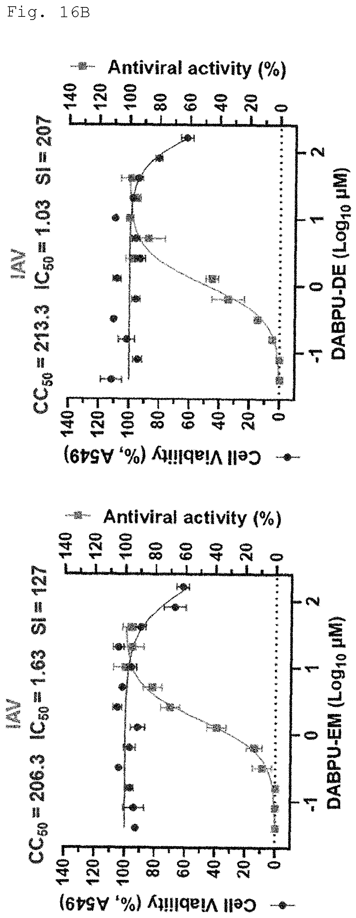

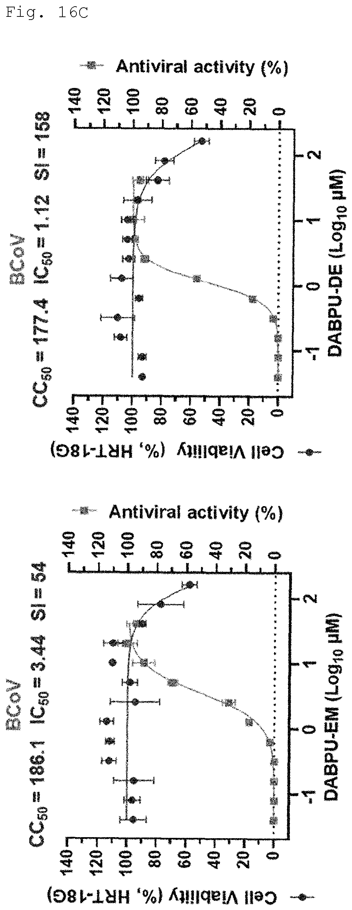

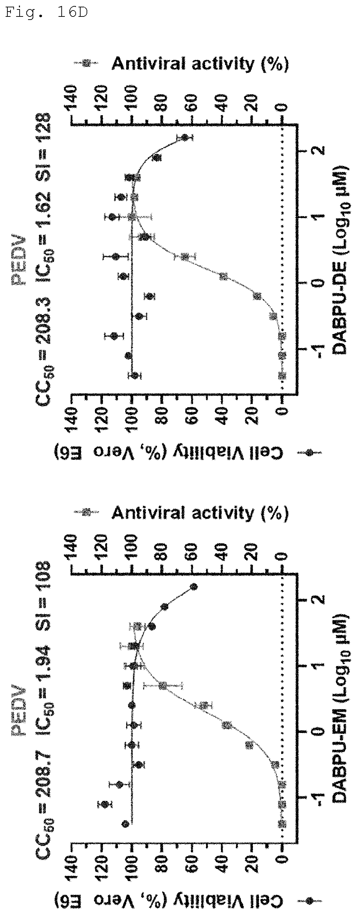

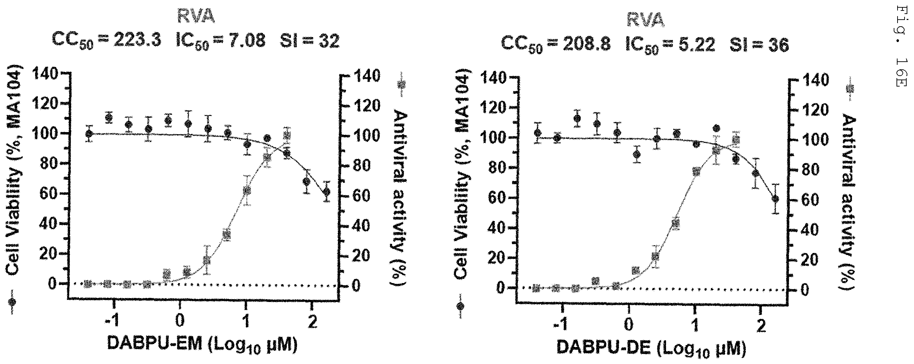

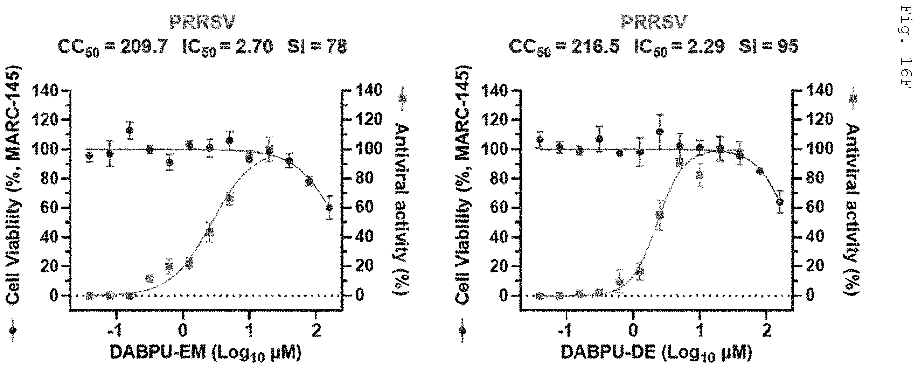

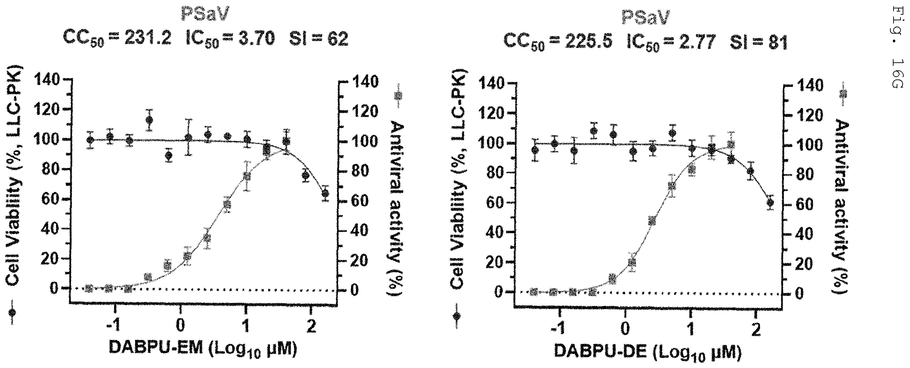

As shown in Table 9 and FIGS. 16a to 16g, DABPU-EM had a half-maximal inhibitory concentration of 1.63 to 7.08, while DABPU-DE ranged in half-maximal inhibitory concentration from 1.03 to 5.22. Notably, IC50s against influenza A virus and SARS-CoV-2, which have caused significant global impact, were measured to be 1.63 and 1.71 for DABPU-EM and 1.03 and 1.20 for DABPU-DE, respectively. These results indicate that both DABPU-EM and DABPU-DE are highly effective in inhibiting these viruses, even at low concentrations.

Specifically, DABPU-EM efficiently inhibited SARS-CoV-2 KCDC03 strain, influenza A virus PR8 strain, bovine coronavirus KWD20 strain, porcine epidemic diarrhea coronavirus QIAP1401 strain, bovine species A rotavirus NCDV strain, porcine reproductive and respiratory syndrome virus LMY strain, and porcine sapovirus Cowden strain, compared to the cytotoxicity, with a selective index (SI) of 122, 127, 54, 108, 32, 78, and 62, therefor, respectively.

Furthermore, DABPU-DE effectively SARS-CoV-2 KCDC03 strain, influenza A virus PR8 strain, bovine coronavirus KWD20 strain, porcine epidemic diarrhea coronavirus QIAP1401 strain, bovine species A rotavirus NCDV strain, porcine reproductive and respiratory syndrome virus LMY strain, and porcine sapovirus Cowden strain, compared to the cytotoxicity, with a selective index (SI) of 185, 207, 158, 128, 36, 95, and 81 therefor, respectively. Overall, DABPU-DE exhibited a higher viral inhibition capability compared to DABPU-EM.

### EXPERIMENTAL EXAMPLE 13: Inhibitory Effect of Lipase Inhibitors DABPU-EM and DABPU-DE on Viral Genome Copy Number and Progeny Production

Using the same method as in Experiment Example 6, the inhibitory effects of DABPU-EM and DABPU-DE on viral genome copy number and progeny virus production were detected. Each virus was allowed to adsorb onto the cells which were then cultured in a freshly changed medium containing DABPU-EM or DABPU-DE. Thereafter, the cells were detached from the wells for subsequent experiments.

To analyze the inhibitory effects of DABPU-EM and DABPU-DE on progeny virus production in each virus, the same procedures as in Experiment Example 6 were followed, with the exception that, after adsorption of the supernatant, each culture medium was added and additional incubation was made for 18 hours for the plates infected with SARS-CoV-2, 12 hours for those with influenza A virus, 18 hours for these with bovine coronavirus, 18 hours for those with porcine epidemic diarrhea coronavirus, 12 hours for those with bovine species A rotavirus, 18 hours for those with porcine reproductive and respiratory syndrome virus, 18 hours for those with porcine sapovirus for 18 hours. Subsequently, the supernatant was removed, the cells were fixed with 4% paraformaldehyde solution for 15 minutes, washed three times in each well with phosphate-buffered saline, treated with 0.2% Triton X-100 at 20°C for 10 minutes, and stained to examine virus-positive cells.

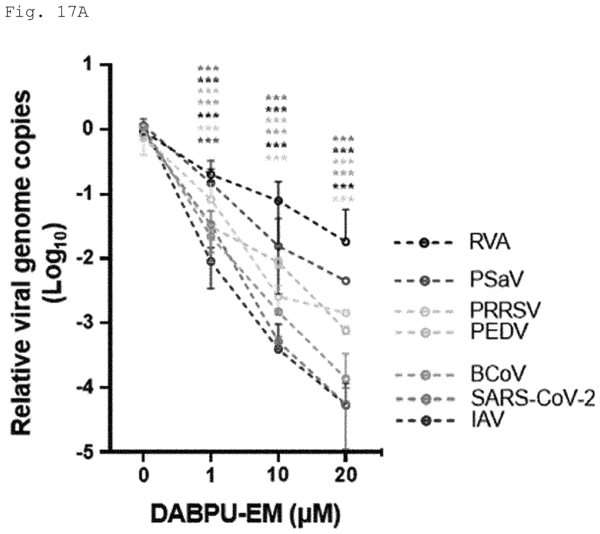

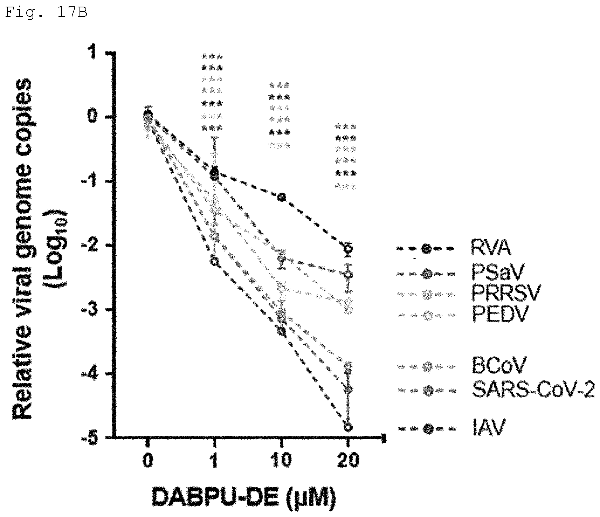

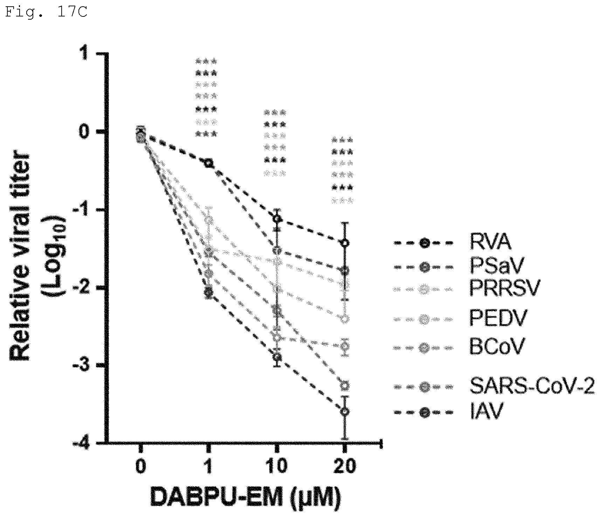

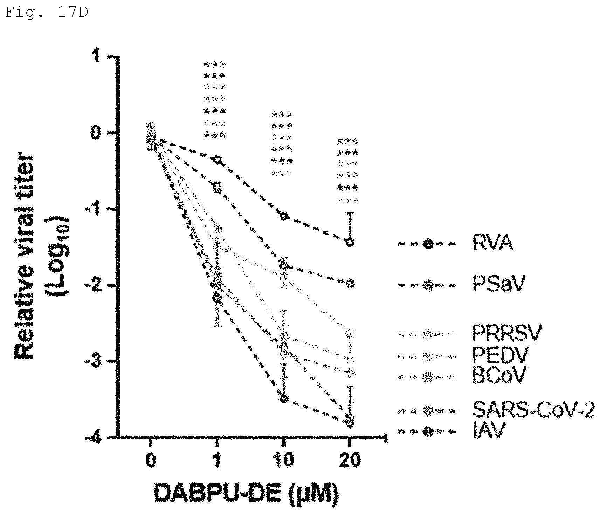

As evident from FIGS. 17a to 17d, both DABPU-EM and DABPU-DE reduced the genome copy number and progeny virus production in all tested viruses at concentrations of 1, 10, and 20 µM in a dose-dependent manner with significance. Therefore, it was confirmed that using lipase inhibitors can have an antiviral effect against a broad spectrum of RNA viruses.

### EXPERIMENTAL EXAMPLE 14: Effects of Lipase Inhibitor Atglistatin (DABPU-DM) Derivatives DABPU-DE and DABPU-EM on Survival Rate, Weight Loss, Clinical Symptoms, Virus Proliferation, and Lesion in Mice Infected with Influenza A Virus (IAV)

To verify that antiviral effects are induced as DABPU-EM and DABPU-DE inhibit lipases in animals infected with influenza A virus, the same method as in Experiment Example 7 was employed.

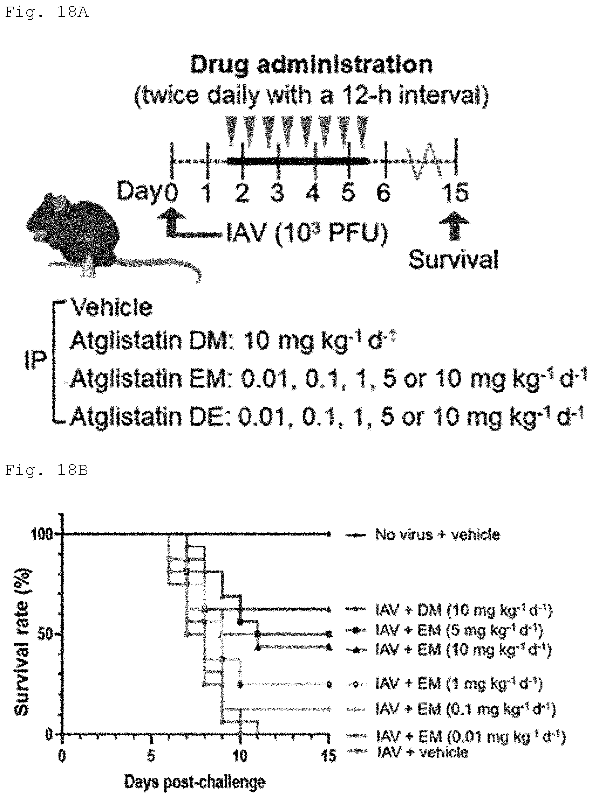

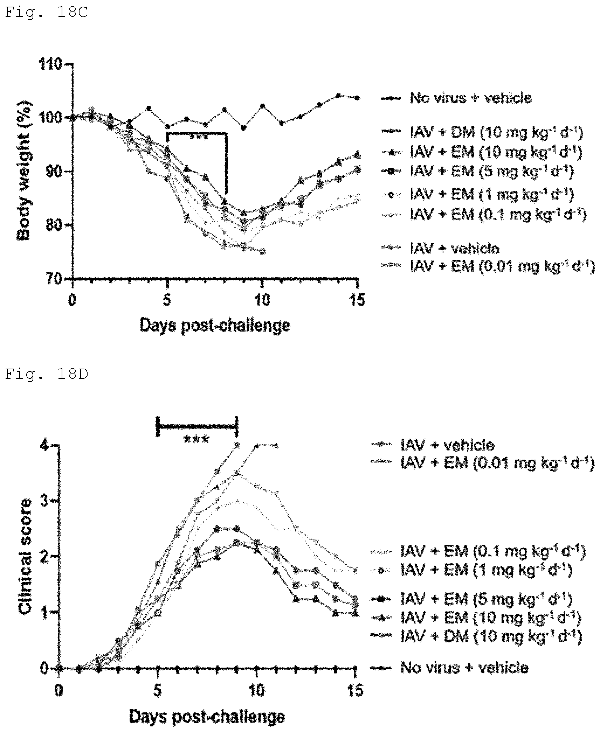

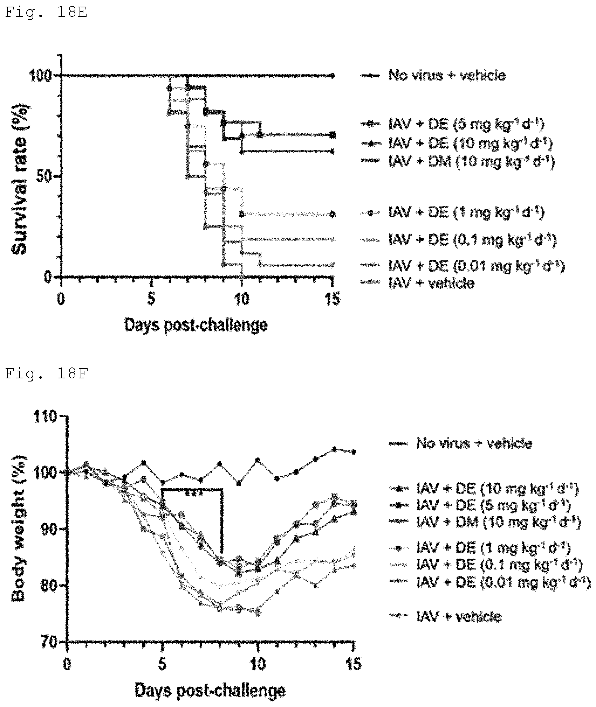

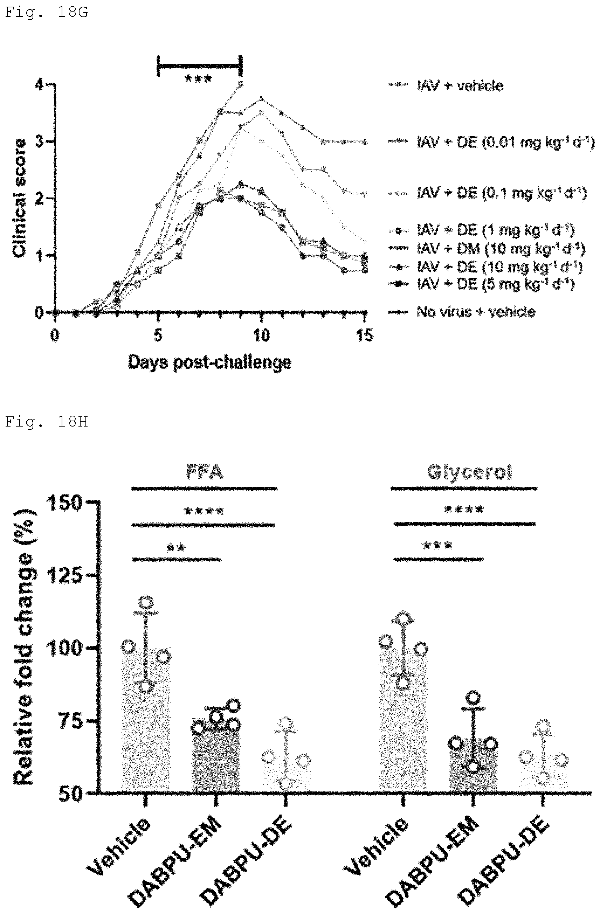

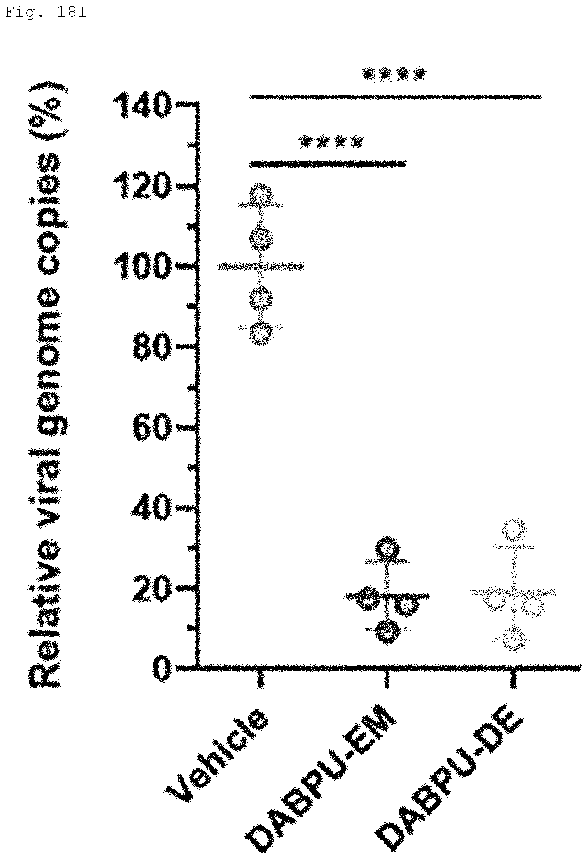

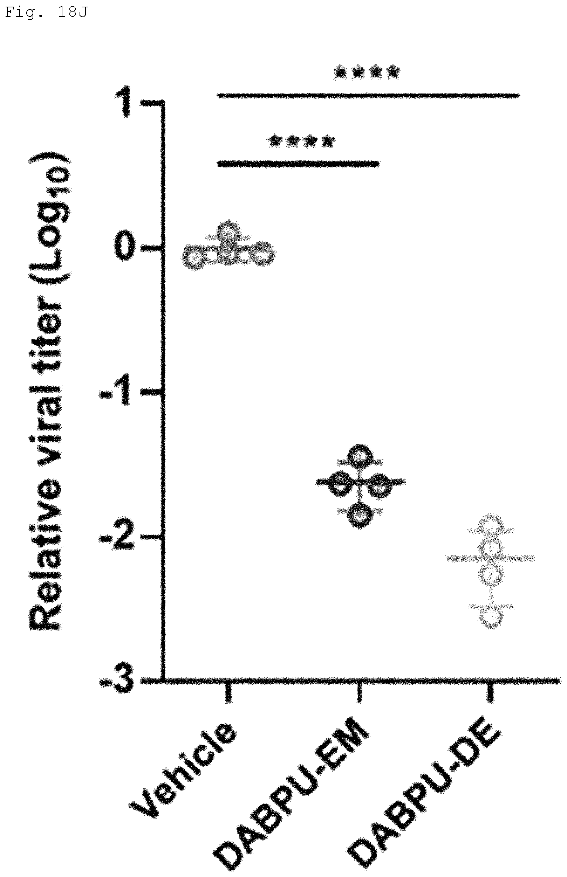

As can be seen in FIGS. 18b to 18g, the administration of the atglistatin derivatives DABPU-EM and DABPU-DE to mice infected with influenza A virus resulted in an improvement in survival rate, body weight loss, and clinical symptoms in a dose-dependent manner. Notably, the survival rate was increased by 62.5% in the group treated with 5 mg kg⁻¹ day⁻¹ atglistatin (DABPU-DM), 50% in the group treated with 10 mg kg⁻¹ day⁻¹ DABPU-EM, and 68.8% in the groups treated with 5 mg kg⁻¹ day⁻¹ and 10 mg kg kg⁻¹ day⁻¹ DABPU-DE. In conclusion, DABPU-DE demonstrated a better therapeutic effect than atglistatin (DABPU-DM) in influenza A virus infection.

Next, examination was made of the effects of DABPU-EM or DABPU-DE treatment on free fatty acid and glycerol levels, viral genome copy number and progeny virus numbers in the lung tissues in the same manner as in Experimental Example 7. Additionally, to assess how much influenza A virus-induced pneumonia could be alleviated, immunofluorescence staining for virus antigen and lipid droplets in mouse lung tissues, histopathological analysis, and immunohistochemical staining for virus antigens were performed as follows.

As shown in FIGS. 18h to 18j, the administration of the lipase inhibitors DABPU-EM and DABPU-DE to mice infected with influenza A virus significantly reduced free fatty acids and glycerol levels (h), virus gene copy number (i), and progeny virus number (j) in lung tissues. However, DABPU-DE was more effective in reducing these measures, compared to DABPU-EM.

For the evaluation of lipid droplets and influenza A virus antigens in the lung tissues, the fixed tissues were prepared into cryosections 3 µm thick in the same manner as in Experiment Example 7.

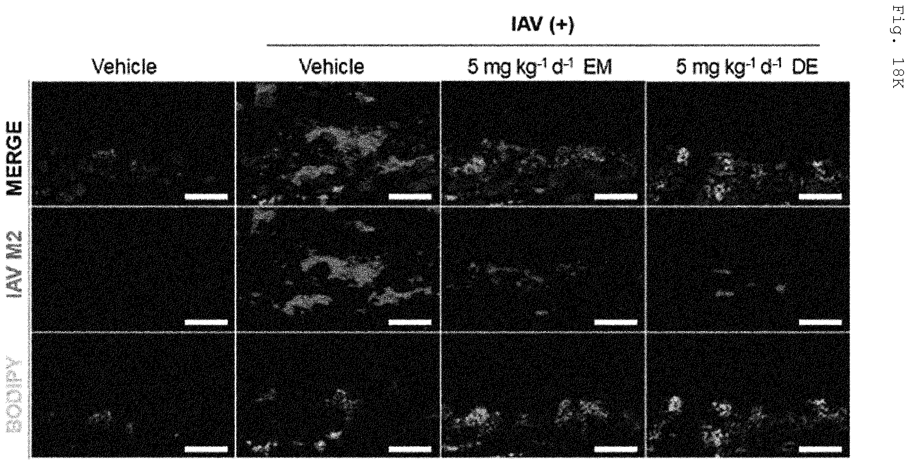

As can be seen in FIG. 18K, the administration of the lipase inhibitors DABPU-EM and DABPU-DE to mice infected with influenza A virus resulted in significantly decreasing virus antigen levels in the bronchiolar epithelial cells of the treated mice compared to the untreated virus-inoculated group. However, there was no significant decrease in the number of lipid droplets.

For histopathological analysis of the lung tissues, the fixed tissues were dehydrated through graded alcohols, cleared in xylene, embedded in paraffin, and sectioned into 3-pm slices for histological examination under an optical microscope in the same manner as in Experiment Example 7.

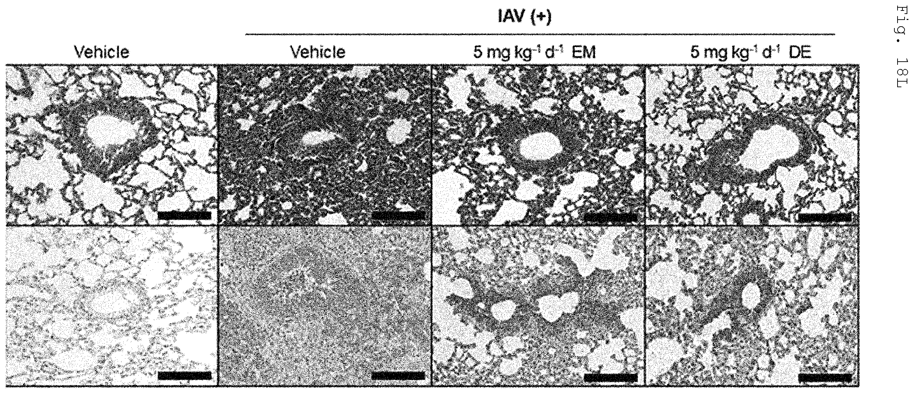

As shown in FIG. 18L, the administration of the lipase inhibitors DABPU-EM and DABPU-DE to mice infected with influenza A virus led to alleviation and reduction of pulmonary lesions, including interstitial pneumonia, and the number of virus-positive cells in the treated group compared to the untreated virus-inoculated group. Particularly, DABPU-DE showed higher effectiveness than DABPU-EM.

In conclusion, the atglistatin derivatives DABPU-EM and DABPU-DE demonstrated efficacy in protecting mice infected with influenza A virus. When administered at 10 mg kg⁻¹ day⁻¹, DABPU-EM and DABPU-DE increased the survival rate to 50% and 68.8%, respectively, compared to a 100% mortality rate in the control group. Notably, DABPU-DE showed a higher virus inhibition ability than DABPU-EM.

### EXPERIMENTAL EXAMPLE 15: Effects of Treatment with Lipase Inhibitor Atglistatin (DABPU-DM) Derivative DABPU-DE on Severity of Pneumonia, Virus Proliferation, Weight Loss, and Lesion in SARS-CoV-2-Infected Hamsters

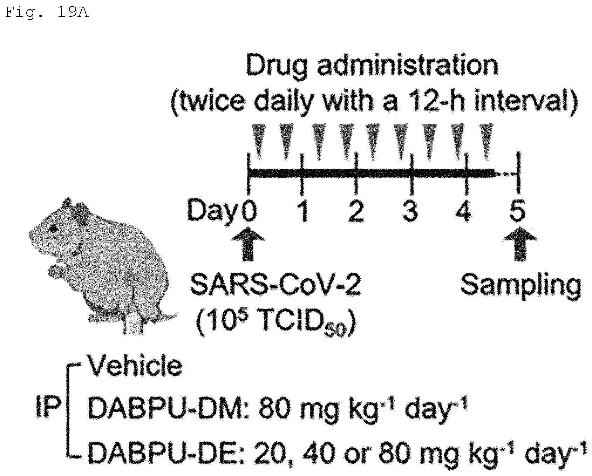

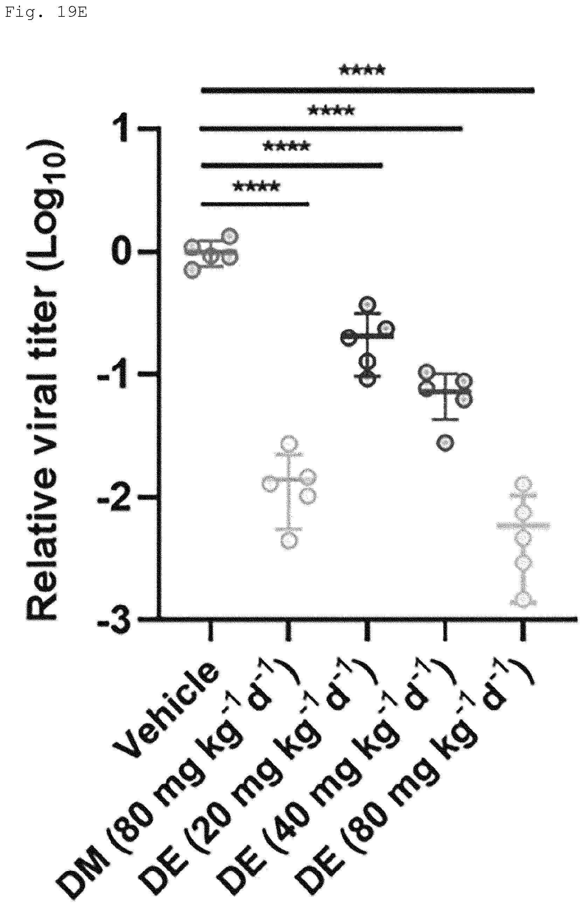

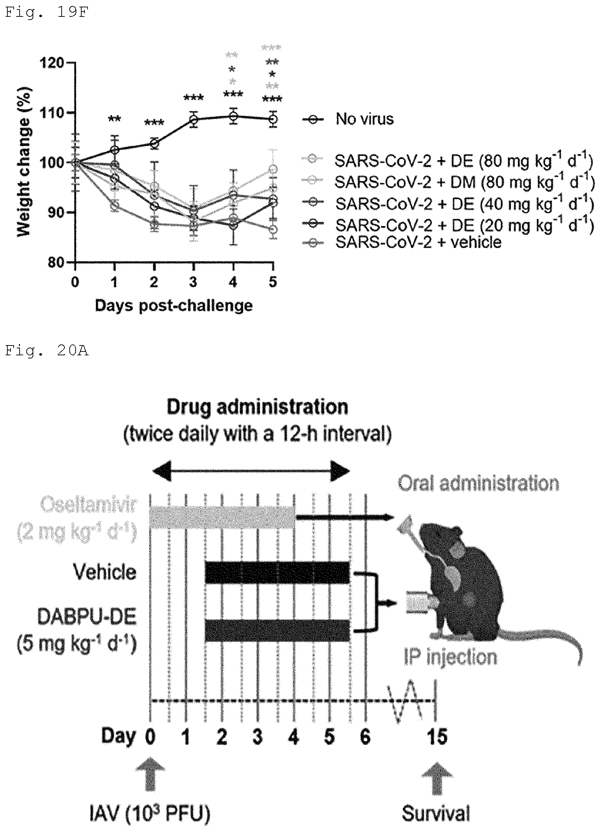

To verify that antiviral effects are induced by lipase inhibition in animals infected with SARS-CoV-2, the same method as in Experiment Example 8 was employed (FIG. 19A) .

For an assay for the antiviral efficacy of atglistatin (DABPU-DM) and DABPU-DE, a total of six experimental groups were established as follows: a non-injected and untreated group (No virus + vehicle), a group injected with SARS-CoV-2 KCDC03 strain and untreated (SARS-CoV-2 + vehicle), a group injected with SARS-CoV-2 KCDC03 strain and treated with 40 mg/kg/day atglistatin (DABPU-DM) (SARS-CoV-2 + DM (40 mg kg⁻¹ day⁻¹)), a group injected with SARS-CoV-2 KCDC03 strain and treated with 20 mg/kg/day DABPU-DE (SARS-CoV-2 + DE (20 mg kg⁻¹ day⁻¹)), a group injected with SARS-CoV-2 KCDC03 strain and treated with 40 mg/kg/day DABPU-DE (SARS-CoV-2 + DE (40 mg kg⁻¹ day⁻¹)), and a group injected with SARS-CoV-2 KCDC03 strain and treated with 80 mg/kg/day DABPU-DE (SARS-CoV-2 + DE (80 mg kg⁻¹ day⁻¹)). Each group of five female Golden Syrian hamsters, each 11 weeks old, was acclimatized for one week under the described conditions before use in subsequent experiments (FIG. 19A).

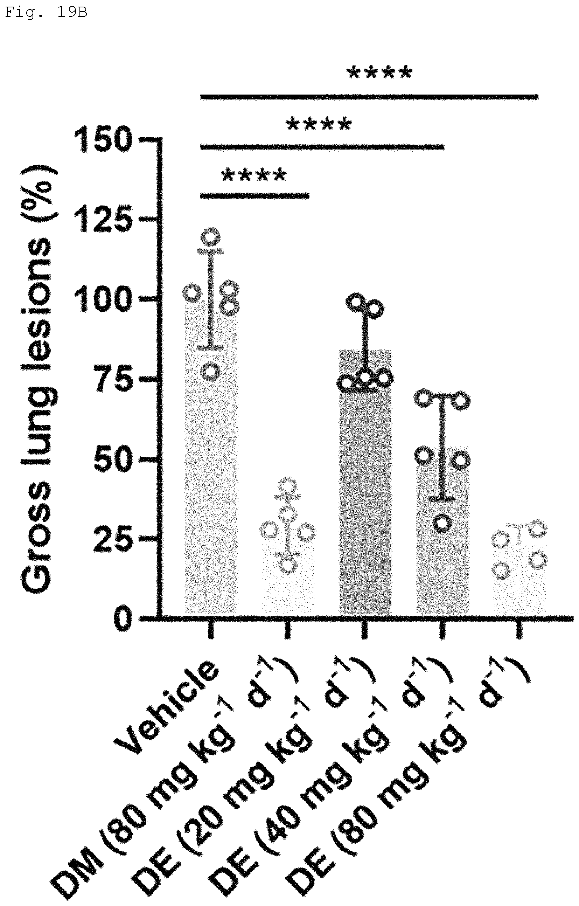

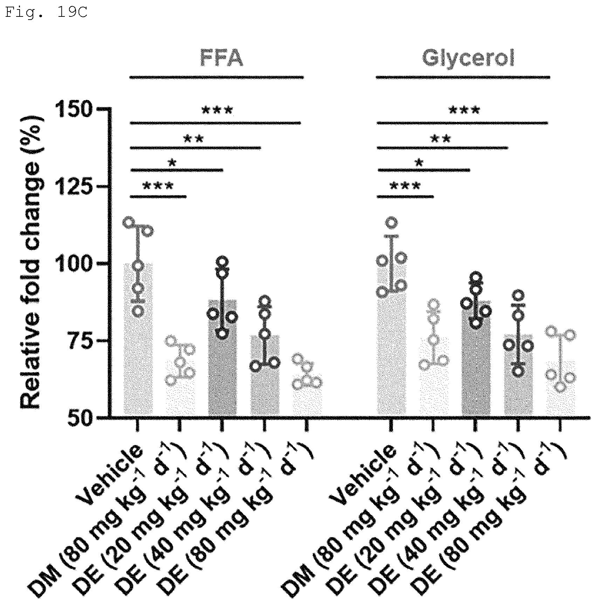

As can be seen in FIGS. 19b and 19c, the administration of atglistatin (DABPU-DM) and DABPU-DE to mice infected with SARS-CoV resulted in alleviating pneumonia severity in a dose-dependent manner and decreasing free fatty acid and glycerol production with significance. Notably, pneumonia severity was reduced by 70% in the group treated with 80 mg kg⁻¹ day⁻¹ atglistatin (DABPU-DM) for 4.5 days. In conclusion, DABPU-DE demonstrated a better therapeutic effect than atglistatin (DABPU-DM) in SARS-CoV-2 infection.

Next, examination was made of the effects of atglistatin (DABPU-DM) or DABPU-DE treatment on viral genome copy number and progeny virus numbers in the lung tissues in the same manner as in Experimental Example 8. In this regard, the drugs were assayed for inhibitory activity against viral genome replication by performing real-time PCR for the SARS-CoV-2 nucleocapsid gene and the host β-actin gene to quantitate viral genome copies.

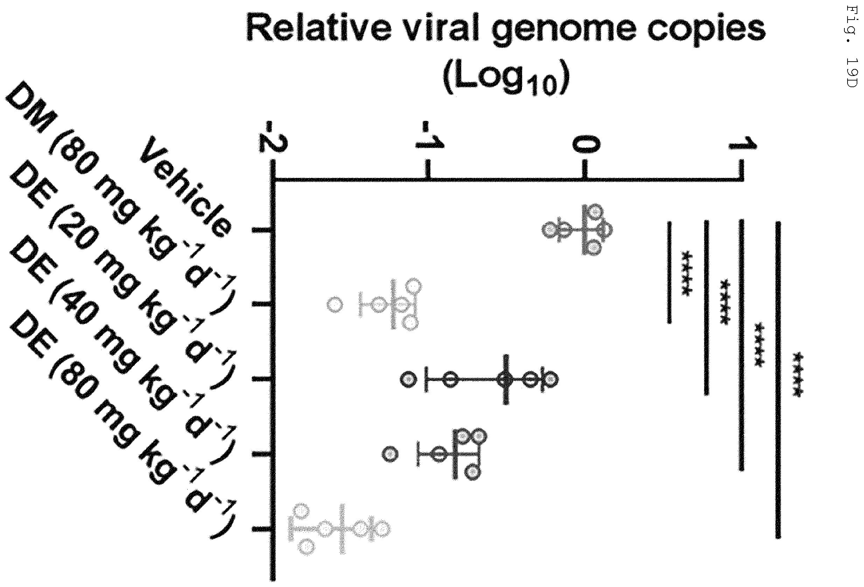

As shown in FIGS. 19d to 19f, the administration of the lipase inhibitors atglistatin (DABPU-DM) and DABPU-DE to hamsters infected with SARS-CoV-2 significantly reduced viral genome copy number (d), and progeny virus number (e) in lung tissues, thus ultimately blocking weight loss in the hamsters (f).

In conclusion, atglistatin (DABPU-DM) and its derivative DABPU-DE were effective for protecting hamsters from infection with SARS-CoV-2. Particularly, a group treated with 80 mg kg⁻¹ day⁻¹ DABPU-DE decreased the severity of pneumonia by 75%, which exhibited a higher therapeutic effect than the group treated with 80 mg kg⁻¹ day⁻¹ atglistatin (DABPU-DM). Additionally, the group treated with 80 mg kg⁻¹ day⁻¹ DABPU-DE had lower levels of free fatty acids and glycerol in lung tissues, compared to the group treated with 80 mg kg⁻¹ day⁻¹ atglistatin (DABPU-DM). This indicates that atglistatin inhibits the breakdown of lipid droplets, thereby suppressing the production of free fatty acids and consequently inhibiting virus proliferation. This result demonstrates that DABPU-DE has a more substantial inhibitory effect compared to atglistatin (DABPU-DM).

### EXPERIMENTAL EXAMPLE 16: Effects of Administration of DABPU-DE and Anti-Influenza A Virus (IAV) Agent Either Individually or in Combination on Survival Rate, Weight Loss, Clinical Symptoms, Virus Proliferation, and Lesion in Infected Mice and Effects of Administration of DABPU-DE and Anti-SARS-CoV-2 Agent Either Individually or in Combination on Survival Rate, Weight Loss, Clinical Symptoms, Virus Proliferation, and Lesion in Infected Hamsters

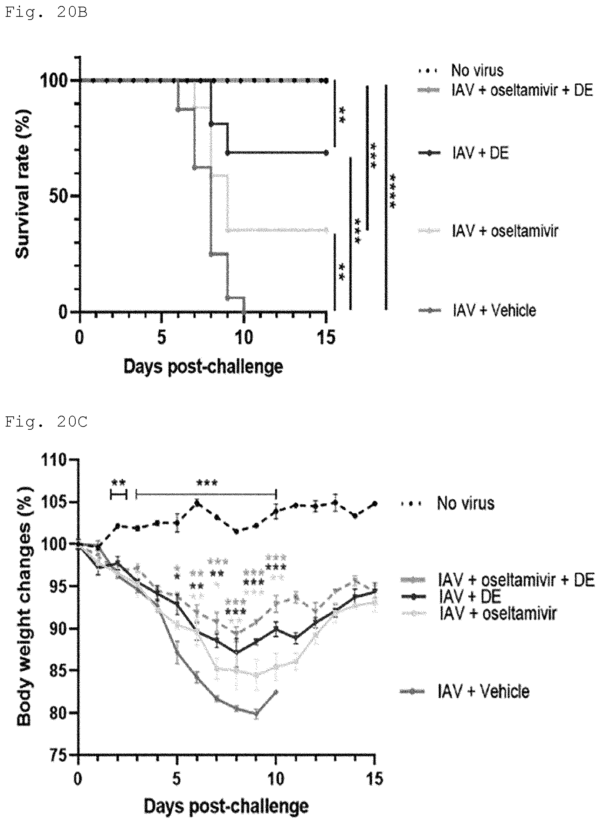

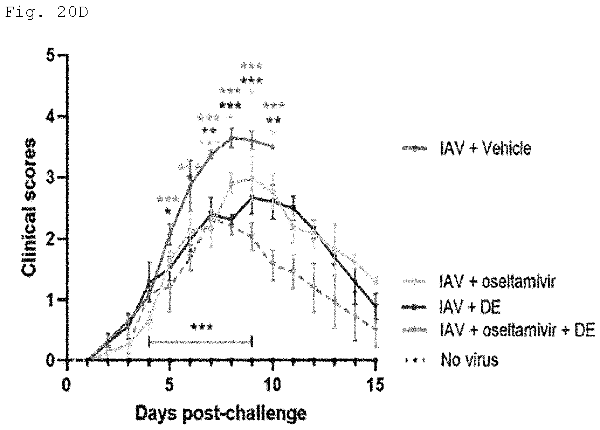

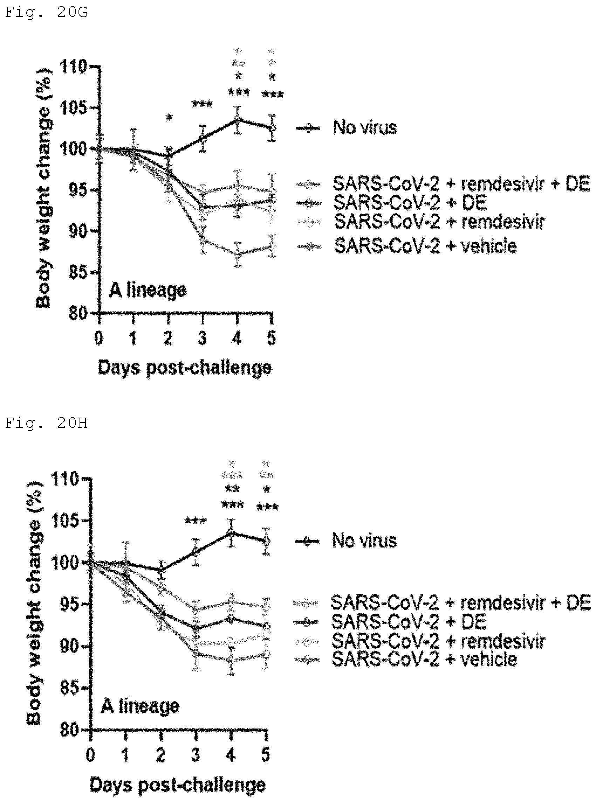

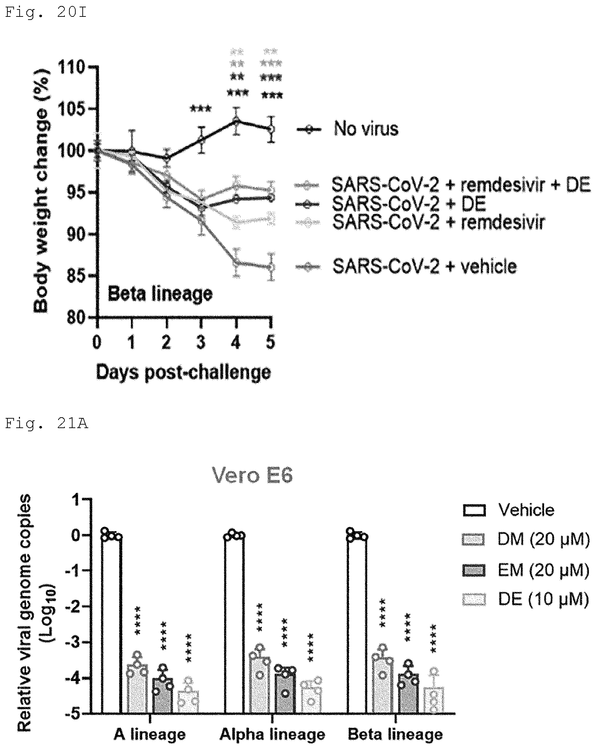

For use in verifying the antiviral effect when administering the atglistatin (DABPU-DM) derivative DABPU-DE and the antiviral agent oseltamivir either individually or in combination into influenza A virus-infected animals, 150 mg/ml of DABPU-DE and 100 mg/ml of oseltamivir were dissolved in DMSO and H₂O, respectively. To evaluate the antiviral effect depending on the concentration, 0.5 mg/ml DABPU-DE and 0.2 mg/ml oseltamivir were prepared. In this regard, a mix of DMSO:40% PEG:H₂O at a ratio of 1:4:5 or H₂O was used as a solvent. When administered to mice, 200 µL of the solutions accounted for doses of 5 mg kg⁻¹ day⁻¹ for DABPU-DE and 2 mg kg⁻¹ day⁻¹ for oseltamivir (see FIG. 20A).

For an assay for antiviral efficacy when administering DABPU-DE and atglistatin either individually or in combination, a total of five experimental groups were established as follows: a non-injected and untreated group (No virus + vehicle), a group injected with influenza A virus PR8 strain and untreated (IAV + vehicle), a group injected with influenza A virus PR8 strain and treated with 2 mg/kg/day oseltamivir (IAV + oseltamivir), a group injected with influenza A virus PR8 strain and treated with 5 mg/kg/day DABPU-DE (IAV + DE), and a group injected with influenza A virus PR8 strain and treated with 2 mg/kg/day oseltamivir and 5 mg/kg/day DABPU-DE (IAV + oseltamivir + DE) . Each group of 16 female wild-type C57BL/6J mice, each seven weeks old, was acclimatized for one week under the described conditions and the same experiment as in Experimental Example 9 was conducted.

As can be seen in FIGS. 20b to 20d, compared to the administration of DABPU-DE or atglistatin alone, the combined administration of oseltamivir and DABPU-DE allowed the animals to be alive 100%, and further improved survival rate, body weight, and clinical symptoms, with a more pronounced effect in the treatment of influenza A virus infection.

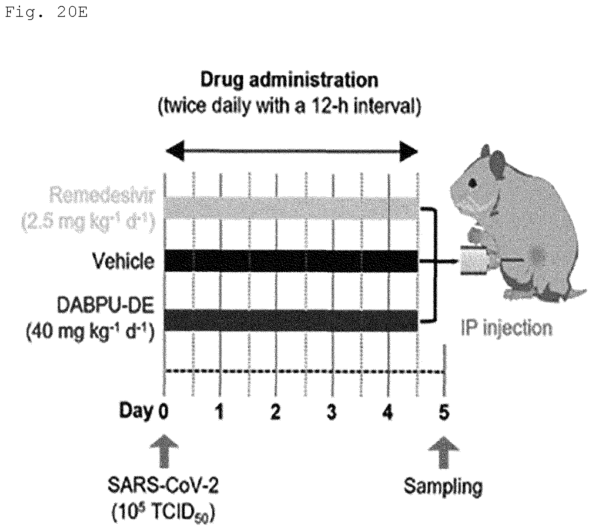

For use in verifying the antiviral effect when administering the atglistatin (DABPU-DM) derivative DABPU-DE and the antiviral agent remdesivir either individually or in combination into animals infected with SARS-CoV-2 KCDC03 strain (A lineage), KDCA51463 strain (Alpha lineage), or KDCA55905 strain (Beta lineage), 150 mg/ml of DABPU-DE and 50 mg/ml of remdesivir were dissolved in DMSO. To evaluate the antiviral effect depending on the concentration, 7.5 mg/ml DABPU-DE and 0.47 mg/ml remdesivir were prepared. In this regard, a mix of DMSO:40% PEG:H₂O at a ratio of 1:4:5 or a mix of DMSO: Corn oil at a ratio of 1:9 was used as a solvent. When administered to hamsters, 800 µL of the solutions accounted for doses of 40 mg kg⁻¹ day⁻¹ for DABPU-DE and 2.5 mg kg⁻¹ day⁻¹ for remdesivir (see FIG. 20E) .

For an assay for antiviral efficacy against SARS-CoV-2 KCDC03 strain (A lineage), KDCA51463 strain (Alpha lineage), and KDCA55905 strain (Beta lineage) when administering remdesivir and DABPU-DE either individually or in combination, a total of five experimental groups were established as follows: a non-injected and untreated group (No virus + vehicle), a group injected with SARS-CoV-2 and untreated (SARS-CoV-2 + vehicle), a group injected with SARS-CoV-2 and treated with 2.5 mg/kg/day oseltamivir (SARS-CoV-2 + oseltamivir), a group injected with SARS-CoV-2 and treated with 40 mg/kg/day DABPU-DE (SARS-CoV-2 + DE), and a group injected with SARS-CoV-2 and treated with 2.5 mg/kg/day remdesivir and 40 mg/kg/day DABPU-DE (IAV + oseltamivir + DE). Each group of 5 Golden Syrian hamsters, each 11 weeks old, was acclimatized for one week under the described conditions and the same experiment as in Experimental Example 9 was conducted.

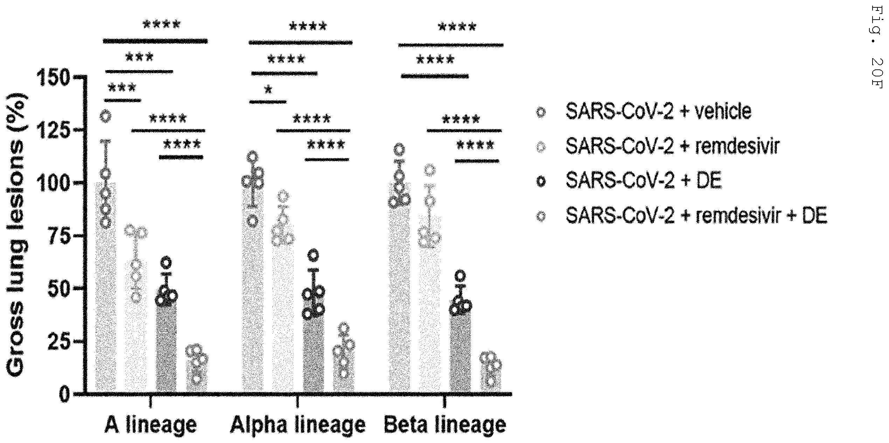

As seen in FIGS. 20f to 20i, the combined administration of remdesivir and DABPU-DE resulted in a significant improvement in the severity of pneumonia by 84% for SARS-CoV-2 lineage A, 80% for SARS-CoV-2 lineage Alpha, and 87% for SARS-CoV-2 lineage Beta. The combined treatment of remdesivir/DABPU-DE showed greater improvement in the severity of pneumonia and reduction in weight loss, compared to the administration of remdesivir or DABPU-DE alone, and it exhibited more pronounced therapeutic effects in infections with various variants of SARS-CoV-2.

Consequently, the lipase inhibitor DABPU-DE, when co-administered with oseltamivir for influenza A virus infections, showed a more significant therapeutic effect, compared to the administration of oseltamivir alone. Similarly, the co-administration of DABPU-DE with remdesivir exhibited a more significant therapeutic effect for infections with various variants of SARS-CoV-2, compared to the administration of remdesivir alone.

Referring to the results of Experimental Example 9, the group treated with 40 mg kg⁻¹ day⁻¹ DABPU-DE alone showed a more improvement in the severity of pneumonia in all SARS-CoV-2 infections, compared to the group treated with 40 mg kg⁻¹ day⁻¹ atglistatin alone. Particularly, the group treated with a combination of 40 mg kg⁻¹ day⁻¹ DABPU-DE and remdesivir showed more improvement in all SARS-CoV-2 infections compared to the group treated with a combination of 40 mg kg⁻¹ day⁻¹ atglistatin and remdesivir.

That is, the inhibition of the breakdown of lipid droplets in infections with influenza A virus and SARS-CoV-2 helped suppress virus proliferation, and this inhibitory effect was more pronounced with DABPU-DE than with atglistatin.

### EXPERIMENTAL EXAMPLE 17: Inhibitory Effect of Atglistatin (DABPU-DM) and Derivatives DABPU-DE and DABPU-EM against Proliferation of SARS-CoV-2 and Influenza A Virus (IAV) in Various Cells

For use in verifying the antiviral effect when applying the atglistatin (DABPU-DM) derivative DABPU-DE to human cells infected with SARS-CoV-2 KCDC03 strain (Lineage A), SARS-CoV-2 KDCA51463 strain (Alpha lineage, British variant), SARS-CoV-2 KDCA55905 strain (Beta lineage, South African variant), and influenza A virus PR8 strain, 1, 10, 20 mM atglistatin (DABPU-DM), DABPU-EM, and DABPU-DE were prepared in DMSO. Subsequently, they were each diluted in a culture medium to form final concentrations of 1, 10, and 20 µM.

To achieve the goal, the monolayer of Vero E6 cells in 12-well plates were infected with SARS-CoV-2 strain KCDC03, KDCA51463, or KDCA55905 at a multiplicity of infection (MOI) of 0.01 ffu/cell. The virus was allowed to adsorb to the cells for 1 hour in a 37°C CO2 incubator. Subsequently, the cells were washed with phosphate-buffered saline and cultured for 18 hours in DMEM supplemented with 1 ug/ml TPCK-treated trypsin, 100 u/ml penicillin, and 100 ug/ml streptomycin, in a 37°C CO2 incubator. Then, the wells were washed three times with phosphate-buffered saline, and the medium was replaced with a medium containing a final concentration of20 µM DABPU-DM, DABPU-EM, or DABPU-DE. After an additional 18 hours of incubation, the supernatant was used for determining titers of progeny viruses using cell culture immunofluorescence assay (CCIF assay), and RNA was extracted to measure the number of viral genome copies in the same manner as in Experimental Example 4.

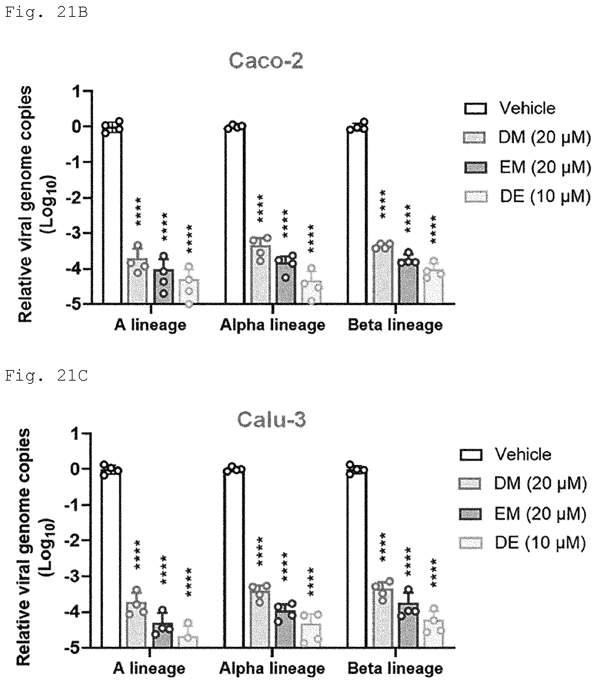

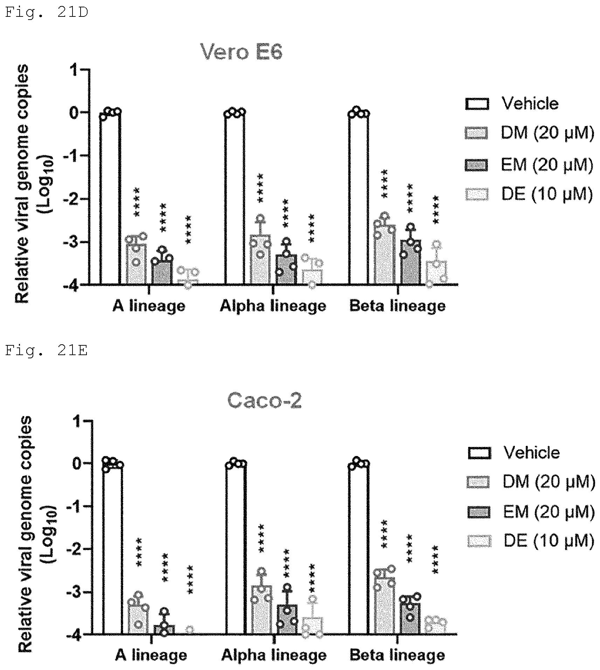

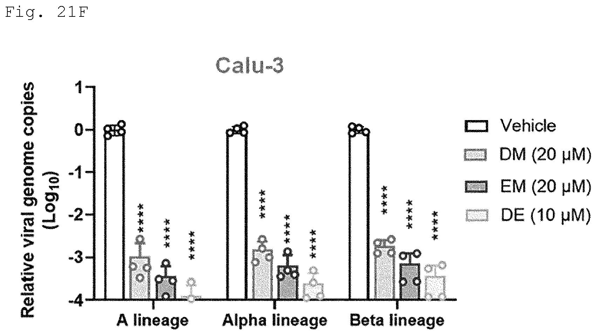

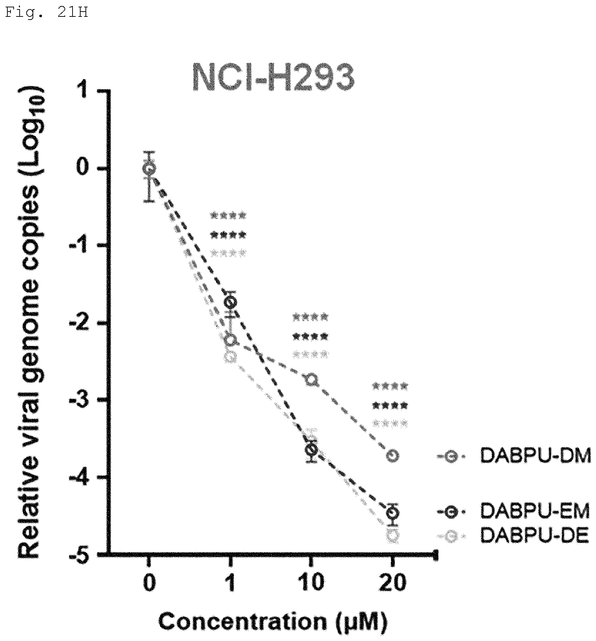

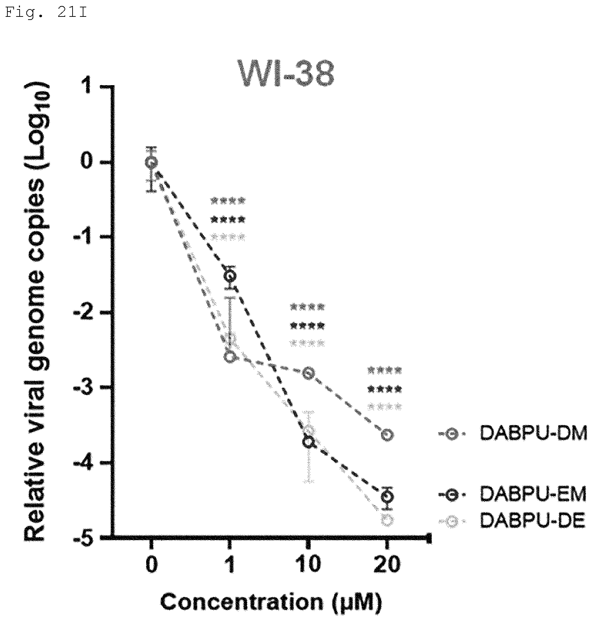

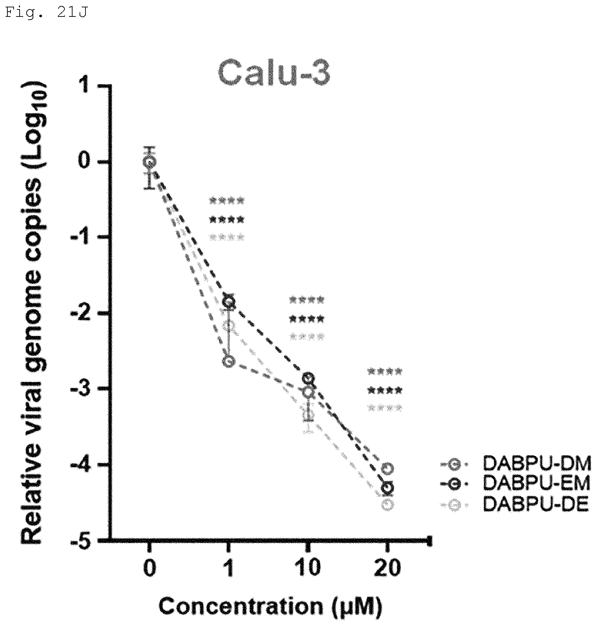

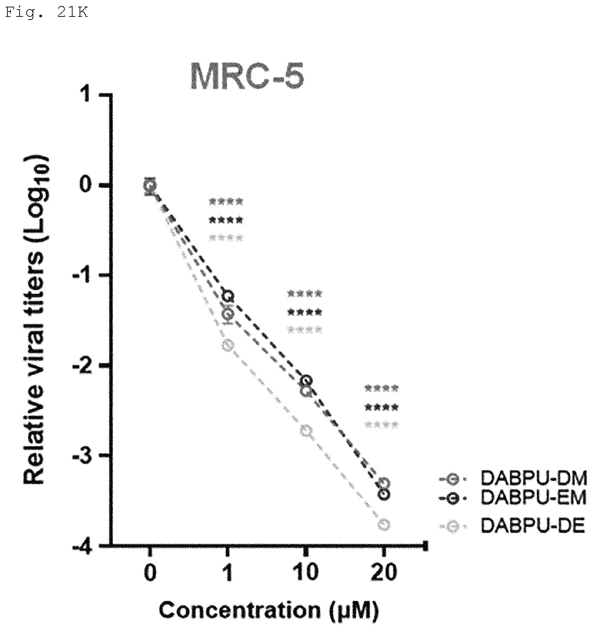

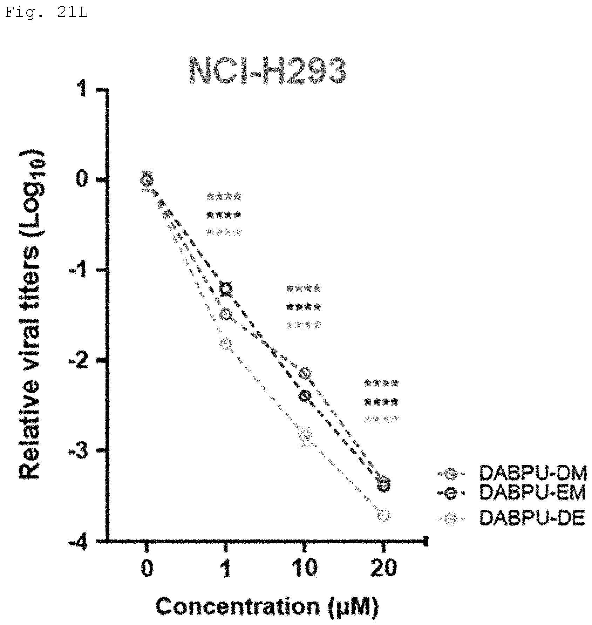

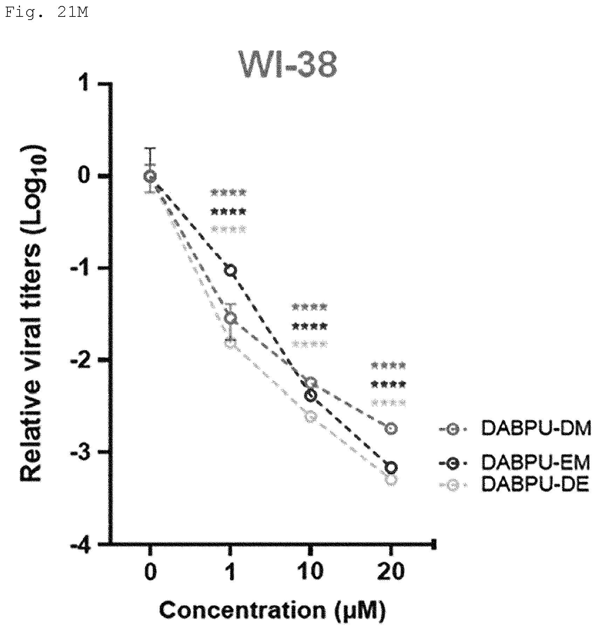

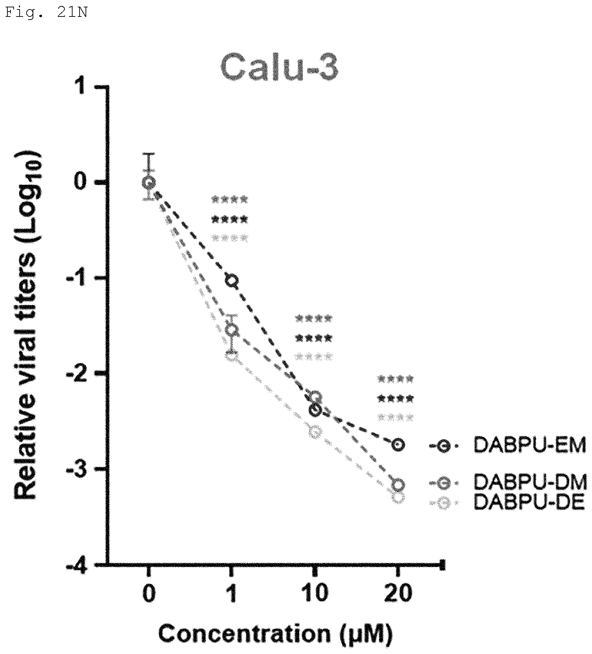

Among cultured cells from SARS-CoV-2-infected humans, as shown in FIGS. 21a to 21f, treatment groups with DABPU-DM, DABPU-EM, or DABPU-DE significantly reduced the number of viral genome copies, compared to the solvent (vehicle) treated group.

In conclusion, atglistatin (DABPU-DM) and its derivatives, DABPU-EM or DABPU-DE, demonstrated significant inhibitory effects on the proliferation of SARS-CoV-2. The inhibitory effect was consistently observed in various strains of the virus, including SARS-CoV-2 KCDC03 (Lineage A), SARS-CoV-2 KDCA51463 (Alpha lineage, British variant), and SARS-CoV-2 KDCA55905 (Beta lineage, South African variant). Notably, DABPU-DE showed superior viral inhibition compared to atglistatin (DABPU-DM) . This suggests that not only atglistatin (DABPU-DM) but also its derivatives, DABPU-DE and DABPU-EM, can be utilized as therapeutic agents for the treatment of SARS-CoV-2 infections in humans.

To achieve the goal, the monolayer of MRC-5, NCI-H293, WI-38, and Calu-3 cells in 12-well plates were infected with influenza A virus PR8 strain at a multiplicity of infection (MOI) of 0.01 ffu/cell. The virus was allowed to adsorb to the cells for 1 hour in a 37°C CO2 incubator. Subsequently, the cells were washed with phosphate-buffered saline and cultured for 12 hours in DMEM supplemented with 1 ug/ml TPCK-treated trypsin, 100 u/ml penicillin, and 100 ug/ml streptomycin, in a 37°C CO2 incubator. Then, the wells were washed three times with phosphate-buffered saline, and the medium was replaced with a medium containing a final concentration of 1, 10, 20 µM DABPU-DM, DABPU-EM, or DABPU-DE. After an additional 12 hours of incubation, the supernatant was used for determining titers of progeny viruses using cell culture plaque assay, and RNA was extracted to measure the number of viral genome copies in the same manner as in Experimental Example 4.

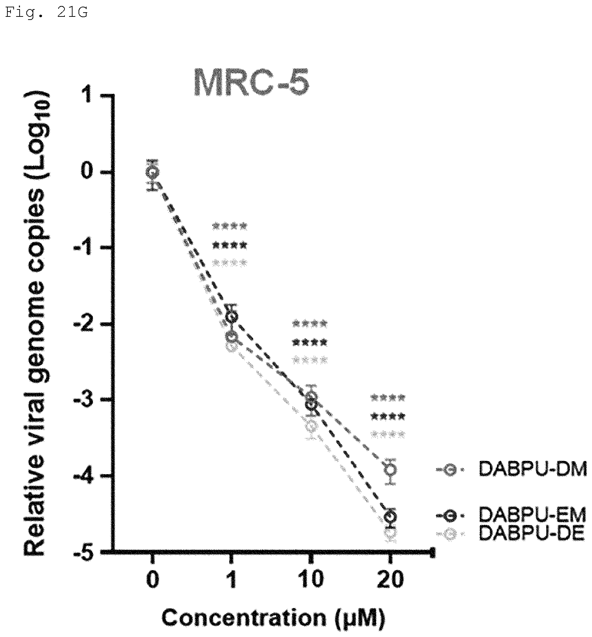

Among MRC-5, NCI-H293, WI-38, and Calu-3 cells from humans infected with influenza A virus, as shown in FIGS. 21g to 21n, the group treated with DABPU-DM, DABPU-EM, or DABPU-DE significantly reduced in viral genome copy number and progeny virus number, compared to the solvent (vehicle) treated group. This result implies that not only atglistatin (DABPU-DM) but also its derivatives, DABPU-DE and DABPU-EM, can be utilized as therapeutic agents for treating influenza A virus infections in humans.

Taken together, the data obtained above demonstrated the inhibitory efficacies of atglistatin (DABPU-DM) and its derivatives, DABPU-EM or DABPU-DE against the proliferation of SARS-CoV-2 and influenza A virus in various human-derived cultured cells, implying that atglistatin (DABPU-DM) and its derivatives, DABPU-EM or DABPU-DE, can be used as therapeutic agents to treat SARS-CoV-2 and influenza A virus infections in humans.

### Industrial Applicability

The present disclosure relates to a pharmaceutical composition including a lipase inhibitor for the prevention or treatment of RNA virus infections and, more specifically, to a pharmaceutical composition including derivatives of an adipose triglyceride lipase (ATGL) inhibitor for the prevention or treatment of RNA virus infections.

## Claims

1. A pharmaceutical composition comprising a lipase inhibitor for prevention or treatment of an RNA virus infection.

2. The pharmaceutical composition of claim 1, wherein the lipase inhibitor is at least one selected from the group consisting of an adipose triglyceride lipase (ATGL) inhibitor, a hormone-sensitive lipase (HSL) inhibitor, and a monoacylglycerol lipase (MGL) inhibitor.

3. The pharmaceutical composition of claim 2, wherein the ATGL inhibitor comprises 3-(4'-(dimethylamino)-(1,1'-biphenyl)-3-yl)-1,1-dimethylurea and a derivative thereof.

4. The pharmaceutical composition of claim 3, wherein the derivative of 3-(4'-(dimethylamino)-(1,1'-biphenyl)-3-yl)-1,1-dimethylurea is at least one selected from the group consisting of:
3-(4'-dimethylamino)-(1,1'-biphenyl)-3-yl)-1,1-diethylurea,
3-(4'-(dimethylamino)-(1,1'-biphenyl)-3-yl)-1-ethyl-1-methylurea,
3-(4'-dimethylamino)-(1,1'-biphenyl)-3-yl)-1,1-diphenylurea,
N-(4'-dimethylamino)-(1,1'-biphenyl)-3-yl)pyrolidine-1-carboxamide, and
N-(4'-(dimethylamino)-(1,1'-biphenyl)-3-yl)morpholine-4-carboxamide.

5. The pharmaceutical composition of claim 1, wherein the pharmaceutical composition further comprises at least one antiviral agent selected from the group consisting of oseltamivir and remdesivir.

6. The pharmaceutical composition of claim 1, wherein the RNA virus is at least one selected from the group consisting of SARS coronavirus types 1 and (SARS-CoV-1 and SARS-CoV-2), influenza A virus (IAV), bovine coronavirus (BCoV), porcine epidemic diarrhea coronavirus (PEDV), bovine species A rotavirus (RVA), porcine reproductive and respiratory syndrome virus (PRRSV), porcine sapovirus (PSaV), norovirus, feline infectious peritonitis virus (FIPV), MERS Corona Virus, Zika virus, dengue fever virus, and hepatitis A and C viruses.

7. An antiviral composition comprising a lipase inhibitor.

8. The antiviral composition of claim 7, wherein the lipase inhibitor is at least one selected from the group consisting of an adipose triglyceride lipase(ATGL) inhibitor, a hormone-sensitive lipase (HSL) inhibitor, and a monoacylglycerol lipase (MGL) inhibitor.

9. The antiviral composition of claim 7, wherein the ATGL inhibitor comprises 3-(4'-(dimethylamino)-(1,1'-biphenyl)-3-yl)-1,1-dimethylurea and a derivative thereof.

10. The antiviral composition of claim 7, wherein the derivative of 3-(4'-(dimethylamino)-(1,1'-biphenyl)-3-yl)-1,1-dimethylurea is at least one selected from the group consisting of:
3-(4'-dimethylamino)-(1,1'-biphenyl)-3-yl)-1,1-diethylurea,
3-(4'-(dimethylamino)-(1,1'-biphenyl)-3-yl)-1-ethyl-1-methylurea,
3-(4'-dimethylamino)-(1,1'-biphenyl)-3-yl)-1,1-diphenylurea,
N-(4'-dimethylamino)-(1,1'-biphenyl)-3-yl)pyrolidine-1-carboxamide, and
N-(4'-(dimethylamino)-(1,1'-biphenyl)-3-yl)morpholine-4-carboxamide

11. The antiviral composition of claim 7, wherein the antiviral composition further comprises at least one antiviral agent selected from the group consisting of oseltamivir and remdesivir.

12. The antiviral composition of claim 7, wherein the RNA virus is at least one selected from the group consisting of SARS coronavirus types 1 and (SARS-CoV-1 and SARS-CoV-2), influenza A virus (IAV), bovine coronavirus (BCoV), porcine epidemic diarrhea coronavirus (PEDV), bovine species A rotavirus (RVA), porcine reproductive and respiratory syndrome virus (PRRSV), porcine sapovirus (PSaV), norovirus, feline infectious peritonitis virus (FIPV), MERS Corona Virus, Zika virus, dengue fever virus, and hepatitis A and C viruses.
